# EUROPEAN PATENT SPECIFICATION

(11) **EP 1 262 548 B1**
(45) Date of publication and mention of the grant of the patent: **08.10.2008**
(21) Application number: 01912268.8
(22) Date of filing: 12.03.2001
(51) Int. Cl.: C07K 16/28, A61K 39/395, A61P 35/02

(54) **POLYPEPTIDE INDUCING APOPTOSIS**
APOPTOSE-INDUZIERENDES POLYPEPTID
POLYPEPTIDE PROVOQUANT L'APOPTOSE

(30) Priority: 10.03.2000 US 523095; 17.04.2000 JP 2000115246; 20.10.2000 JP 2000321822
(43) Date of publication of application: 04.12.2002
(73) Proprietor: CHUGAI SEIYAKU KABUSHIKI KAISHA, Tokyo, 115-8543 (JP)
(72) Inventor: FUKUSHIMA, Naoshi, c/o CHUGAI SEIYAKU KABUSHIKI KAISHA, Gotemba-shi, Shizuoka 412-8513 (JP); TSUCHIYA, Masayuki, c/o CHUGAI SEIYAKU KABUSHIKI KAISHA, Gotemba-shi, Shizuoka 412-8513 (JP); OH-EDA, Masayoshi, c/o CHUGAI SEIYAKU KABUSHIKI KAISHA, Gotemba-shi, Shizuoka 412-8513 (JP); UNO, Shinsuke, c/o CHUGAI SEIYAKU KABUSHIKI KAISHA, Gotemba-shi, Shizuoka 412-8513 (JP); KIKUCHI, Yasufumi, c/o CHUGAI SEIYAKU KABUSHIKI KAISHA, Gotemba-shi, Shizuoka 412-8513 (JP)
(74) Representative: HOFFMANN EITLE
(86) International application number: PCT/JP2001/001912
(87) International publication number: WO 2001/066737

(56) References cited:
- EP-A1- 0 903 149
- WO-A1-00/53634
- WO-A1-99/12973
- US-A- 4 946 778
- MATEO V. ET AL.: 'CD47 ligation induces caspase-independent cell death in chronic lymphcytic leukemia' NATURE MEDICINE vol. 5, no. 11, 1999, pages 1277 - 1284, XP002942112
- PETTERSEN R.D. ET AL.: 'CD47 signals T cell death' J. IMMUNOL. vol. 162, no. 12, 15 June 1999, pages 7031 - 7040, XP002942113

## Description

### TECHNICAL FIELD

This invention relates to a reconstructed polypeptide with a property of inducing apoptosis of nucleated blood cells having Integrin Associated Protein (IAP) and causing no hemagglutination. More specifically, the present invention relates to the reconstructed polypeptides comprising two or more H chain v regions and two or more L chain V regions of a monoclonal antibody inducing apoptosis of nucleated blood cells having IAP. The reconstructed polypeptides are useful as a therapeutic agent for blood dyscrasia such as leukemia.

### BACKGROUND ART

Japanese Patent Application 9-67499 discloses the preparation of a specific monoclonal antibody using a splenic stromal cell line as a sensitizing antigen aiming at developing specific antibodies that can recognize the aforementioned splenic stromal cells and the preparation of novel monoclonal antibodies that recognize mouse Integrin Associated Protein (mouse IAP) as an antigen. JP-Appl. 9-67499 also discloses that the monoclonal antibodies are capable of inducing apoptosis of myeloid cells.

WO99/12973 discloses monoclonal antibodies whose antigen is human Integrin Associated Protein (hereinafter referred to as human IAP; amino acid sequence and nucleotide sequence thereof are described in J. Cell Biol., 123, 485-496, 1993; see also Journal of Cell Science, 108, 3419-3425, 1995) and which are capable of inducing apotosis of human nucleated blood cells (myeloid cell and lymphocyte) having said human IAP. These monoclonal antibodies are referred to antibody MABL-1 and antibody MABL-2, and hybridomas producing these antibodies are also referred to MABL-1 (FERM BP-6100) and MABL-2 (FERM BP-6101), respectively.

Japanese Patent Application 11-63557 describes the preparation of single-chain Fvs having single chain Fv regions from the monoclonal antibodies whose antigen is human IAP. The single-chain Fvs are capable of inducing apoptosis of nucleated blood cells having human IAP.

The monoclonal antibody recognizing IAP as an antigen induces apoptosis of nucleated blood cells having human IAP, but it also causes hemagglutination in vitro. It indicates that the administration of a large amount of the monoclonal antibody recognizing IAP as an antigen may result in a side effect such as hemagglutination.

### DISCLOSURE OF INVENTION

An object of this invention is to provide reconstructed polypeptides with improved property of inducing apoptosis of nucleated blood cells having Integrin Associated Protein (IAP) and with decreased or completely eliminated property of causing hemagglutination. Another object of the present invention is to provide therapeutic agents for blood dyscrasia comprising the substance obtained above which induces apoptosis of nucleated blood cells having Integrin Associated Protein (IAP).

Therefore, the present invention relates to the reconstructed polypeptides which binds to Integrin Associated Protein (IAP), induces apoptosis of nucleated blood cells having IAP and causes no hemagglutination.

The invention relates also to the reconstructed polypeptides, reshaped antibodies.

The reshaped antibody may include any reconstructed polypeptide which comprises an L chain V region and an H chain V region derived from a monoclonal antibody, e.g. antibody MABL-1, antibody MABL-2 or the like inducing apoptosis of nucleated blood cells having IAP, preferably human IAP, which polypeptide induces apoptosis of nucleated blood cells having IAP, preferably human IAP and causes no hemagglutination. Further, the present invention also includes reconstructed polypeptides wherein an amino acid sequence of the V region is partially altered.

The present invention also relates to the humanization of the reconstructed polypeptide. The humanized reconstructed polypeptide comprises a humanized L chain V region and/or a humanized H chain V region. Specifically, the humanized polypeptide of the invention consists of the humanized L chain V region which comprises a framework region (FR) derived from an L chain V region of human monoclonal antibody and an CDR derived from an L chain V region of mouse monoclonal antibody and/or the humanized H chain V region which comprises an FR derived from an H chain V region of human monoclonal antibody and a CDR derived from an H chain V region of mouse monoclonal antibody. In this case, the amino acid sequence of FR or CDR may be partially altered, e.g. deleted, replaced or added.

Furthermore, the present invention relates to reconstructed polypeptides capable of inducing apoptosis of nucleated blood cells having human IAP, which polypeptides comprise an L chain C region of human antibodies and an L chain V region of the mouse monoclonal antibodies, and/or an H chain C region of human antibodies and an H chain V region of the mouse monoclonal antibodies.

The present invention also relates to reconstructed polypeptides inducing apoptosis of nucleated blood cells having human IAP, which polypeptides comprise a CDR derived from a monoclonal antibody of other mammals than mouse such as human, rat, bovine, sheep or the like, which corresponds to the aforementioned mouse CDR, or an L chain V region and an H chain V region which contain the aforementioned CDR. Such CDRs, L chain V regions and H chain V regions may include CDRs derived from a human monoclonal antibody prepared from, for example, a transgenic mouse or the like, and L chain V regions and H chain V regions derived from a human monoclonal antibody containing the aforementioned CDR.

The invention also relates to DNAs encoding the various reconstructed polypeptides as aforementioned and genetic engineering techniques for the production of recombinant vectors comprising the DNAs.

The invention also relates to host cells transformed with the recombinant vectors. Examples of host cells mammalian cells such as human cells, mouse cells or the like and microorganisms such as E. coli, Bacillus subtilis, yeast or the like.

The invention relates to a process for producing the reconstructed polypeptides, which comprises culturing the above hosts and extracting the reconstructed polypeptides from the culture thereof.

The present invention further relates to a process for producing a dimer of the single-chain Fv which comprises culturing host mammalian cells producing the single-chain Fv in a serum-free medium to secrete the single-chain Fv into the medium and isolating the dimer of the single-chain Fv formed in the medium.

The present invention relates to therapeutic agents for blood dyscrasia comprising as an active ingredient the reconstructed polypeptide obtained in the above which induces apoptosis of nucleated blood cells having Integrin Associated Protein (IAP). The therapeutic agents for blood dyscrasia of the invention are useful for the treatment of blood dyscrasia, for example, leukemia such as acute myeloid leukemia, chronic myelogenous leukemia, acute lymphoblastic leukemia, chronic lymphoblastic leukemia, adult T-cell leukemia, multiple myeloma, mixed leukemia and hairy cell leukemia, malignant lymphoma (Hodgkin's disease, non-Hodgkin's lymphoma), aplastic anemia, myelodysplasia syndrome and polycythemia vera.

The reconstructed polypeptides of the present invention preferably comprise two H chain V regions and two L chain V regions derived from the monoclonal antibodies. The structure of the reconstructed polypeptides may be a dimer of single-chain Fv comprising one H chain V region and one L chain V region or a polypeptide comprising two H chain V regions and two L chain V regions. In the reconstructed polypeptides of the invention, the H chain V region and the L chain V region are preferably linked through a peptide linker which consists of one or more amino acids. The resulting reconstructed polypeptides contain variable regions of the parent antibodies and retain the complementarity determining region (CDR) thereof, and therefore bind to the antigen with the same specificity as that of the parent monoclonal antibodies.

### H chain V region

In the present invention, the H chain V region derived from the monoclonal antibody can be the H chain V region of the monoclonal antibody inducing apoptosis of nucleated blood cells having IAP or the H chain V region whose amino acid sequence is partially modified, preferably the H chain V region of the monoclonal antibody recognizing human IAP and inducing apoptosis of nucleated blood cells having IAP or the H chain V region whose amino acid sequence is partially modified. Preferable is the H chain V region derived from antibody MABL-1 or antibody MABL-2, or the H chain V region wherein the amino acid sequence of the H chain V region is partially modified. More preferable is humanized H chain V region comprising FR from an H chain V region of human monoclonal antibody and CDR from the H chain V region of mouse monoclonal antibody. The H chain V region further can be an H chain V region derived from a human monoclonal antibody corresponding to the aforementioned H chain V region of mouse monoclonal antibody, which can be produced by recombination technique. The H chain V region of the invention may be a fragment of aforementioned H chain V region, which fragment preserves the antigen binding capacity.

### L chain V region

The L chain V region of the present invention can be L chain V region of the monoclonal antibody inducing apoptosis of nucleated blood cells having IAP or the L chain V region wherein the amino acid sequences of the L chain V region is partially modified, preferably the L chin V region of the monoclonal antibody recognizing human IAP and inducing apoptosis of nucleated blood cells having IAP or the L chain V region whose amino acid sequences is partially modified. Preferable is L chain V region derived from antibody MABL-1 or antibody MABL-2, or its L chain V region whose amino acid sequence is partially modified. More preferable is humanized L chain V region comprising FR from L chain V region of human monoclonal antibody and CDR from the L chain v region of mouse monoclonal antibody. The L chain V regions further can be an L chain V region derived from human monoclonal antibody corresponding to the aforementioned L chain V region of mouse monoclonal antibody, which can be produced by recombination technique. The L chain V regions of the invention may be fragments of L chain V region, which fragments preserve the antigen binding capacity.

### Complementarity determining region (CDR)

Each V region of L chain and H chain forms an antigen-binding site. The variable region of the L and H chains is composed of comparatively conserved four common framework regions linked to three hypervariable regions or complementarity determining regions (CDR) (Kabat, E.A. et al., "sequence of Protein of Immunological Interest", US Dept. Health and Human Services, 1983).

Major portions in the four framework regions (FRs) form β-sheet structures and thus three CDRs form a loop. CDRs may form a part of the β-sheet structure in certain cases. The three CDRs are held sterically close position to each other by FR, which contributes to the formation of the antigen-binding site together with three CDRs.

These CDRs can be identified by comparing the amino acid sequence of V region of the obtained antibody with known amino acid sequences of V regions of known antibodies according to the empirical rule in Kabat, E.A. et al., "Sequence of Protein of Immunological Interest".

### Single-chain Fv

A single-chain Fv is a polypeptide monomer comprising an H chain V region and an L chain V region linked each other which are derived from monoclonal antibodies. The resulting single-chain Fvs contain variable regions of the parent monoclonal antibodies and preserve the complementarity determining region thereof, and therefore the single-chain Fvs bind to the antigen by the same specificity as that of the parent monoclonal antibodies (JP-Appl. 11-63557). A part of the variable region and/or CDR of the single-chain Fv of the invention or a part of the amino acid sequence thereof may be partially altered, for example deleted, replaced or added. The H chain V region and L chain V region composing the single-chain Fv of the invention are mentioned before and may be linked directly or through a linker, preferably a peptide linker. The constitution of the single-chain Fv may be [H chain V region]-[L chain V region] or [L chain V region]-[H chain V region]. In the present invention, the single-chain Fv can form a dimer, trimer or which can be tetramer included in the reconstructed polypeptide of the invention.

### Single-chain reconstructed polypeptide

The single-chain reconstructed polypeptides of the present invention comprising two or more H chain V regions and two or more L chain V regions comprise two or more H chain V regions and L chain V regions as mentioned above. Each region of the peptide should be arranged such that the reconstructed single chain polypeptide forms a specific steric structure, concretely mimicking a steric structure formed by the dimer of single-chain Fv. For instance, the V regions are arranged in the order of the following manner: [H chain V region]-[L chain V region]-[H chain V region]-[L chain V region]; or
[L chain V region]-[H chain V region]-[L chain V region]-[H chain V region],
wherein these regions are connected through a peptide linker, respectively.

### Liner

In this invention, the linkers for the connection between the H chain V region and the L chain V region may be any peptide linker which can be introduced by the genetic engineering procedure or any linker chemically synthesized. For instance, linkers disclosed in literatures, e.g. Protein Engineering, 9(3), 299-305, 1996 may be used in the invention. Examples of the peptide linkers may include, for example;
Ser
Gly-Ser
Gly-Gly-Ser
Ser-Gly-Gly
Gly-Gly-Gly-Ser
Ser-Gly-Gly-Gly
Gly-Gly-Gly-Gly-Ser
Ser-Gly-Gly-Gly-Gly
Gly-Gly-Gly-Gly-Gly-Ser
Ser-Gly-Gly-Gly-Gly-Gly
Gly-Gly-Gly-Gly-Gly-Gly-Ser
Ser-Gly-Gly-Gly-Gly-Gly-Gly
(Gly-Gly-Gly-Gly-Ser)ₙ and
(Ser-Gly-Gly-Gly-Gly),
wherein n is an integer not less than one. Length of the peptide linker is in the range of 1 to 15 amino acids, preferably 2 to 12 amino acids, more preferably 3 to 10 amino acids. Procedures for introducing these linkers are mentioned in Explanation of DNA encoding the reconstructed polypeptide or the invention.

The chemically synthesized linkers, i.e. the chemical crosslinking agents, according to the invention can be any linkers conventionally employed for the linkage of peptides. Examples of the linkers may include N-hydroxy succineimide (NHS), disuccinimidyl suberate (DSS), bis(sulfosuccinimidyl)suberate (BS³), dithiobis(succinimidyl propionate) (DSP), dithiobis(sulfosuccinimidyl propionate) (DTSSP), ethylene glycolbis(succinimidyl succinate) (EGS), ethylene glycolbis(sulfosuccinimidyl succinate) (sulfo-EGS), disuccinimidyl tartrate (DST), disulfosuccinimidyl tartrate (sulfo-DST), bis[2-(succinimido oxycarbonyloxy)ethyl]sulfone (BSOCOES), bis[2-(sulfosuccinimido oxycarbonyloxy) ethyl]sulfone (sulfo-BSOCOES) or the like. These are commercially available.

To form a dimer of the single-chain Fv it is preferable to select a linker suitable to dimerize in the solution such as culture medium more than 20%, preferably more than 50%, more preferably more than 80%, most preferably more than 90% of the single-chain Fv produced in the host cells. Specifically, preferable is a linker composed of 2 to 12 amino acids, preferably 3 to 10 amino acids or other linkers corresponding thereto.

### Preparation of reconstructed polypeptides

The reconstructed polypeptide binding to cells with human IAP are obtainable by connecting an H chain V region and an L chain V region derived from monoclonal antibodies against human IAP through the aforementioned linker. Examples of the single-chain Fvs are MABL1-scFv comprising the H chain V region and the L chain v region derived from the antibody MABL-1, and MABL2-scFv comprising the H chain V region and the L chain V region derived from the antibody MABL-2.

For the preparation of the reconstructed polypeptide, a signal peptide may be attached to N-terminal of the polypeptide where the polypeptide is desired to be a secretory peptide. A well-known amino acid sequence useful for the purification of polypeptide such as the FLAG sequence may be attached for the efficient purification of the polypeptide. The polypeptide can be efficiently purified with anti-FLAG antibody.

For the preparation of the reconstructed polypeptide of the invention, it is necessary to obtain a DNA encoding the reconstructed polypeptide, i.e. a DNA encoding the single-chain Fv or a DNA encoding the reconstructed polypeptide monomer. These DNAs are obtainable from the DNAs encoding the H chain V region and the L chain V region of MABL1-scFv and/or MABL2-scFv. They are also obtainable by amplifying a DNA encoding desired amino acid sequence within the aforementioned sequence by PCR using the DNA as a template and a pair of primers corresponding to both ends thereof.

In the case where each V region having partially modified amino acid sequence is desired, the V regions in which one or some amino acids are modified, i.e. deleted, replaced or added can be obtained by a procedure known in the art using PCR. A part of the amino acid sequence in the V region is preferably modified by the PCR known in the art in order to prepare the reconstructed polypeptide which is sufficiently active against the specific antigen.

For the determination of primers for the PCR amplification, it is necessary to decide the type of the H chain and L chain of the antibody MABL-1 and/or the antibody MABL-2. It has been reported, however, that the antibody MABL-1 has κ type L chains and γl type H chains and the antibody MABL-2 has κ type L chains and γ2a type H chains (JP-Appl. 11-63557). For the PCR amplification of the DNA encoding the H chain and L chain of the antibody MABL-1 and/or the antibody MABL-2, primers described in Jones, S.T. et al., Bio/Technology, 9, 88-89, 1991 may be employed.

For the amplification of the L chain V regions of the antibody MABL-1 and the antibody MABL-2 using the polymerase chain reaction (PCR), 5'-end and 3'-end oligonucleotide primers are decided as aforementioned. In the same manner, 5'-end and 3'-end oligonucleotide primers are decided for the amplification of the H chain V regions of the antibody MABL-1 and the antibody MABL-2.

In embodiments of the invention, the 5'-end primers which contain a sequence "GANTC" providing the restriction enzyme Hinf I digestion site at the neighborhood of 5'-terminal thereof are used and the 3'-end primers which contain a nucleotide sequence "CCCGGG" providing the XmaI digestion site at the neighborhood of 5'-terminal thereof are used. Other restriction enzyme digestion sites may be used instead of these sites as long as they are used for subcloning a desired DNA fragment into a cloning vector.

Specifically designed PCR primers are employed to provide suitable nucleotide sequences at 5'-end and 3'-end of the cDNAs encoding the V regions of the antibodies MABL-1 and MABL-2 so that the cDNAs are readily inserted into an expression vector and appropriately function in the expression vector (e.g. this invention devises to increase transcription efficiency by inserting Kozak sequence). The V regions of the antibodies MABL-1 and MABL-2 obtained by amplifying by PCR using these primers are inserted into HEF expression vector containing the desired human C region (see WO92/19759). The cloned DNAs can be sequenced by using any conventional process which comprises, for example, inserting the DNAs into a suitable vector and then sequencing using the automatic DNA sequencer (Applied Biosystems).

A linker such as a peptide linker can be introduced into the reconstructed polypeptide of the invention in the following manner. Primers which have partially complementary sequence with the primers for the H chain v regions and the L chain V regions as described above and which code for the N-terminal or the C-terminal of the linker are designed. Then, the PCR procedure can be carried out using these primers to prepare a DNA encoding the peptide linker having desired amino acid sequence and length. The DNAs encoding the H chain V region and the L chain v region can be connected through the resulting DNA to produce the DNA encoding the reconstructed polypeptide of the invention which has the desired peptide linker. Once the DNA encoding one of the reconstructed polypeptides is prepared, the DNAs encoding the reconstructed polypeptides with or without the desired peptide linker can readily be produced by designing various primers for the linker and then carrying out the PCR using the primers and the aforementioned DNA as a template.

Each V region of the reconstructed polypeptide of the present invention can be humanized by using conventional techniques (e.g. Sato, K. et al., Cancer Res., 53, 851-856 (1993)). Once a DNA encoding a humanized Fv is prepared, a humanized single-chain Fv, a fragment of the humanized single-chain Fv, a humanized monoclonal antibody and a fragment of the humanized monoclonal antibody can readily be produced according to conventional methods. Preferably, amino acid sequences of the V regions thereof may be partially modified, if necessary.

Furthermore, a DNA derived from other mammalian origin, for example a DNA of human, can be produced in the same manner as used to produce DNA encoding the H chain V region and the L chain V region derived from mouse mentioned in the above. The resulting DNA can be used to prepare an H chain V region and an L chain V region of other mammal, especially human origin, a single-chain Fv derived from human and a fragment thereof, and a monoclonal antibody of human origin and a fragment thereof.

As mentioned above, when the aimed DNAs encoding the V regions of the reconstructed polypeptides and the V regions the reconstructed humanized polypeptides are prepared, the expression vectors containing them and hosts transformed with the vectors can be obtained according to conventional methods. Further, the hosts can be cultured according to a conventional method to produce the reconstructed single-chain Fv, the reconstructed humanized single-chain Fv, the humanized monoclonal antibodies and fragments thereof. They can be isolated from cells or a medium and can be purified into a homogeneous mass. For this purpose any isolation and purification methods conventionally used for proteins, e.g. chromatography, ultra-filtration, salting-out and dialysis, may be employed in combination, if necessary, without limitation thereto.

When the reconstructed single-chain Fv of the present invention is produced by culturing an animal cell such as COS7 cells or CHO cells, preferably CHO cells, in a serum-free medium, the reconstructed single-chain Fv is efficiently dimerizedin the medium. The dimer of the single-chain Fv as formed above can be isolated stably and efficiently and preserved for a long period in the dimer form. The serum-free medium employed in the invention may be any medium conventionally used for the production of a recombinant protein without limit thereto.

For the production of the reconstructed polypeptides binding to cells with human IAP of the present invention, any expression systems can be employed, for example, eukaryotic cells such as animal cells, e.g., established mammalian cell lines, filamentous fungi and yeast, and prokaryotic cells such as bacterial cells e.g., E. coli. Preferably, the reconstructed polypeptides of the invention are expressed in mammalian cells, for example COS7 cells or CHO cells.

In these cases, conventional promoters useful for the expression in mammalian cells can be used. Preferably, human cytomegalovirus (HCMV) immediate early promoter is used. Expression vectors containing the HCMV promoter include HCMV-VH-HCγ 1, HCMV-VL-HCK and the like which are derived from pSV2neo (WO92/19759).

Additionally, other promoters for gene expression in mammal cell which may be used in the invention include virus promoters derived form retrovirus, polyoma virus, adenovirus and simian virus 40 (SV40) and promoters derived from mammal such as human polypeptide-chain elongation factor-1α (HEF-1α). SV40 promoter can easily be used according to the method of Mulligan, R.C., et al. (Nature 277, 108-114 (1979)) and HEF-la promoter can also be used according to the methods of Mizushima, S. et al. (Nucleic Acids Research, 18, 5322 (1990)).

Replication origin (ori) which can be used in the invention includes ori derived from SV40, polyoma virus, adenovirus, bovine papilloma virus (BPV) and the like. For the purpose of the amplification of gene copy number in the host cell system and the like, an expression vector may contain, as a selection marker, phosphotransferase APH (3') II or I (neo) gene, thymidine kinase (TK) gene, E. coli xanthine-guanine phosphoribosyl transferase (Ecogpt) gene or dihydrofolate reductase (DHFR) gene.

The antigen-binding activity of the reconstructed polypeptide as prepared above can be evaluated using the binding-inhibitory ability of the mouse antibodies, MABL-1 and MABL-2, to human IAP as an index. Concretely, the activity is evaluated in terms of the absence or presence of concentration-dependent inhibition of the binding of the mouse antibody MABL-2 to human IAP antigen as an index.

More in detail, animal cells transformed with an expression vector containing a DNA encoding the reconstructed polypeptide of the invention, e.g., COS7 cells or CHO cells, are cultured. The cultured cells and/or the supernatant of the medium or the reconstructed polypeptide purified from them are used to determine the binding to antigen. As a control, a supernatant of the cultural medium is used in which cells transformed only with the expression vector were cultured. A test sample of the reconstructed polypeptide of the invention or the supernatant of the control is added to mouse leukemia cell line, L1210 cells, expressing human Integrin Associated Protein (IAP) and then an assay such as the flow cytometry is carried out to evaluate the antigen-binding activity.

The apotosis-inducing effect in vitro is evaluated in the following manner: A test sample of the above reconstructed polypeptide is added to the cells into which the human IAP gene has been introduced and is evaluated on its inducibility of human IAP-specific cell death in the cells.

The apoptosis-inducing effect in vivo is evaluated in the following manner: A mouse model of human myeloma is prepared. To the mice is intravenously administered the monoclonal antibody or the reconstructed polypeptide of the invention, which induces apoptosis of nucleated blood cells having IAP, To mice of a control group is administered PBS alone. The induction of apoptosis is evaluated in terms of antitumor effect based on the change of human IgG content in serum of the mice and their survival time.

Hemagglutination effect is tested in the following manner: A suspension of erythrocytes is prepared from blood of healthy donors. Test samples of different concentrations are added to the suspension, which are then incubated to determine the hemagglutination.

Reconstructed polypeptides of the invention, which comprises two or more H chain v regions and two or more L chain V regions, may be a dimer, trimer or tetramer of the single-chain Fv comprising one H chain V region and one L chain V region, or a polypeptide in which two or more H chain V regions and two or more L chain V regions are connected. It is considered that owing to such construction the peptide mimics three dimensional structure of the antigen binding site of the parent monoclonal antibody and therefore retains an excellent antigen-binding property.

The polypeptide of the invention has a superior mobility to tissues or tumors over whole IgG and a remarkably reduced or no side effect of hemagglutination. Therefore, it is expected that the peptide of the invention can be used as a therapeutic agent for blood dyscrasia, for example, leukemia such as acute myeloid leukemia, chronic myelogenous leukemia, acute lymphoblastic leukemia, chronic lymphoblastic leukemia, adult T-cell leukemia, multiple myeloma, mixed leukemia and hairy cell leukemia, malignant lymphoma (Hodgkin's disease, non-Hodgkin's lymphoma), hypoplastic anemia, osteomyelodysplasia and polycythemia vera. It is further expected that the peptide of the invention can be used as a contrast agent by RI-labeling. The effect of the peptide can be enhanced by attaching to a RI-compound or a toxin.

### BEST MODE FOR WORKING THE INVENTION

The present invention will concretely be illustrated in reference to the following examples, which in no way limit the scope of the invention.

For illustrating the production process of the reconstructed polypeptides of the invention, examples of producing single-chain Fvs are shown below. Mouse antibodies against human IAP, MABL-1 and MABL-2 were used in the examples of producing the reconstructed polypeptides. Hybridomas MABL-1 and MABL-2 producing them respectively were internationally deposited as FERM BP-6100 and FERM BP-6101 with the National Institute of Bioscience and Human Technology, Agency of Industrial Science and Technology, Minister of International Trade and Industry (1-3 Higasi 1-chome, Tsukuba-shi, Ibaraki-ken, Japan), an authorized depository for microorganisms, on September 11, 1997.

### Example 1 (Cloning of DNAs encoding V region of mouse monoclonal antibodies to human IAP)

DNAs encoding variable regions of the mouse monoclonal antibodies to human IAP, MABL-1 and MABL-2, were cloned as follows.

### 1.1 Preparation of messenger RNA (maNA)

mRNAs of the hybridomas MABL-1 and MABL-2 were obtained by using mRNA Purification Kit (Pharmacia Biotech).

### 1.2 Synthesis of double-stranded cDNA

Double-stranded cDNA was synthesized from about 1 µg of the mRNA using Marathon cDNA Amplification Kit (CLONTECH) and an adapter was linked thereto.

### 1.3 PCR Amplification of genes encoding variable regions of an antibody by

PCR was carried out using Thermal Cycler (PERKIN ELMER ) .

### (1) Amplification of a gene coding for L chain V region of MABL-1

Primers used for the PCR method are Adapter Primer-1 (CLONTECH) shown in SEQ ID No. 1, which hybridizes to a partial sequence of the adapter, and MKC (Mouse Kappa Constant) primer (Bio/Technology, 9, 88-89, 1991) shown in SEQ ID No. 2, which hybridizes to the mouse kappa type L chain V region.

50 µl of the PCR solution contains 5 µl of 10 x PCR Buffer II, 2 mM MgCl₂, 0.16 mM dNTPs (dATP, dGTP, dCTP and dTTP), 2.5 units of a DNA polymerase, AmpliTaq Gold (PERKIN ELMER), 0.2 µM of the adapter primer of SEQ ID No. 1, 0.2 µMof the MKC primer of SEQ ID No. 2 and 0.1 µg of the double-stranded cDNA derived from MABL-1. The solution was preheated at 94°C of the initial temperature for 9 minutes and then heated at 94°C for 1 minute, at 60°C for 1 minute and at 72°C for 1 minute 20 seconds in order. This temperature cycle was repeated 35 times and then the reaction mixture was further heated at 72°C for 10 minutes.

### (2) Amplification of cDNA encoding H chain V region of MABL-1

The Adapter primer-1 shown in SEQ ID No. 1 and MHC-yl (Mouse Heavy Constant) primer (Bio/Technology, 9, 88-89,1991) shown in SEQ ID No. 3 were used as primers for PCR.

The amplification of cDNA was performed according to the method of the amplification of the L chain V region gene, which was described in Example 1.3-(1), except for using 0.2 µM of the MHC-γl primer instead of 0.2 µM of the MKC primer.

### (3) Amplification of cDNA encoding L chain V region of MABL-2

The Adapter Primer-1 of SEQ ID No. 1 and the MKC primer of SEQ ID No. 2 were used as primers for PCR.

The amplification of cDNA was carried out according to the method of the amplification of the L chain V region gene of MABL-1 which was described in Example 1.3-(1), except for using 0.1 µg of the double-stranded cDNA derived from MABL-2 instead of 0.1 µg of the double-stranded cDNA from MABL-1.

### (4) Amplification of cDNA encoding H chain V region of MAHL-2

The Adapter Primer-1 of SEQ ID No. 1 and MHC-γ2a primer (Bio/Technology, 9, 88-89, 1991) shown in SEQ ID No. 4 were used as primers for PCR.

The amplification of cDNA was performed according to the method of the amplification of the L chain V region gene, which was described in Example 1.3-(3), except for using 0.2 µM of the MHC-γ2a primer instead of 0.2 µM of the MKC primer.

### 1.4 Purification of PCR products

The DNA fragment amplified by PCR as described above was purified using the QIAquick PCR Purification Kit (QIAGEN) and dissolved in 10 mM Tris-HCl (pH 8.0) containing 1 mM EDTA.

### 1.5 Ligation and Transformation

About 140 ng of the DNA fragment comprising the gene encoding the mouse kappa type L chain V region derived from MABL-1 as prepared above was ligated with 50 ng of pGEM-T Easy vector (Promega) in the reaction buffer comprising 30 mM Tris-HCl (pH 7.8), 10 mM MgCl₂, 10 mM dithiothreitol, 1 mM ATP and 3 units of T4 DNA Ligase (Promega) at 15°C for 3 hours.

Then, 1 µl of the reaction mixture was added to 50 µl of E. coli DH5α competent cells (Toyobo Inc.) and the cells were stored on ice for 30 minutes, incubated at 42°C for 1 minute and stored on ice for 2 minutes again. 100 µl of SOC medium (GIBCO BRL) was added. The cells of E. coli were plated on LB (Molecular Cloning: A Laboratory Manual, Sambrook et al., Cold Spring Harbor Laboratory Press, 1989) agar medium containing 100 µg/ml of ampicillin (SIGMA) and cultured at 37°C overnight to obtain the transformant of E. coli.

The transformant was cultured in 3 ml of LB medium containing 50 µg/ml of ampicillin at 37°C overnight and the plasmid DNA was prepared from the culture using the QIAprep Spin Miniprep kit (QIAGEN).

The resulting plasmid comprising the gene encoding the mouse kappa type L chain V region derived from the hybridoma MABL-1 was designated as pGEM-M1L.

According to the same manner as described above, a plasmid comprising the gene encoding the mouse H chain V region derived from the hybridoma MABL-1 was prepared from the purified DNA fragment and designated as pGEM-M1H.

A plasmid comprising the gene encoding the mouse kappa type L chain V region derived from the hybridoma MABL-2 was prepared from the purified DNA fragment and designated as pGEM-M2L.

A plasmid comprising the gene encoding the mouse H chain V region derived from the hybridoma MABL-2 was prepared from the purified DNA fragment and designated as pGEM-M2H.

### Example 2 (DNA Sequencing)

The nucleotide sequence of the cDNA encoding region in the aforementioned plasmids was determined using Auto DNA Sequencer (Applied Biosystem) and ABI PRISM Dye Terminator Cycle Sequencing Ready Reaction Kit (Applied Biosystem) according to the manufacturer's protocol.

The nucleotide sequence of the gene encoding the L chain V region from the mouse antibody MABL-1, which is included in the plasmid pGEM-MlL, is shown in SEQ ID No. 5.

The nucleotide sequence of the gene encoding the H chain V region from the mouse antibody MABL-1, which is included in the plasmid pGEM-M1H, is shown in SEQ ID No. 6.

The nucleotide sequence of the gene encoding the L chain V region from the mouse antibody MABL-2, which is included in the plasmid pGEM-M2L, is shown in SEQ ID No. 7.

The nucleotide sequence of the gene encoding the H chain V region from the mouse antibody MABL-2, which is included in the plasmid pGEM-M2H, is shown in SEQ ID No. 8.

### Example 3 (Determination of CDR)

The V regions of L chain and H chain generally have a similarity in their structures and each four framework regions therein are linked by three hypervariable regions, i.e., complementarity determining regions (CDR). An amino acid sequence of the framework is relatively well conserved, while an amino acid sequence of CDR has extremely high variation (Kabat, E.A., et al., "Sequences of Proteins of Immunological Interest", US Dept. Health and human Services, 1983).

On the basis of these facts, the amino acid sequences of the variable regions from the mouse monoclonal antibodies to human IAP were applied to the database of amino acid sequences of the antibodies made by Kabat et al. to investigate the homology. The CDR regions were determined based on the homology as shown in Table 1.

**Table 1**

| Plasmid | SEQ ID NO. | CDR(1) | CDR(2) | CDR (3) |
|---|---|---|---|---|
| pGEM-M1L | 5 | 43-58 | 74-80 | 113-121 |
| pGEM-M1H | 6 | 50-54 | 69-85 | 118-125 |
| pGEM-M2L | 7 | 43-58 | 74-80 | 113-121 |
| pGEM-M2H | 8 | 50-54 | 69-85 | 118-125 |

### Example 4 (Identification of Cloned cDNA Expression

### (Preparation of Chimera MABL-1 antibody and Chimera MABL-2 antibody.)

### 4.1 Preparation of vectors expressing chimera MABL-1 antibody

cDNA clones, pGEM-M1L and pGEM-M1H, encoding the V regions of the L chain and the H chain of the mouse antibody MABL-1, respectively, were modified by the PCR method and introduced into the HEF expression vector (WO92/19759) to prepare vectors expressing chimera MABL-1 antibody.

A forward primer MLS (SEQ ID No. 9) for the L chain V region and a forward primer MHS (SEQ ID No. 10) for the H chain V region were designed to hybridize to a DNA encoding the beginning of the leader sequence of each V region and to contain the Kozak consensus sequence (J. Mol. Biol., 196, 947-950, 1987) and HindIII restriction enzyme site. A reverse primer MLAS (SEQ ID No. 11) for the L chain V region and a reverse primer MHAS (SEQ ID No. 12) for the H chain V region were designed to hybridize to a DNA encoding the end of the J region and to contain the splice donor sequence and BamHI restriction enzyme site.

100 µl of a PCR solution comprising 10 µl of 10 × PCR Buffer II, 2 mM MgCl₂, 0.16 mM dNTPs (dATP, dGTP, dCTP and dTTP), 5 units of DNA polymerase AmpliTaq Gold, 0.4 µM each of primers and 8 ng of the template DNA (pGEM-M1L or pGEM-M1H) was preheated at 94°C of the initial temperature for 9 minutes and then heated at 94°C for 1 minute, at 60°C for 1 minute and at 72°C for 1 minute 20 seconds in order. This temperature cycle was repeated 35 times and then the reaction mixture was further heated at 72°C for 10 minutes.

The PCR product was purified using the QIAquick PCR Purification Kit (QIAGEN) and then digested with HindIII and BamHI. The product from the L chain V region was cloned into the HEF expression vector, HEF-κ and the product from the H chain V region was cloned into the HEF expression vector, HEF-γ. After DNA sequencing, plasmids containing a DNA fragment with a correct DNA sequence are designated as HEF-M1L and HEF-M1H, respectively.

### 4.2 Preparation of vectors expressing chimera MABL-2 antibodies

Modification and cloning of cDNA were performed in the same manner described in Example 4.1 except for using pGEM-M2L and pGEM-M2H as template DNA instead of pGEM-M1L and pGEM-M1H. After DNA sequencing, plasmids containing DNA fragments with correct DNA sequences are designated as HEF-M2L and HEF-M2H, respectively.

### 4.3 Transfection to COS7 cells

The aforementioned expression vectors were tested in COS7 cells to observe the transient expression of the chimera MABL-1 and MABL-2 antibodies.

### (1) Transfection with genes for the chimera MABL-1 antibody

COS7 cells were co-transformed with the HEF-M1L and HEF-M1H vectors by electroporation using the Gene Pulser apparatus (BioRad). Each DNA (10 µg) and 0.8 ml of PBS with 1 × 10⁷ cells/ml were added to a cuvette. The mixture was treated with pulse at 1.5 kV, 25 µF of electric capacity.

After the restoration for 10 minutes at a room temperature, the electroporated cells were transferred into DMEM culture medium (GIBCO BRL) containing 10% γ-globulin-free fetal bovine serum. After culturing for 72 hours, the supernatant was collected, centrifuged to remove cell fragments and recovered.

### (2) Transfection with genes coding for the chimera MABL-2 antibody

The co-transfection to COS7 cells with the genes coding for the chimera MABL-2 antibody was carried out in the same manner as described in Example 4.3-(1) except for using the HEF-M2L and HEF-M2H vectors instead of the HEF-M1L and HEF-M1H vectors. The supernatant was recovered in the same manner.

### 4.4 Flow cytometry

Flow cytometry was performed using the aforementioned culture supernatant of COS7 cells to measure binding to the antigen. The culture supernatant of the COS7 cells expressing the chimera MABL-1 antibody or the COS7 cells expressing the chimera MABL-2 antibody, or human IgG antibody (SIGMA) as a control was added to 4 × 10⁵ cells of mouse leukemia cell line L1210 expressing human IAP and incubated on ice. After washing, the FITC-labeled anti-human IgG antibody (Cappel) was added thereto. After incubating and washing, the fluorescence intensity thereof was measured using the FACScan apparatus (BECTON DICKINSON).

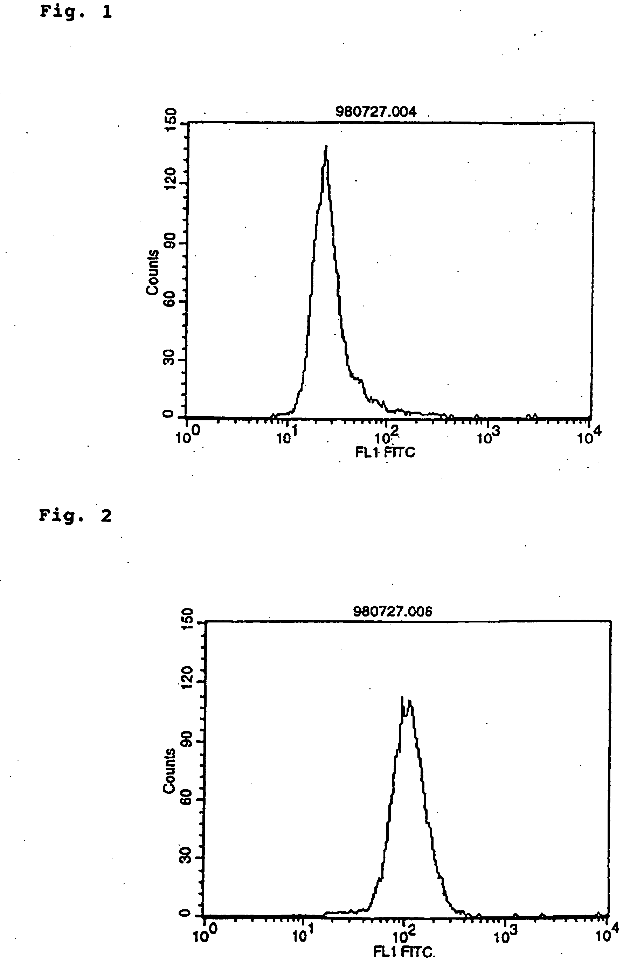

Since the chimera MABL-1 and MABL-2 antibodies were specifically bound to L1210 cells expressing human IAP, it is confirmed that these chimera antibodies have proper structures of the V regions of the mouse monoclonal antibodies MABL-1 and MABL-2, respectively (Figures 1-3). Example 5 (Preparation of reconstructed single-chain Fv (SCFV) of the antibody MABL-1 and antibody MABL-2)

### 5.1 Preparation of reconstructed single-chain Fv of antibody MABL-1

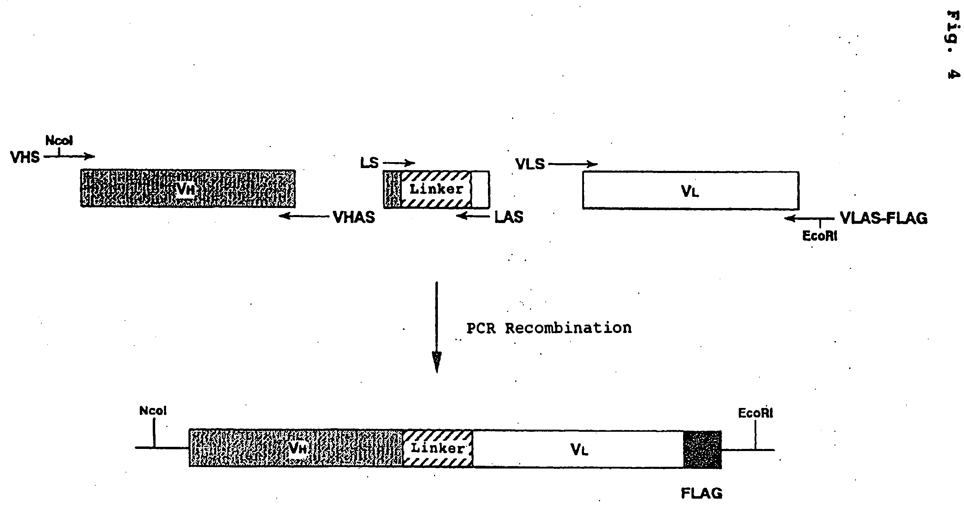

The reconstructed single-chain Fv of antibody MABL-1 was prepared as follows. The H chain V region and the L chain V of antibody MABL-1, and a linker were respectively amplified by the PCR method and were connected to produce the reconstructed single-chain Fv of antibody MABL-1. The production method is illustrated in Figure 4. Six primers (A-F) were employed for the production of the single-chain Fv of antibody MABL-1. Primers A, C and E have a sense sequence and primers B, D and F have an antisense sequence.

The forward primer VHS for the H chain V region (Primer A, SEQ ID No. 13) was designed to hybridize to a DNA encoding the N-terminal of the H chain V region and to contain NcoI restriction enzyme recognition site. The reverse primer VHAS for H chain V region (Primer B, SEQ ID No. 14) was designed to hybridize to a DNA coding the C-terminal of the H chain V region and to overlap with the linker.

The forward primer LS for the linker (Primer C, SEQ ID No. 15) was designed to hybridize to a DNA encoding the N-terminal of the linker and to overlap with a DNA encoding the C-terminal of the H chain V region. The reverse primer LAS for the linker (Primer D, SEQ ID No. 16) was designed to hybridize to a DNA encoding the C-terminal of the linker and to overlap with a DNA encoding the N-terminal of the L chain V region.

The forward primer VLS for the L chain V region (Primer E,SEQ_{.}ID No. 17) was designed to hybridize to a DNA encoding the C-terminal of the linker and to overlap with a DNA encoding the N-terminal of the L chain V region. The reverse primer VLAS-FLAG for L chain V region (Primer F, SEQ ID No. 18) was designed to hybridize to a DNA encoding the C-terminal of the L chain V region and to have a sequence encoding the FLAG peptide (Hopp. T. P. et al., Bio/Technology, 6, 1204-1210, 1988), two stop codons and EcoRI restriction enzyme recognition site.

In the first PCR step, three reactions, A-B, C-D and E-F, were carried out and PCR products thereof were purified. Three PCR products obtained from the first PCR step were assembled by their complementarity. Then, the primers A and F were added and the full length DNA encoding the reconstructed single-chain Fv of antibody MABL-1 was amplified (Second PCR). In the first PCR, the plasmid PGEM-M1H encoding the H chain V region of antibody MABL-1 (see Example 2), a plasmid pSC-DP1 which comprises a DNA sequence encoding a linker region comprising: Gly Gly Gly Gly Ser Gly Gly Gly Gly Ser Gly Gly Gly Gly Ser (SEQ ID No. 19) (Huston, J.S., et al., Proc. Natl. Acad. Sci. USA, 85, 5879-5883, 1988) and the plasmid pGEM-M1L encoding the L chain V region of antibody NABL-1 (see Example 2) were employed as template, respectively.

50 µl of the solution for the first PCR step comprises 5 µl of 10 × PCR Buffer II, 2 mM MgCl₂, 0.16 mM dNTPs, 2.5 units of DNA polymerase, AmpliTaq Gold (PERKIN ELMER), 0.4 µM each of primers and 5 ng each of template DNA. The PCR solution was preheated at 94°C of the initial temperature for 9 minutes and then heated at 94°C for 1 minute, at 65°C for 1 minute and at 72°C for 1 minute and 20 seconds in order. This temperature cycle was repeated 35 times and then the reaction mixture was further heated at 72°C for 7 minutes.

The PCR products A-B (371bp), C-D (63bp) and E-F (384bp) were purified using the QIAquick PCR Purification Kit (QIAGEN) and were assembled in the second PCR. In the seconds PCR, 98 µl of a PCR solution comprising 120 ng of the first PCR product A-B, 20 ng of the PCR product C-D and 120 ng of the PCR product E-F, 10 µl of 10 × PCR Buffer II, 2mM MgCl₂, 0.16 mM dNTPs, 5 units of DNA polymerase AmpliTaq Gold (PERKIN ELMER) was preheated at 94°C of the initial temperature for 8 minutes and then heated at 94°C for 2 minutes, at 65°C for 2 minutes and at 72°C for 2 minutes in order. This temperature cycle was repeated twice and then 0.4 µM each of primers A and F were added into the reaction, respectively. The mixture was preheated at 94°C of the initial temperature for 1 minutes and then heated at 94°C for 1 minute, at 65°C for 1 minute and at 72°C for 1 minute and 20 seconds in order. This temperature cycle was repeated 35 times and then the reaction mixture was further heated at 72°C for 7 minutes.

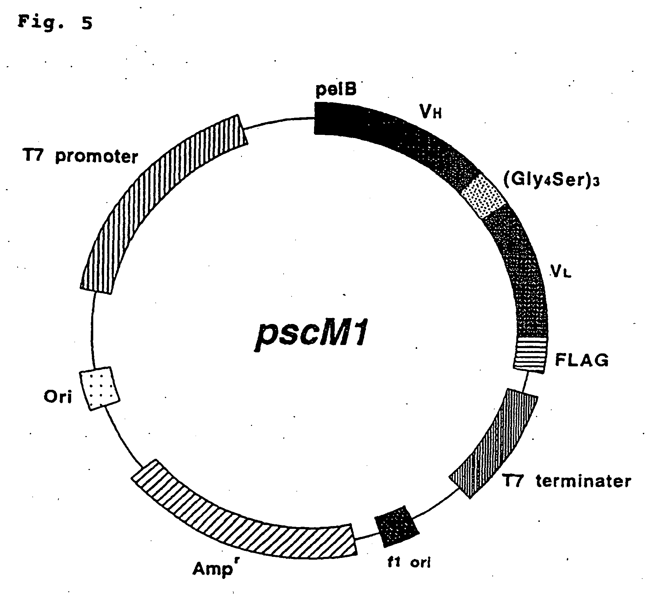

A DNA fragment of 843 bp produced by the second PCR was purified and digested by NcoI and EcoRI. The resultant DNA fragment was cloned into pSCFVT7 vector. The expression vector pSCFVT7 contains a pelB signal sequence suitable for E. coli periplasmic expression system (Lei, S.P., et al., J. Bacteriology, 169, 4379-4383, 1987). After the DNA sequencing, the plasmid containing the DNA fragment encoding correct amino acid sequence of the reconstructed single-chain Fv of antibody MABL-1 is designated as "pscM1" (see Figure 5). The nucleotide sequence and the amino acid sequence of the reconstructed single-chain Fv of antibody MABL-1 contained in the plasmid pscM1 are shown in SEQ ID No. 20.

The pscM1 vector was modified by the PCR method to prepare a vector expressing the reconstructed single-chain Fv of antibody MABL-1 in mammalian cells. The resultant DNA fragment was introduced into pCHO1 expression vector. This expression vector, pCHO1, was constructed by digesting DHFR-ΔE-rvH-PM1-f (WO92/19759) with EcoRI and SmaI to eliminate the antibody gene and connecting the EcoRI-NotI-BamHI Adapter (Takara Shuzo) thereto.

As a forward primer for PCR, Sal-VHS primer shown in SEQ ID No. 21 was designed to hybridize to a DNA encoding the N-terminal of the H chain V region and to contain SalI restriction enzyme recognition site. As a reverse primer for PCR, FRH1anti primer shown in SEQ ID No. 22 was designed to hybridize to a DNA encoding the end of the first framework sequence.

100 µl of PCR solution comprising 10 µl of 10 × PCR Buffer II, 2 mM MgCl₂, 0.1,6 mM dNTPs, 5 units of the DNA polymerase, AmpliTaq Gold, 0.4 µl M each of primer and 8 ng of the template DNA (pscM1) was preheated at 95°C of the initial temperature for 9 minutes and then heated at 95°C for 1 minute, at 60°C for 1 minute and at 72°C for 1 minute and 20 seconds in order. This temperature cycle was repeated 35 times and then the reaction mixture was further heated at 72°C for 7 minutes.

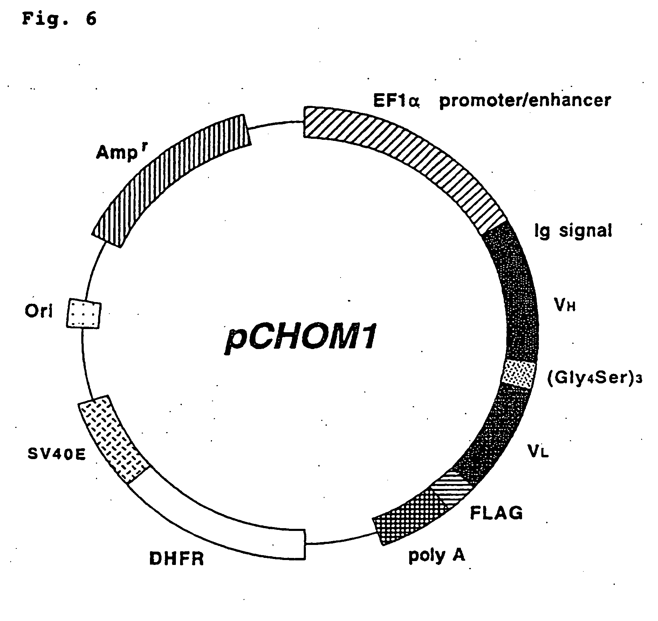

The PCR product was purified using the QIAquick PCR Purification Kit (QIAGEN) and digested by SalI and MboII to obtain a DNA fragment encoding the N-terminal of the reconstructed single-chain Fv of antibody MABL-1 The pscM1 vector was digested by MboII and EcoRI to obtain a DNA fragment encoding the C-terminal of the reconstructed single-chain Fv of antibody MABL-1. The SalI-MboII DNA fragment and the MboII-EcoRI DNA fragment were cloned into pCHO1-Igs vector. After DNA sequencing, the plasmid comprising the desired DNA sequence was designated as "pCHOM1" (see Figure 6). The expression vector, pCHO1-Igs, contains a mouse IgG1 signal sequence suitable for the secretion-expression system in mammalian cells (Nature, 322, 323-327, 1988). The nucleotide sequence and the amino acid sequence of the reconstructed single-chain Fv of antibody MABL-1 contained in the plasmid pCHOM1 are shown in SEQ ID No. 23.

### 5.2 Preparation of reconstructed single-chain Fv of antibody MAHL-2

The reconstructed single-chain Fv of antibody MABL-2 was prepared in accordance with the aforementioned Example 5.1. Employed in the first PCR step were plasmid pGEM-M2H encoding the H chain V region of MABL-2 (see Example 2) instead of pGEM-M1H and plasmid pGEM-M2L encoding the L chain V region of MABL-2 (see Example 2) instead of pGEM-M1L, to obtain a plasmid pscM2 which comprises a DNA fragment encoding the desired amino acid sequence of the single-chain Fv of antibody MABL-2. The nucleotide sequence and the amino acid sequence of the reconstructed single-chain Fv of antibody MABL-2 contained in the plasmid pscM2 are shown in SEQ ID No. 24.

The pscM2 vector was modified by the PCR method to prepare a vector, pCHOM2, for the expression in mammalian cells which contains the DNA fragment encoding the correct amino acid sequence of reconstructed the single-chain Fv of antibody MABL-2. The nucleotide sequence and the amino acid sequence of the reconstructed single-chain Fv of antibody MABL-2 contained in the plasmid pCHOM2 are shown in SEQ ID No. 25.

### 5.3 Transfection to COS7 cells

The pCHOM2 vector was tested in COS7 cells to observe the transient expression of the reconstructed single-chain Fv of antibody MABL-2.

The COS7 cells were transformed with the pCHOM2 vector by electroporation using the Gene Pulser apparatus (BioRad). The DNA (10 µg) and 0.8 ml of PBS with 1 ×10⁷ cells/ml were added to a cuvette. The mixture was treated with pulse at 1.5 kV, 25 µF of electric capacity.

After the restoration for 10 minutes at a room temperature, the electroporated cells were transferred into IMDM culture medium (GIBCO BRL) containing 10% fetal bovine serum. After culturing for 72 hours, the supernatant was collected, centrifuged to remove cell fragments and recovered.

### 5.4 Detection of the reconstructed single-chain Fv of antibody MABL-2 in culture supernatant of COS7 cells

The existence of the single-chain Fv of antibody MABL-2 in the culture supernatant of COS7 cells which had been transfected with the pCHOM2 vector was confirmed by the Western Blotting method.

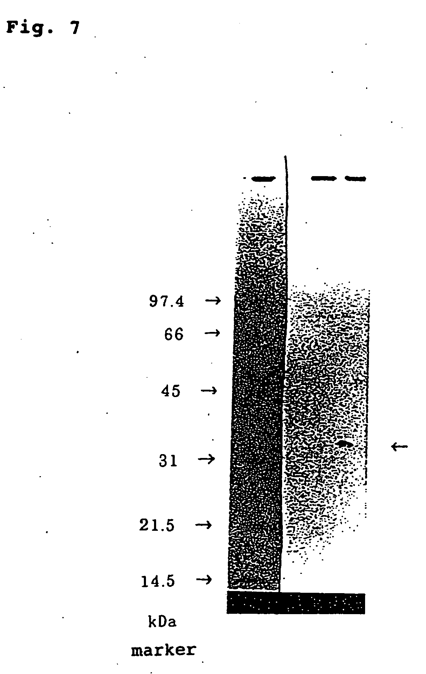

The culture supernatant of COS7 cells transfected with the pCHOM2 vector and the culture supernatant of COS7 cells transfected with the pCHO1 as a control were subjected to SDS electrophoresis and transferred to REINFORCED NC membrane (Schleicher & schuell). The membrane was blocked with 5% skim milk (Morinaga Nyu-gyo), washed with 0.05% Tween 20-PBS and mixed with an anti-FLAG antibody (SIGMA). The membrane was incubated at room temperature, washed and mixed with alkaline phosphatase-conjugated mouse IgG antibody (Zymed). After incubating and washing at room temperature, the substrate solution (Kirkegaard Perry Laboratories) was added to develop color (Figure 7).

A FLAG-peptide-specific protein was detected only in the culture supernatant of the pCHOM2 vector-introduced COS7 cells and thus it is confirmed that the reconstructed single-chain Fv of antibody MABL-2 was secreted in this culture supernatant.

### 5.5 Flow cytometry

Flow cytometry was performed using the aforementioned COS7 cells culture supernatant to measure the binding to the antigen. The culture supernatant of the COS7 cells expressing the reconstructed single-chain Fv of antibody MABL-2 or the culture supernatant of COS7 cells transformed with pCHO1 vector as a control was added to 2 × 10⁵ cells of the mouse leukemia cell line L1210 expressing human Integrin Associated Protein (IAP) or the cell line L1210 transformed with pCOS1 as a control. After incubating on ice and washing, the mouse anti-FLAG antibody (SIGMA) was added. Then the cells were incubated and washed. Then, the FITC labeled anti-mouse IgG antibody (BECTON DICKINSON) was added thereto and the cells were incubated and washed again. Subsequently, the fluorescence intensity was measured using the FACScan apparatus (BECTON DICKINSON).

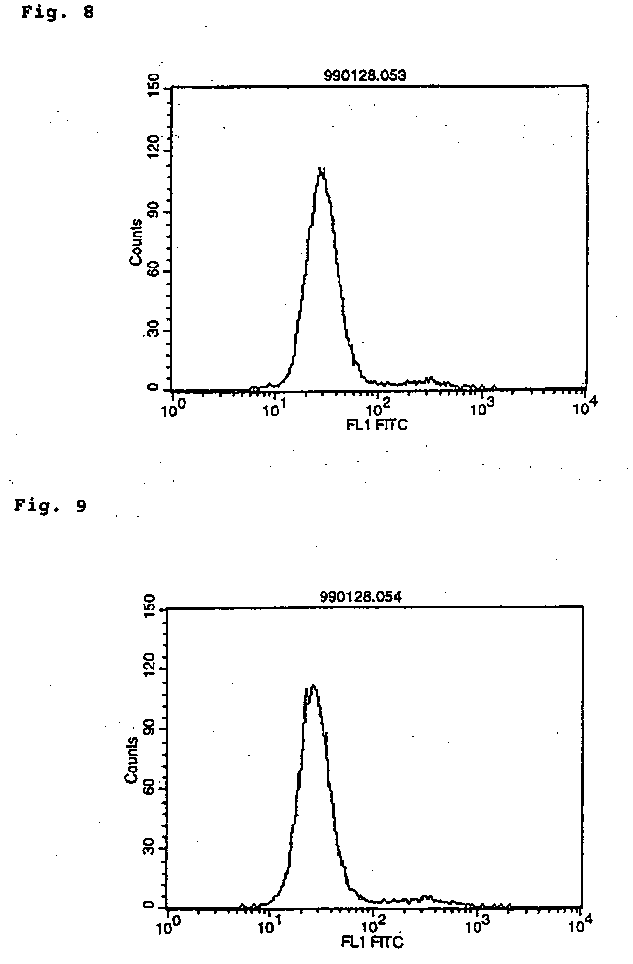

Since the single-chain Fv of antibody MABL-2 was specifically bound to L1210 cells expressing human IAP, it is confirmed that the reconstructed single-chain Fv of antibody MABL-2 has an affinity to human Integrin Associated Pro
tein (IAP) (see Figures 8-11).

### 5.6 Competitive ELISA

The binding activity of the reconstructed single-chain Fv of antibody MABL-2 was measured based on the inhibiting activity against the binding of mouse monoclonal antibodies to the antigen.

The anti-FLAG antibody adjusted to 1 µg/ml was added to each well on 96-well plate and incubated at 37°C for 2 hours. After washing, blocking was performed with 1% BSA-PBS. After incubating and washing at a room temperature, the culture supernatant of COS7 cells into which the secretion-type human IAP antigen gene (SEQ ID No. 26) had been introduced was diluted with PBS into twofold volume and added to each well. After incubating and washing at a room temperature, a mixture of 50 µl of the biotinized MABL-2 antibody adjusted to 100 ng/ml and 50 µl of sequentially diluted supernatant of the COS7 cells expressing the reconstructed single-chain Fv of antibody MABL-2 were added into each well. After incubating and washing at a room temperature, the alkaline phosphatase-conjugated streptoavidin (Zymed) was added into each well. After incubating and washing at a room temperature, the substrate solution (SIGMA) was added and absorbance of the reaction mixture in each well was measured at 405 nm.

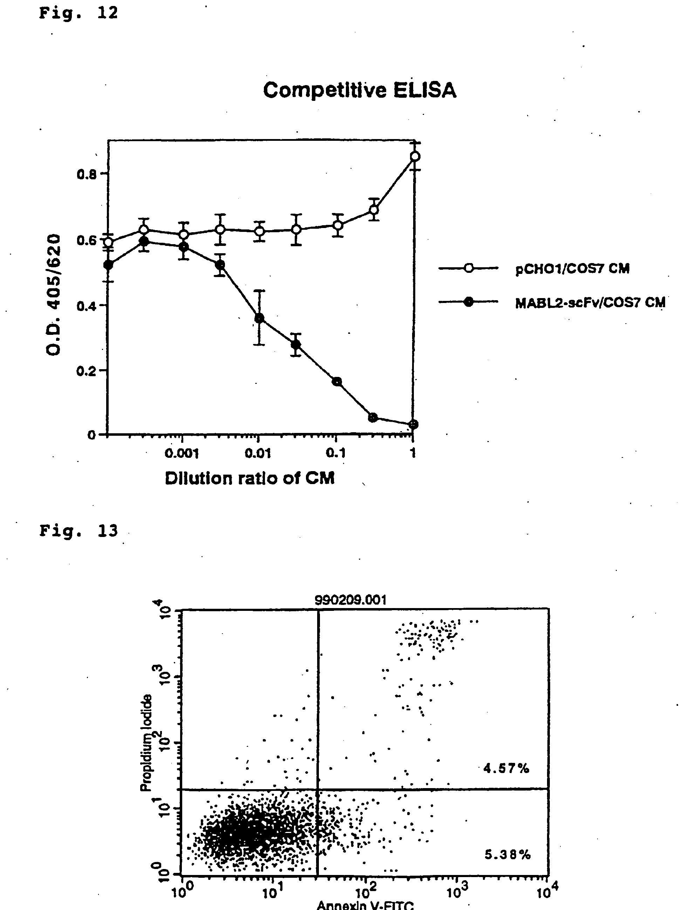

The results revealed that the reconstructed single-chain Fv of antibody MABL-2 (MABL2-scFv) evidently inhibited concentration-dependently the binding of the mouse antibody MABL-2 to human IAP antigen in comparison with the culture supernatant of the PCHO1-introduced COS7 cells as a control (Figure 12). Accordingly, it is suggested that the reconstructed single-chain Fv of antibody MABL-2 has the correct structure of each of the V regions from the mouse monoclonal antibody MABL-2.

### 5.7 Apoptosis-inducing Effect in vitro

An apoptosis-inducing action of the reconstructed single-chain Fv of antibody MABL-2 was examined by Annexin-V staining (Boehringer Mannheim) using the L1210 cells transfected with human IAP gene, the L1210 cells transfected with the pCOS1 vector as a control and CCRF-CEM cells.

To each 1 × 10⁵ cells of the above cells was added the culture supernatant of the COS7 cells expressing the reconstructed single-chain Fv of antibody MABL-2 or the culture supernatant of COS7 cells transfected with the pCHO1 vector as a control at 50% final concentration and the mixtures were cultured for 24 hours. Then, the Annexin-V staining was performed and the fluorescence intensity was measured using the FACScan apparatus (BECTON DICKINSON).

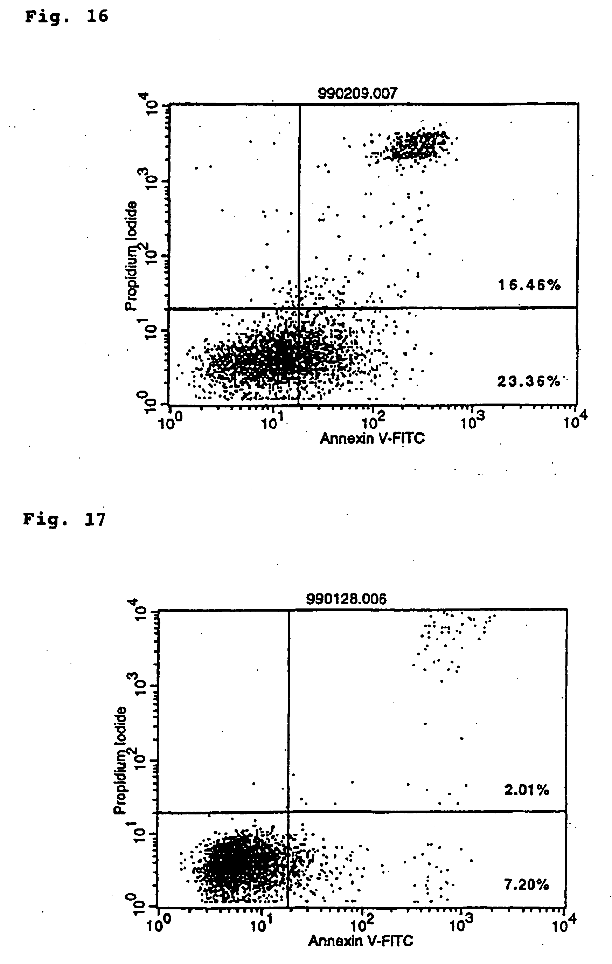

Results of the Annexin-V staining are shown in Figures 13-18, respectively. Dots in the left-lower region represent living cells and dots in the right-lower region represent cells at the early stage of apoptosis and dots in the right-upper region represent cells at the late stage of apoptosis. The results show that the reconstructed single-chain Fv of antibody MABL-2 (MABL2-scFv) remarkably induced cell death of L1210 cells specific to human IAP antigen (Figures 13-16) and that the reconstructed single-chain Fv also induced remarkable cell death of CCRF-CEM cells in comparison with the control (Figures 17-18).

### 5.8 Expression of MABL-2 derived single-chain Fv in CHO cells

CHO cells were transfected with the pCHOM2 vector to establish a CHO cell line which constantly expresses the single-chain Fv (polypeptide) derived from the antibody MABL-2.

CHO cells were transformed with the pCHOM2 vector by the electroporation using the Gene Pulser apparatus (BioRad). A mixture of DNA (10 µg) and 0.7 ml of PBS with CHO cells (1 × 10⁷ cells/ml) was added to a cuvette. The mixture was treated with pulse at 1.5 kV, 25 µF of electric capacity. After the restoration for 10 minutes at a room temperature, the electroporated cells were transferred into nucleic acid free α-MEM medium (GIBCO BRL) containing 10% fetal bovine serum and cultured. The expression of desired protein in the resultant clones was confirmed by SDS-PAGE and a clone with a high expression level was selected as a cell line producing the single-chain Fv derived from the antibody MABL-2. The cell line was cultured in serum free medium CHO-S-SFM II (GIBCO BRL) containing 10 nM methotrexate (SIGMA). Then, the culture supernatant was collected, centrifuged to remove cell fragments and recovered.

### 5.9 Purification of MABL-2 derived single-chain Fv produced in CHO cells

The culture supernatant of the CHO cell line expressing the single-chain Fv obtained in Example 5.8 was concentrated up to twenty times using a cartridge for the artificial dialysis (PAN130SF, ASAHI MEDICALS). The concentrated solution was stored at -20°C and thawed on purification.

Purification of the single-chain Fv from the culture supernatant of the CHO cells was performed using three kinds of chromatography, i.e., Blue-sepharose, a hydroxyapatite and a gel filtration.

### (1) Blue-sepharose column chromatography

The concentrated supernatant was diluted to ten times with 20 mM acetate buffer (pH 6.0) and centrifuged to remove insoluble materials (10000 × rpm, 30 minutes). The supernatant was applied onto a Blue-sepharose column (20 ml) equilibrated with the same buffer. After washing the column with the same buffer, proteins adsorbed in the column were eluted by a stepwide gradient of NaCl in the same buffer, 0.1, 0.2, 0.3, 0.5 and up to 1.0 M. The pass-through fraction and each eluted fraction were analyzed by SDS-PAGE. The fractions in which the single-chain Fv were confirmed (the fractions eluted at 0.1 to 0.3M NaCl) were pooled and concentrated up to approximately 20 times using CentriPrep-10 (AMICON).

### (2) Hydroxyapatite

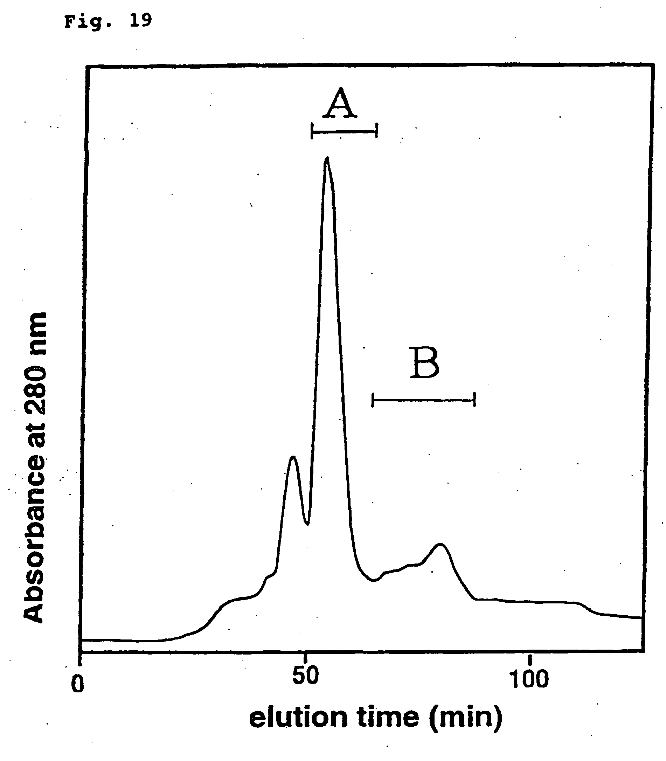

The concentrated solution obtained in (1) was diluted to 10 times with 10 mM phosphate buffer (pH 7.0) and applied onto the hydroxyapatite column (20 ml, BIORAD). The column was washed with 60 ml of 10 mM phosphate buffer (pH 7.0). Then, proteins adsorbed in the column were eluted by a linear gradient of sodium phosphate buffer up to 200 mM (see Figure 19). The analysis of each fraction by SDS-PAGE confirmed the single-chain Fv in fraction A and fraction B.

### (3) Gel filtration

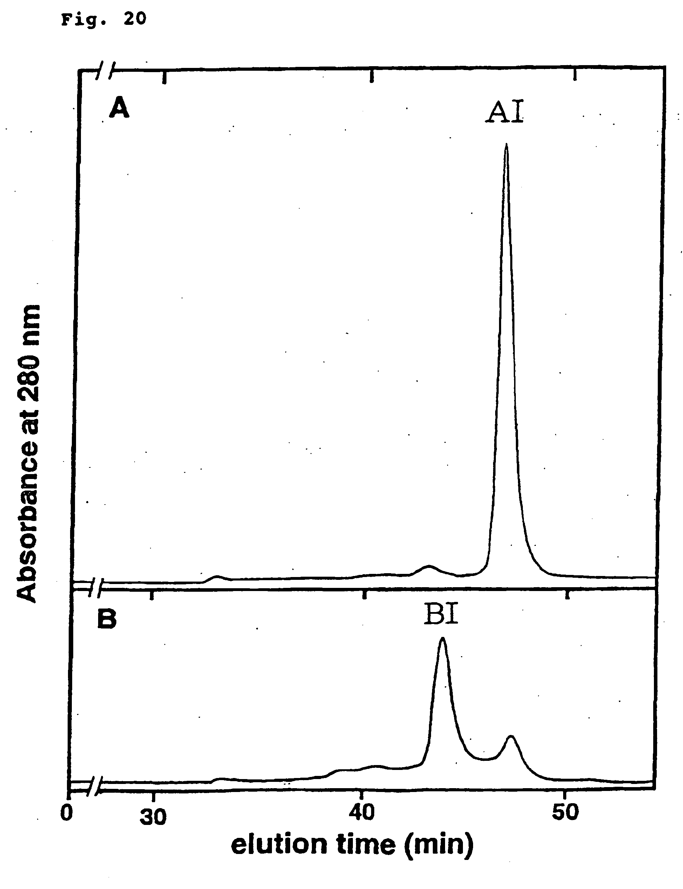

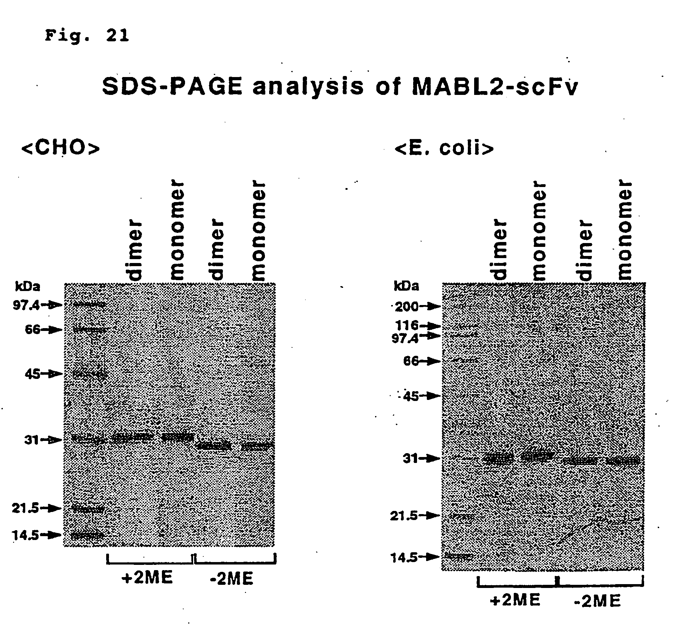

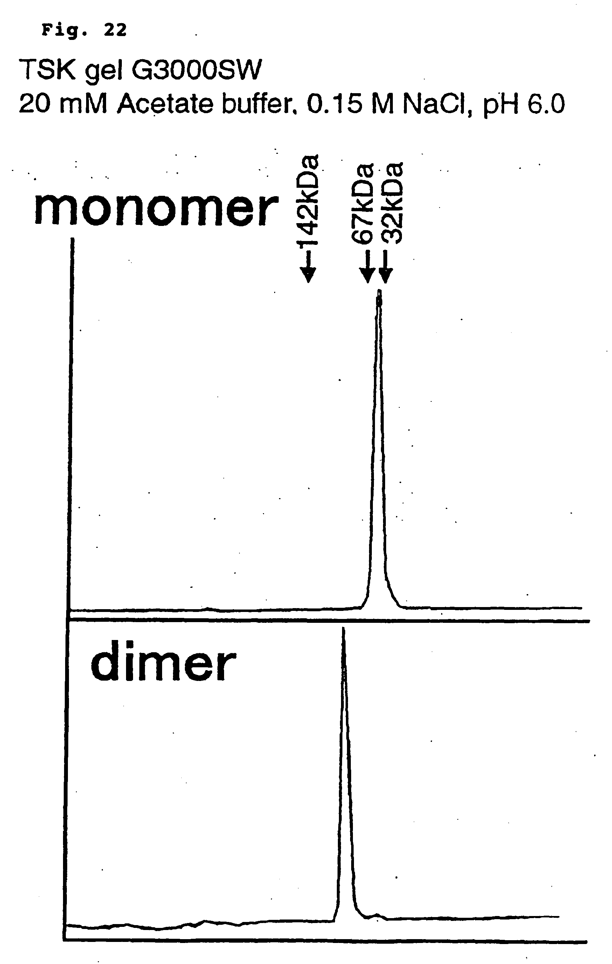

Each of fractions A and B in (2) was separately concentrated with CentriPrep-10 and applied onto TSKgel G3000SWG column (21.5 × 600 mm) equilibrated with 20 mM acetate buffer (pH 6.0) containing 0.15 M NaCl. Chromatograms are shown in Figure 20. The analysis of the fractions by SDS-PAGE confirmed that both major peaks (AI and BI) are of desired single-chain Fv. In the gel filtration analysis, the fraction A was eluted at 36 kDa of apparent molecular weight and the fraction B was eluted at 76 kDa. The purified single-chain Fvs (AI, BI) were analyzed with 15% SDS polyacrylamide gel. Samples were treated in the absence or presence of a reductant and the electrophoresis was carried out in accordance with the Laemmli's method. Then the protein was stained with Coomassie Brilliant Blue. As shown in Figure 21, both AI and BI gave a single band at 35 kDa of apparent molecular weight, regardless of the absence or presence of the reductant. From the above, it is concluded that AI is a monomer of the single-chain Fv and BI is a non-covalently bound dimer of the single-chain Fv. The gel filtration analysis of the fractions AI and BI with TSKgel G3000SW column (7.5 × 60 mm) revealed that a peak of the monomer is detected only in the fraction AI and a peak of the dimer is detected only in the fraction BI (Figure 22). The dimer fraction (fraction BI) accounted for 4percent (%) of total single-chain Fvs. More than 90% of the dimer in the dimer fraction was stably preserved for more than a month at 4°C.

### 5.10 Construction of vector expressing single-chain Fv derived from antibody MABL-2 in E. coli cell

The pscM2 vector was modified by the PCR method to prepare a vector effectively expressing the single-chain Fv from the antibody MABL-2 in E. coli cells. The resultant DNA fragment was introduced into pSCFVT7 expression vector.

As a forward primer for PCR, Nde-VHSm02 primer shown in SEQ ID No. 27 was designed to hybridize to a DNA encoding the N-terminal of the H chain V region and to contain a start codon and NdeI restriction enzyme recognition site. As a reverse primer for PCR, VLAS primer shown in SEQ ID No. 28 was designed to hybridize to a DNA encoding the C-terminal of the L chain V region and to contain two stop codons and EcoRI restriction enzyme recognition site. The forward primer, Nde-VHSm02, comprises five point mutations in the part hybridizing to the DNA encoding the N-terminal of the H chain V region for the effective expression in E. coli.

100 µl of a PCR solution comprising 10 µl of 10 x PCR Buffer #1, 1 mM MgCl₂, 0.2 mM dNTPs, 5 units of KOD DNA polymerase (all from TOYOBO), 1 µM of each primer and 100 ng of a template DNA (pscM2) was heated at 98°C for 15 seconds, at 65°C for 2 seconds and at 74°C for 30 seconds in order. This temperature cycle was repeated 25 times.

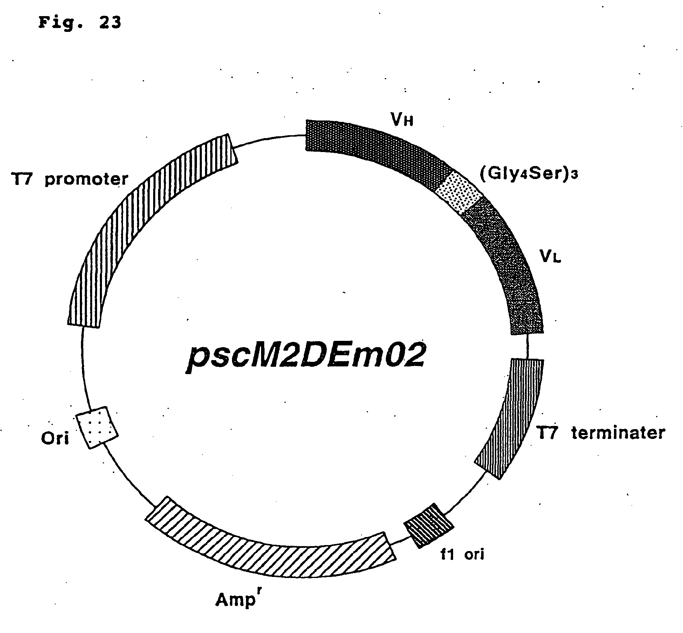

The PCR product was purified using the QIAquick PCR Purification Kit (QIAGEN) and digested by NdeI and EcoRI, and then the resulting DNA fragment was cloned into pSCFVT7 vector, from which pelB signal sequence had been eliminated by the digestion with NdeI and EcoRI. After DNA sequencing, the resulting plasmid comprising a DNA fragment with the desired DNA sequence is designated as "pscM2DEm02" (see Figure 23). The nucleotide sequence and the amino acid sequence of the single-chain Fv derived from the antibody MABL-2 contained in the plasmid pscM2DEm02 are shown in SEQ ID No. 29.

### 5.11 Expression of single-chain Fv derived from antibody MABL-2 in E. coli cells

E. coli BL21(DE3)pLysS (STRATAGENE) was transformed with pscM2DEm02 vector to obtain a strain of E. coli expressing the single-chain Fv derived from antibody MABL-2. The resulting clones were examined for the expression of the desired protein using SDS-PAGE, and a clone with a high expression level was selected as a strain producing the single-chain Fv derived from antibody MABL-2.

### 5.12 Purification of single-chain Fv derived from antibody MABL-2 produced in E.coli

A single colony of E. coli obtained by the transformation was cultured in 3 ml of LB medium at 28°C for 7 hours and then in 70 ml of LB medium at 28°C overnight. This pre-culture was transplanted to 7 L of LB medium and cultured at 28°C with stirring at 300 rpm using the Jarfermenter. When an absorbance of the medium reached O.D.=1.5, the bacteria were induced with 1 mM IPTG and then cultured for 3 hours.

The culture medium was centrifuged (10000 × g, 10 minutes) and the precipitated bacteria were recovered. To the bacteria was added 50 mM Tris-HCl buffer (pH 8.0) containing 5 mM EDTA, 0.1 M NaCl and 1% Triton X-100 and the bacteria were disrupted by ultrasonication (out put: 4, duty cycle: 70%, 1 minute × 10 times). The suspension of disrupted bacteria was centrifuged (12000 × g, 10 minutes) to precipitate inclusion body. Isolated inclusion body was mixed with 50 mM Tris-HCl buffer (pH 8.0) containing 5 mM EDTA, 0.1 M NaCl and 4% Triton X-100, treated by ultrasonication (out put: 4, duty cycle: 50%, 30 seconds × 2 times) again and centrifuged (12000 × g, 10 minutes) to isolate the desired protein as precipitate and to remove containment proteins included in the supernatant.

The inclusion body comprising the desired protein was lysed in 50 mM Tris-HCl buffer (pH 8.0) containing 6 M Urea, 5 mM EDTA and 0.1 M NaCl and applied onto Sephacryl S-300 gel filtration column (5 × 90 cm, Amersharm Pharmacia) equilibrated with 50 mM Tris-HCl buffer (pH 8.0) containing 4M Urea, 5 mM EDTA, 0.1 M NaCl and 10 mM mercaptoethanol at a flow rate of 5 ml/minutes to remove associated single chain Fvs with high-molecular weight. The obtained fractions were analyzed with SDS-PAGE and the fractions with high purity of the protein were diluted with the buffer used in the gel filtration up to O.D₂₈₀=0.25. Then, the fractions were dialyzed three times against 50 mM Tris-HCl buffer (pH 8.0) containing 5 mM EDTA, 0.1 M NaCl, 0.5 M Arg, 2 mM glutathione in the reduced form and 0.2 mM glutathione in the oxidized form in order for the protein to be refolded. Further, the fraction was dialyzed three times against 20 mM acetate buffer (pH 6.0) containing 0.15 M NaCl to exchange the buffer.

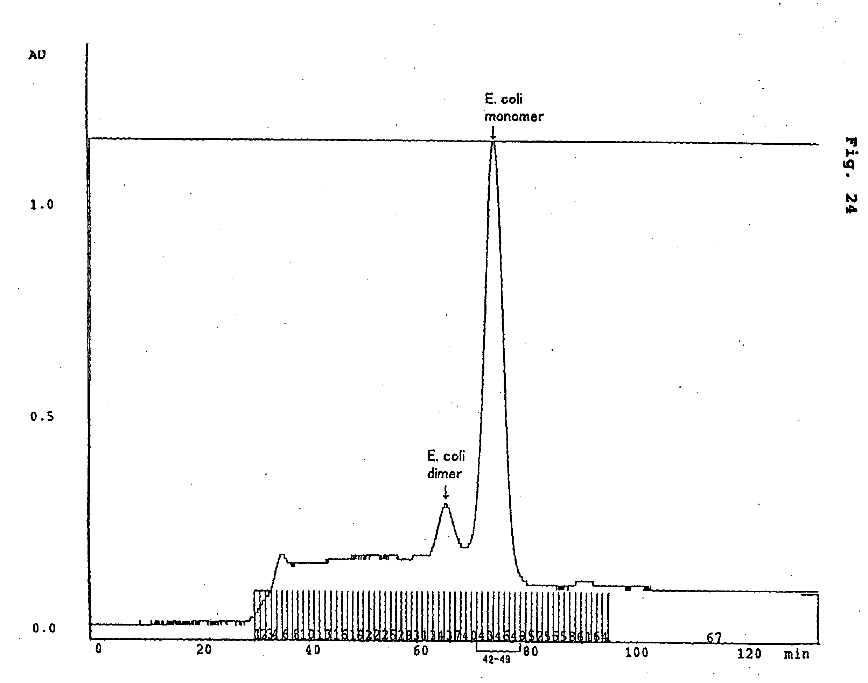

The dialysate product was applied onto Superdex 200 pg gel filtration column (2.6 × 60 cm, Amersharm Pharmacia) equilibrated with 20 mM acetate buffer (pH 6.0) containing 0.15 M NaCl to remove a small amount of high molecular weight protein which was intermolecularly crosslinked by S-S bonds. As shown in Figure 24, two peaks, major and sub peaks, were eluted after broad peaks which are expectedly attributed to an aggregate with a high molecular weight. The analysis by SDS-PAGE (see Figure 21) and the elution positions of the two peaks in the gel filtration analysis suggest that the major peak is of the monomer of the single-chain Fv and the sub peak is of the non-covalently bound dimer of the single-chain Fv. The non-covalently bound dimer accounted for 4 percent of total single-chain Fvs.

### 5.13 Apoptosis-inducing activity in vitro of single-chain Fv derived from antibody MABL-2

An apoptosis-inducing action of the single-chain Fv from antibody MABL-2 (MABL2-scFv) produced by the CHO cells and E. coli was examined according to two protocols by Annexin-V staining (Boehringer Mannheim) using the L1210 cells (hIAP/L1210) into which human IAP gene had been introduced.

In the first protocol sample antibodies at the final concentration of 3 µg/ml were added to 5 × 10⁴ cells of hIAP/L1210 cell line and cultured for 24 hours. Sample antibodies, i.e., the monomer and the dimer of the single-chain Fv of MABL-2 from the CHO cells obtained in Example 5.9, the monomer and the dimer of the single-chain Fv of MABL-2 from E. coli obtained in Example 5.12, and the mouse IgG antibody as a control were analyzed. After culturing, the Annexin-V staining was carried out and the fluorescence intensity thereof was measured using the FACScan apparatus (BECTON DICKINSON).

In the second protocol sample antibodies at the final concentration of 3 µg/ml were added to 5 × 10⁴ cells of hIAP/L1210 cell line, cultured for 2 hours and mixed with anti-FLAG antibody (SIGMA) at the final concentration of 15 µg/ml and further cultured for 22 hours. Sample antibodies of the monomer of the single-chain Fv of MABL-2 from the CHO cells obtained in Example 5.9 and the mouse IgG antibody as a control were analyzed. After culturing, the Annexin-V staining was carried out and the fluorescence intensity thereof was measured using the FACScan apparatus.

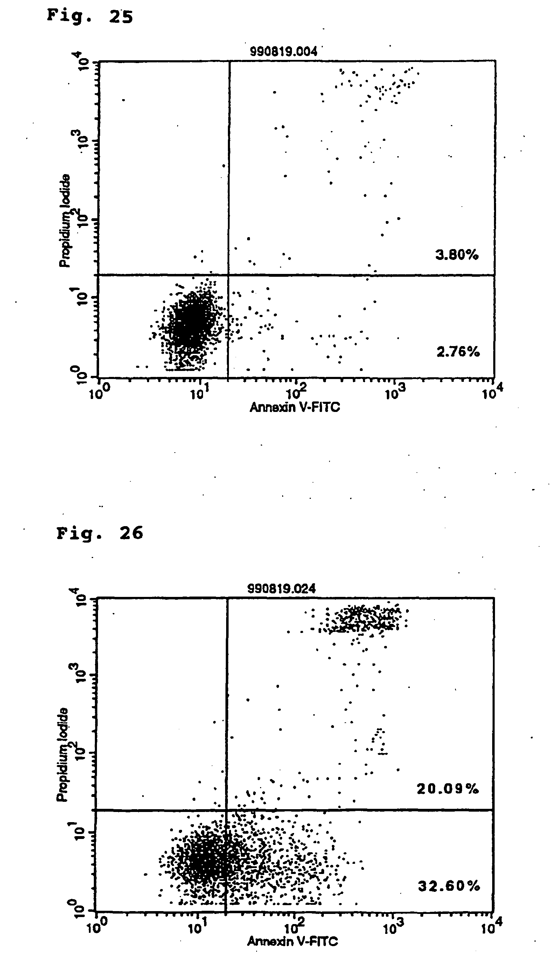

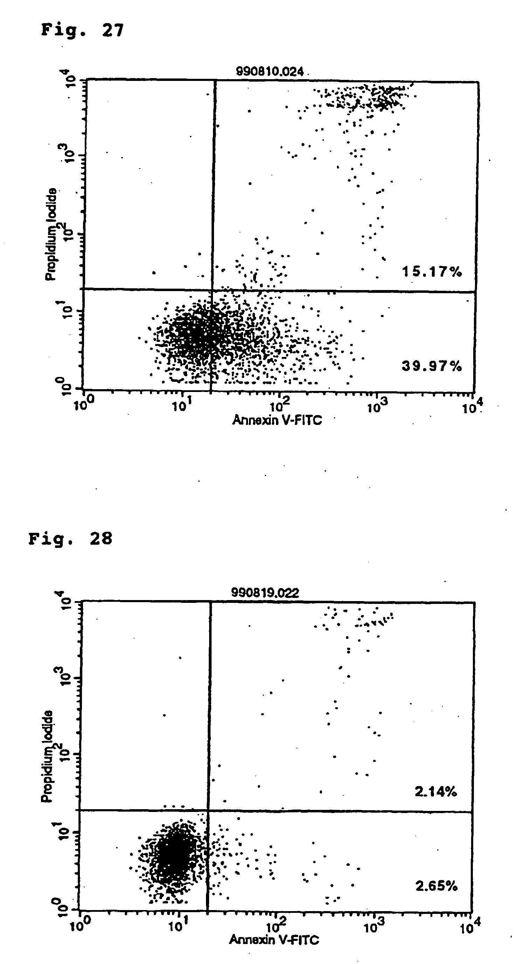

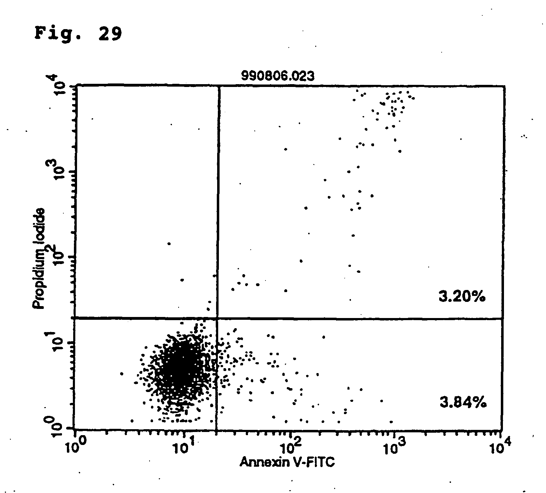

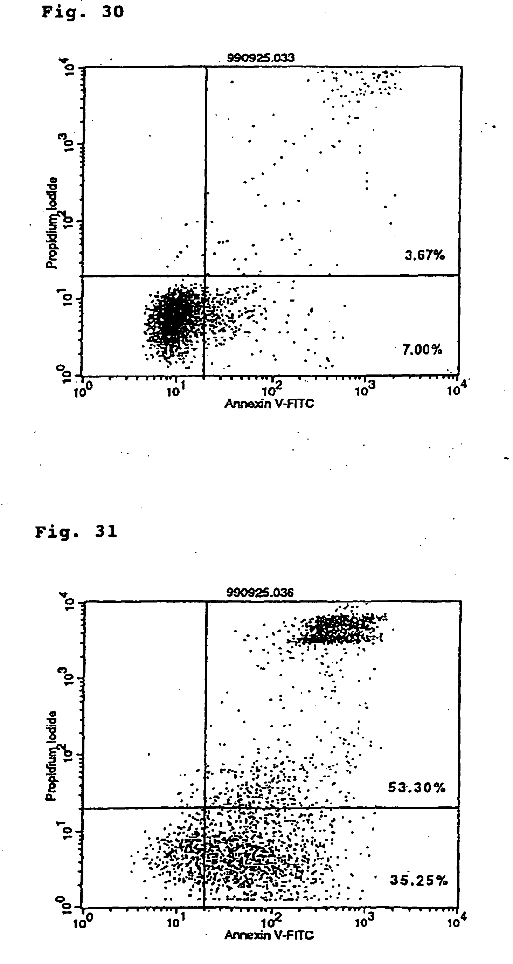

Results of the analysis by the Annexin-V staining are shown in Figures 25-31. The results show that the dimers of the single-chain Fv polypeptide of MABL-2 produced in the CHO cells and E. coli remarkably induced cell death (Figures 26, 27) in comparison with the control (Figure 25), while no apoptosis-inducing action was observed in the monomers of the single-chain Fv polypeptide of MABL-2 produced in the CHO cells and E. coli (Figures 28, 29). When anti-FLAG antibody was used together, the monomer of the single-chain Fv polypeptide derived from antibody MABL-2 produced in the CHO cells induced remarkably cell death (Figure 31) in comparison with the control (Figure 30).

### 5.14 Antitumor effect of the monomer and the dimer of scFv/CHO polypeptide with a model mouse of human myeloma

### (1) Quantitative measurement of human IgG in mouse serum

Measurement of human IgG (M protein) produced by human myeloma cell and contained in mouse serum was carried out by the following ELISA. 100 µL of goat anti-human IgG antibody (BIOSOURCE, Lot#7902) diluted to 1 µg/mL with 0.1% bicarbonate buffer (pH 9.6) was added to each well on 96 wells plate (Nunc) and incubated at 4°C overnight so that the antibody was immobilized. After blocking, 100 µL of the stepwisely diluted mouse serum or human IgG (CAPPEL, Lot#00915) as a standard was added to each well and incubated for 2 hours at a room temperature. After washing, 100 µL of alkaline phosphatase-labeled anti-human IgG antibody (BIOSOURCE, Lot#6202) which had been diluted to 5000 times was added, and incubation was carried out for 1 hour at a room temperature. After washing, a substrate solution was added. After incubation, absorbance at 405 nm was measured using the MICROPLATE READER Model 3550 (BioRad). The concentration of human IgG in the mouse serum was calculated based on the calibration curve obtained from the absorbance values of human IgG as the standard.

### (2) Preparation of antibodies for administration

The monomer and the dimer of the scFv/CHO polypeptide were respectively diluted to 0.4 mg/mL or 0.25 mg/mL with sterile filtered PBS(-) on the day of administration to prepare samples for the administration.

### (3) Preparation of a mouse model of human myeloma

A mouse model of human myeloma was prepared as follows. KPMM2 cells passaged in vivo (JP-Appl. 7-236475) by SCID mouse (Japan Clare) were suspended in RPMI1640 medium (GIBCO-BRL) containing 10% fetal bovine serum (GIBCO-BRL) and adjusted to 3 × 10⁷ cells/mL. 200 µL of the KPMM2 cell suspension (6 × 10⁶ cells/mouse) was transplanted to the SCID mouse (male, 6 week-old) via caudal vein thereof, which had been subcutaneously injected with the asialo GM1 antibody (WAKO JUNYAKU, 1 vial dissolved in 5 mL) a day before the transplantation.

### (4) Administration of antibodies

The samples of the antibodies prepared in (2), the monomer (250 µL) and the dimer (400 µL), were administered to the model mice of human myeloma prepared in (3) via caudal vein thereof. The administration was started from three days after the transplantation of KPMM2 cells and was carried out twice a day for three days. As a control, 200 µL of sterile filtered PBS(-) was likewise administered twice a day for three days via caudal vein. Each group consisted of seven mice.

### (5) Evaluation of antitumor effect of the monomer and the dimer of scFv/CHO polypeptide with the model mouse of human myeloma

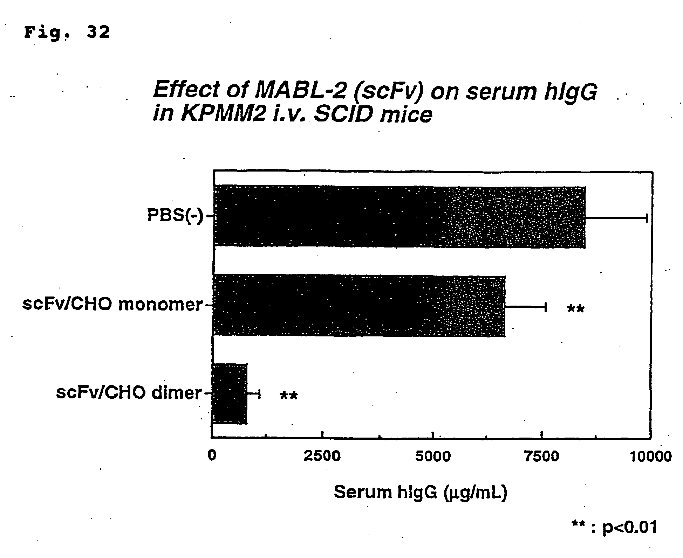

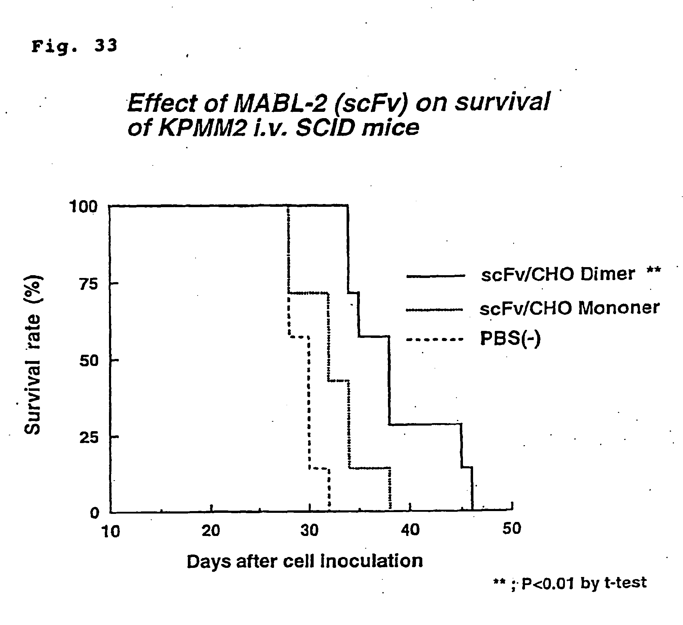

The antitumor effect of the monomer and the dimer of scFv/CHO polypeptide with the model mice of human myeloma was evaluated in terms of the change of human IgG (M protein) concentration in the mouse serum and survival time of the mice. The change of human IgG concentration was determined by measuring it in the mouse serum collected at 24 days after the transplantation of KPMM2 cells by ELISA described in the above (1). The amount of serum human IgG (M protein) in the serum of the PBS(-)-administered group (control) increased to about 8500 µg/mL, whereas the amount of human IgG of the scFv/CHO dimer-administered group was remarkably low, that is, as low as one-tenth or less than that of the control group. Thus, the results show that the dimer of scFv/CHO strongly inhibits the growth of the KPMM2 cells (Figure 32). As shown in Figure 33, a remarkable elongation of the survival time was observed in the scFv/CHO dimer-administered group in comparison with the PBS(-)-administered group.

From the above, it is confirmed that the dimer of scFv/CHO has an antitumor effect for the human myeloma model mice. It is considered that the antitumor effect of the dimer of scFv/CHO, the reconstructed polypeptide of the invention, results from the apoptosis-inducing action of the reconstructed polypeptide.

### 5.15 Hemagglutination Test

Hemagglutination test and determination of hemagglutination were carried out in accordance with "Immuno-Biochemical Investigation", Zoku-Seikagaku Jikken Koza, edited by the Biochemical Society of Japan, published by Tokyo Kagaku Dojin.

Blood was taken from a healthy donor using heparin-treated syringes and washed with PBS(-) three times, and then erythrocyte suspension with a final concentration of 2% in PBS(-) was prepared. Test samples were the antibody MABL-2, the monomer and the dimer of the single-chain Fv polypeptide produced by the CHO cells, and the monomer and the dimer of the single-chain Fv polypeptide produced by E. coli, and the control was mouse IgG (ZYMED). For the investigation of the hemagglutination effect, round bottom 96-well plates available from Falcon were used. 50 µL per well of the aforementioned antibody samples and 50 µL of the 2% erythrocyte suspension were added and mixed in the well. After incubation for 2 hours at 37°C, the reaction mixtures were stored at 4°C overnight and the hemagglutination thereof was determined. As a control, 50 µL per well of PBS(-) was used and the hemagglutination test was carried out in the same manner. The mouse IgG and antibody MABL-2 were employed at 0.01, 0.1, 1.0, 10.0 or 100.0 µg/mL of the final concentration of the antibodies. The single-chain Fvs were employed at 0.004, 0.04, 0.4, 4.0, 40.0 or 80.0 µg/mL of the final concentration and further at 160.0 µg/mL only in the case of the dimer of the polypeptide produced by E. coli. Results are shown in the Table 2. In the case of antibody MABL-2, the hemagglutination was observed at a concentration of more than 0.1 µg/mL, whereas no hemagglutination was observed for both the monomer and the dimer of the single-chain Fv.

**Table 2 Hemagglutination Test**

| | Control | 0.01 | 0.1 | 1 | 10 | 100 | µg/mL | | |
|---|---|---|---|---|---|---|---|---|---|
| mIgG | - | - | - | - | - | - | | | |
| MABL-2 (intact) | - | - | + | +++ | +++ | ++ | | | |

| | Control | 0.004 | 0.04 | 0.4 | 4 | 40 | 80 | µg/mL | |
|---|---|---|---|---|---|---|---|---|---|
| scFv/CHO monomer | - | - | - | - | - | - | - | | |
| scFv/CHO dimer | - | - | - | - | - | - | - | | |

| | Control | 0.004 | 0.04 | 0.4 | 4 | 40 | 80 | 160 | µg/mL |
|---|---|---|---|---|---|---|---|---|---|
| scFv/E.coli monomer | - | - | - | - | - | - | - | | |
| scFv/E.coli dimer | - | - | - | - | - | - | - | - | |

Example 6 Reconstructed polypeptide sc(Fv)₂ comprising two H chain V regions and two L chain V regions and antibody MABL-2 scFvs having linkers with different length

### 6.1 Construction of Plasmid expressing antibody MABL-2 sc(Fv)₂

For the preparation of a plasmid expressing the reconstructed polypeptide [sc(Fv)₂] which comprises two H chain V regions and two L chain V regions derived from the antibody MABL-2, the aforementioned pCHOM2, which comprises the DNA encoding scFv derived from the MABL-2 described above, was modified by the PCR method as mentioned below and the resulting DNA fragment was introduced into pCHOM2.

Primers employed for the PCR are EF1 primer (SEQ ID NO: 30) as a sense primer, which is designed to hybridize to a DNA encoding EF1α, and an antisense primer (SEQ ID NO: 19), which is designed to hybridize to the DNA encoding C-terminal of the L chain V region and to contain a DNA sequence coding for a linker region, and VLLAS primer containing SalI restriction enzyme recognition site (SEQ ID NO 31).

100 µl of the PCR solution comprises 10 µl of 10 × PCR Buffer #1, 1 mM MgCl₂, 0.2 mM dNTPs (dATP, dGTP, dCTP and dTTP), 5 units of KOD DNA polymerase (Toyobo, Inc.), 1 µM of each primer and 100 ng of the template DNA (pCHOM2). The PCR solution was heated at 94°C for 30 seconds, at 50°C for 30 seconds and at 74°C for 1 minute in order. This temperature cycle was repeated 30 times.

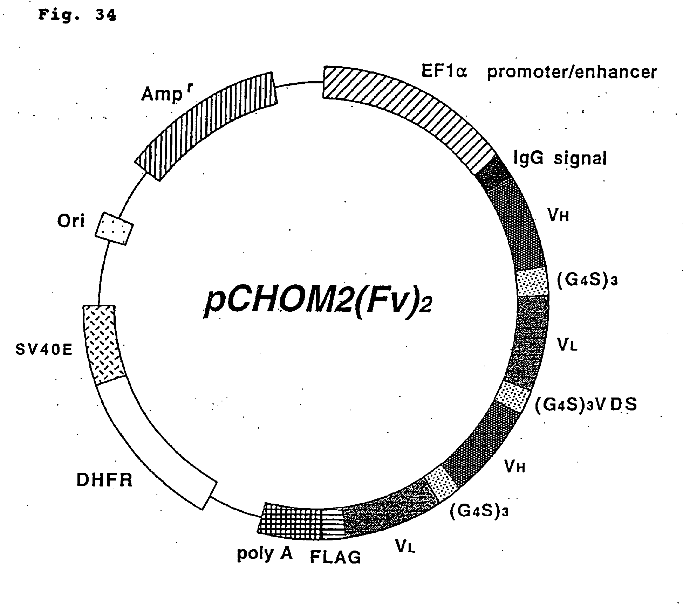

The PCR product was purified using the QIAquick PCR Purification Kit (QIAGEN) and digested by SalI. The resultant DNA fragment was cloned into pBluescript KS⁺ vector (Toyobo, Inc.). After DNA sequencing, a plasmid comprising the desired DNA sequence was digested by SalI and the obtained DNA fragment was connected using Rapid DNA Ligation Kit(BOEHRINGER MANNHEIM) to pCHOM2 digested by SalI. After DNA sequencing, a plasmid comprising the desired DNA sequence is designated as "pCHOM2(Fv)₂" (see Figure 34). The nucleotide sequence and the amino acid sequence of the antibody MABL-2 sc(Fv)₂ region contained in the plasmid pCHOM2(Fv)₂ are shown in SEQ ID No. 32.

### 6.2 Preparation of Plasmid expressing antibody MABL-2 scFvs having linkers with various length

The scFvs containing linkers with different length and the V regions which are designed in the order of [H chain]-[L chain] (hereinafter "HL") or [L chain]-[H chain] (hereinafter "LH") were prepared using, as a template, cDNAs encoding the H chain and the L chain derived from the MABL-2 as mentioned below.

To construct HL type scFv the PCR procedure was carried out using pCHOM2(Fv)₂ as a template. In the PCR step, a pair of CFHL-F1 primer (SEW ID NO: 33) and CFHL-R2 primer (SEQ ID NO: 34) or a pair of CFHL-F2 primer (SEQ ID NO: 35) and CFHL-R1 primer (SEQ ID NO: 36) and KOD polymerase were employed. The PCR procedure was carried out by repeating 30 times the temperature cycle consisting of 94°C for 30 seconds, 60°C for 30 seconds and 72°C for 1 minute in order to produce a cDNA for the H chain containing a leader sequence at 5'-end or a cDNA for the L chain containing FLAG sequence at 3'-end thereof. The resultant cDNAs for the H chain and the L chain were mixed and PCR was carried out by repeating 5 times the temperature cycle consisting of 94°C for 30 seconds, 60°C for 30 seconds and 72°C for 1 minute in order using the mixture as templates and the KOD polymerase. To the reaction mixture were added CFHL-F1 and CFHL-R1 primers and then the PCR reaction was performed by repeating 30 times of the aforementioned temperature cycle to produce a cDNA for HL-0 type without a linker.

To construct LH type scFv, the PCR reaction was carried out using, as a template, pGEM-M2L and pGEM-M2H which contain cDNAs encoding the L chain V region and the H chain V region from the antibody MABL-2, respectively (see JP- Appl. 11-63557). A pair of T7 primer (SEQ ID NO: 37) and CFLH-R2 primer(SEQ ID NO: 38) or a pair of CFLH-F2 primer (SEQ ID NO: 39) and CFLH-R1 (SEQ ID NO: 40) and the KOD polymerase (Toyobo Inc.) were employed. The PCR reaction was performed by repeating 30 times the temperature cycle consisting of 94°C for 30 seconds, 60°C for 30 seconds and 72°C for 1 minute in sequential order to produce a cDNA of an L chain containing a leader sequence at 5'-end or a cDNA of an H chain containing FLAG sequence at 3'-end thereof. The resultant cDNAs of the L chain and the H chain were mixed and PCR was carried out using this mixture as templates and the KOD polymerase by repeating 5 times the temperature cycle consisting of 94°C for 30 seconds, 60°C for 30 seconds and 72°C for 1 minute in order. To the reaction mixture were added T7 and CFLH-R1 primers and the reaction was performed by repeating 30 times of the aforementioned temperature cycle. The reaction product was used as a template and PCR was carried out using a pair of CFLH-F4 primer (SEQ ID NO: 41) and CFLH-R1 primer by repeating 30 times the temperature cycle consisting of 94°C for 30 seconds, 60°C for 30 seconds and 72°C for 1 minute in order to produce a cDNA of LH-0 type without a linker.

The resultant cDNAs of LH-0 and HL-0 types were digested by EcoRI and BamHI restriction enzymes (Takara Shuzo) and the digested cDNAs were introduced into an expression plasmid INPEP4 for mammalian cells using Ligation High (Toyobo Inc.), respectively. Competent E. coli JM109 (Nippon Gene) was transformed with each plasmid and the desired plasmids were isolated from the transformed E. coli using QIAGEN Plasmid Maxi Kit (QUIAGEN). Thus plasmids pCF2LH-0 and pCF2HL-0 were prepared.

To construct the expression plasmids of HL type containing linkers with different size, pCF2HL-0, as a template, and CFHL-X3 (SEQ ID NO: 42), CFHL-X4 (SEQ ID NO: 43), CFHL-X5 (SEQ ID NO: 44), CFHL-X6 (SEQ ID NO: 45) or CFHL-X7 (SEQ ID NO: 46), as a sense primer, and BGH-1 (SEQ ID NO: 47) primer, as an antisense primer, which is complementary with the vector sequence were employed. PCR reaction was carried out using the KOD polymerase by repeating 30 times the temperature cycle consisting of 94°C for 30 seconds, 60°C for 30 seconds and 72°C for 1 minute in order and the reaction products were digested by restriction enzymes XhoI and BamHI (Takara Shuzo). The digested fragments were introduced between XhoI and BamHI sites in the pCF2HL-0 using Ligation High (Toyobo Inc.), respectively. Competent E. coli JM109 was transformed with each plasmid and the desired plasmids were isolated from the transformed E. coli by using Qiagen Plasmid Maxi kit. Thus expression plasmids pCF2HL-3, pCF2HL-4, pCF2HL-5, pCF2HL-6 and pCF2HL-7 were prepared.

To construct expression plasmid for the transient expression in COS7 cells the plasmids pCF2HL-0, pCF2HL-3, pCF2HL-4, pCF2HL-5, pCF2HL-6 and pCF2HL-7 were digested by restriction enzymes EcoRI and BamHI (Takara Shuzo) and the resultant fragments of approximately 800 bp were purified with agarose gel electrophoresis. The obtained fragments were introduced between EcoRI and BamHI sites in an expression plasmid pCOS1 for the expression in mammalian cells by using Ligation High (Toyobo Inc.), respectively. Competent E. coli DH5a (Toyobo Inc.) was transformed with each plasmid and the desired plasmids were isolated from the transformed E. coli using Qiagen Plasmid Maxi kit. Thus the expression plasmids CF2HL-0/pCOS1, CF2HL-3/pCOS1, CF2HL-4/pCOS1, CF2HL-5/pCOS1, CF2HL-6/pCOS1 and CF2HL-7/pCOS1 were prepared.

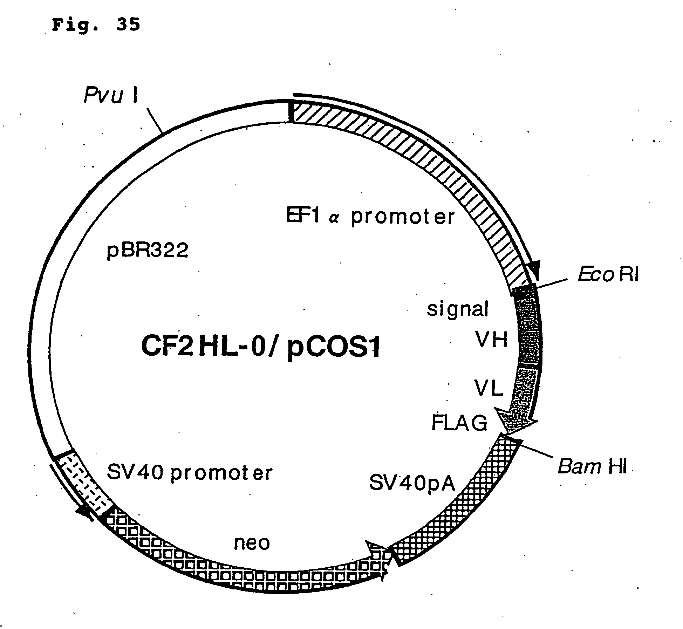

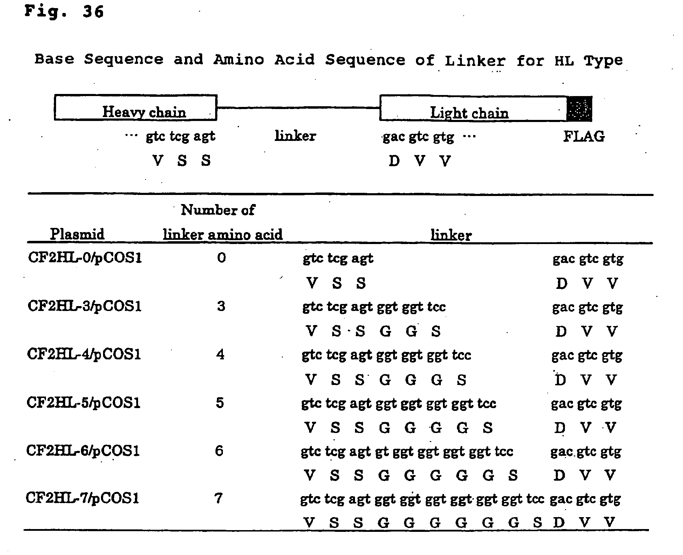

As a typical example of these plasmids, the construction of the plasmid CF2HL-0/pCOS1 is illustrated in Figure 35 and the nucleotide sequence and the amino acid sequence of MABL2-scFv <HL-0> contained in the plasmid are shown in SEQ ID No. 48. Nucleotide sequences and amino acid sequences of the linker regions in these plasmids are also shown in Figure 36.

To construct the expression plasmids of LH type containing linkers with different size, pCF2LH-0, as a template, and CFLH-X3 (SEQ ID NO: 49), CFLH-X4 (SEQ ID NO: 50), CFLH-X5 (SEQ ID NO: 51), CFLH-X6 (SEQ ID NO: 52) or CFLH-X7 (SEQ ID NO: 53), as a sense primer, and BGH-1 primer, as an antisense primer, which is complementary with the vector sequence were employed. PCR reaction was carried out using the KOD polymerase by repeating 30 times the temperature cycle consisting of 94°C for 30 seconds, 60°C for 30 seconds and 72°C for 1 minute in order and the reaction products were digested by restriction enzymes XhoI and BamHI. The digested fragments were introduced into the pCF2LH-0 between XhoI and BamHI sites using Ligation High, respectively. Competent E. coli DH5α (Toyobo Inc.) was transformed with each plasmid and the desired plasmids were isolated from the transformed E. coli using Qiagen Plasmid Maxi kit. Thus expression plasmids pCF2LH-3, pCF2LH-4, pCF2LH-5, pCF2LH-6 and pCF2LH-7 were prepared.

To construct expression plasmid for the transient expression in COS7 cells the plasmids pCF2LH-0, pCF2LH-3, pCF2LH-4, pCF2LH-5, pCF2LH-6 and pCF2LH-7 were digested by restriction enzymes EcoRI and BamHI (Takara Shuzo) and the resultant fragments of approximately 800 bp were purified with agarose gel electrophoresis. The obtained fragments were introduced between XhoI and BamHI sites in an expression plasmid pCOS1 for the expression in mammalian cells by using the Ligation High, respectively. Competent E. coli DH5α (Toyobo Inc.) was transformed with each plasmid and the desired plasmids were isolated from the transformed E. coli using the Qiagen Plasmid Maxi kit. Consequently, the expression plasmids CF2LH-0/pCOS1, CF2LH-3/pCOSl, CF2LH-4/pCOS1, CF2LH-5/pCOS1, CF2LH-6/pCOS1 and CF2LH-7/pCOS1 were prepared.

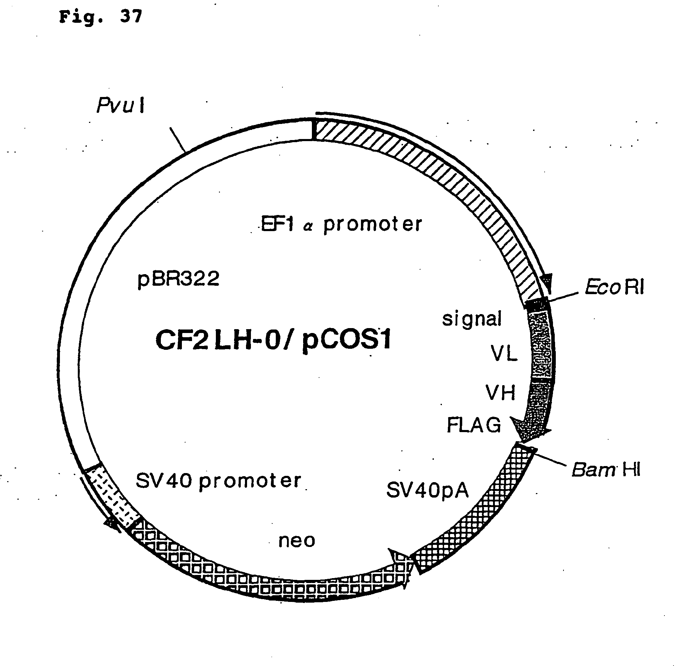

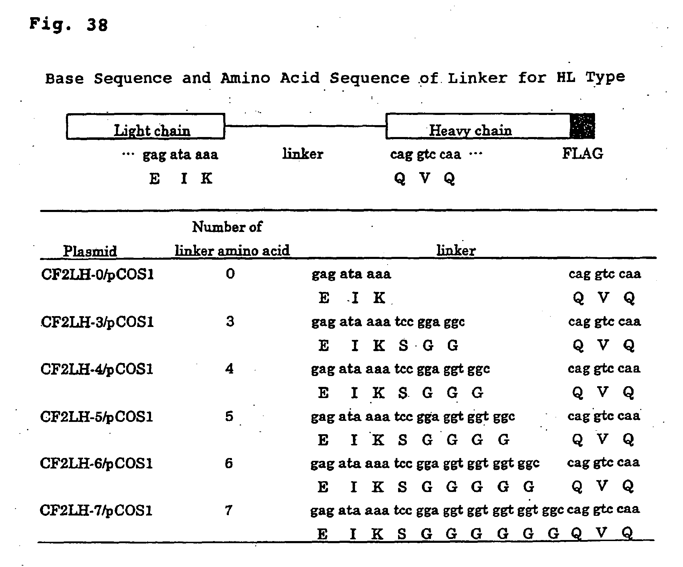

As a typical example of these plasmids, the construction of the plasmid CF2LH-0/pCOS1 is illustrated in Figure 37 and the nucleotide sequence and the amino acid sequence of MABL2-scFv <LH-0> contained in the plasmid are shown in SEQ ID No. 54. Nucleotide sequences and amino acid sequences of the linker regions in these plasmids are also shown in Figure 38.

### 6.3 Expression of scFvs and sc(Fv)₂ in COS7 cells

### (1) Preparation of culture supernatant using serum-containing culture medium

The HL type and LH type of scFvs and sc(Fv)₂ were transiently expressed in COS7 cells (JCRB9127, Japan Health Sciences Foundation). COS7 cells were subcultured in DMEM media (GIBCO BRL) containing 10% fetal bovine serum (HyClone) at 37°C in carbon dioxide atmosphere incubator. The COS7 cells were transfected with CF2HL-0, 3 - 7/pCOS1, or CF2LH-0, 3 - 7/pCOS1 prepared in Example 6.2 or pCHOM2(Fv)₂ vectors by electroporation using the Gene Pulser apparatus (BioRad). The DNA (10 µg) and 0.25 ml of 2 × 10⁷ cells/ml in DMEM culture medium containing 10% FBS and 5 mM BES (SIGMA) were added to a cuvette. After standing for 10 minutes the mixtures were treated with pulse at 0.17kV, 950µF of electric capacity. After the restoration for 10 minutes at room temperature, the electroporated cells were transferred into the DMEM culture medium (10%FBS) in 75 cm³ flask. After culturing for 72 hours, the culture supernatant was collected and centrifuged to remove cell fragments. The culture supernatant was subjected to the filtration using 0.22 µm bottle top filter (FALCON) to obtain the culture supernatant (hereinafter "CM").

### (2) Preparation of culture supernatant using serum-free culture medium

Cells transfected in the same manner as (1) were transferred to the DMEM medium (10% FBS) in 75 cm³ flask and cultured overnight. After the culture, the supernatant was discarded and the cells were washed with PBS and then added to CHO-S-SFM II medium (GIBCO BRL). After culturing for 72 hours, the culture supernatant was collected, centrifuged to remove cell fragments and filtered using 0.22 µm bottle top filter (FALCON) to obtain CM.

### 6.4 Detection of scFvs and sc(Fv)₂ in CM of COS7

The various MABL2-scFVs and sc(Fv)₂ in CM of COS7 prepared in the aforementioned Example 6.3 (2) were detected by Western Blotting method.

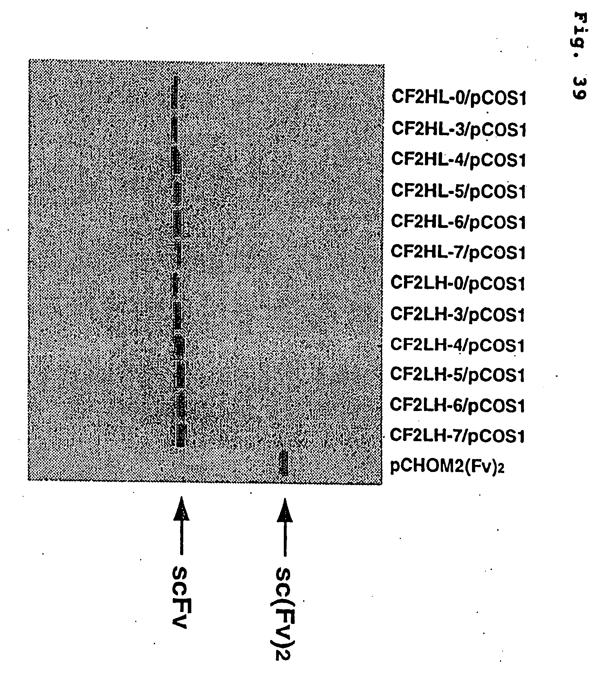

Each CM of COS7 was subjected to SDS-PAGE electrophoresis and transferred to REINFORCED NC membrane (Schleicher & Schuell). The membrane was blocked with 5% skim milk (Morinaga Nyu-gyo) and washed with TBS. Then an anti-FLAG antibody (SIGMA) was added thereto. The membrane was incubated at room temperature and washed. A peroxidase labeled mouse IgG antibody (Jackson Immuno Research) was added. After incubating and washing at room temperature, the substrate solution (Kirkegaard Perry Laboratories) was added to develop color (Figure 39).

### 6.5 Flow cytometry

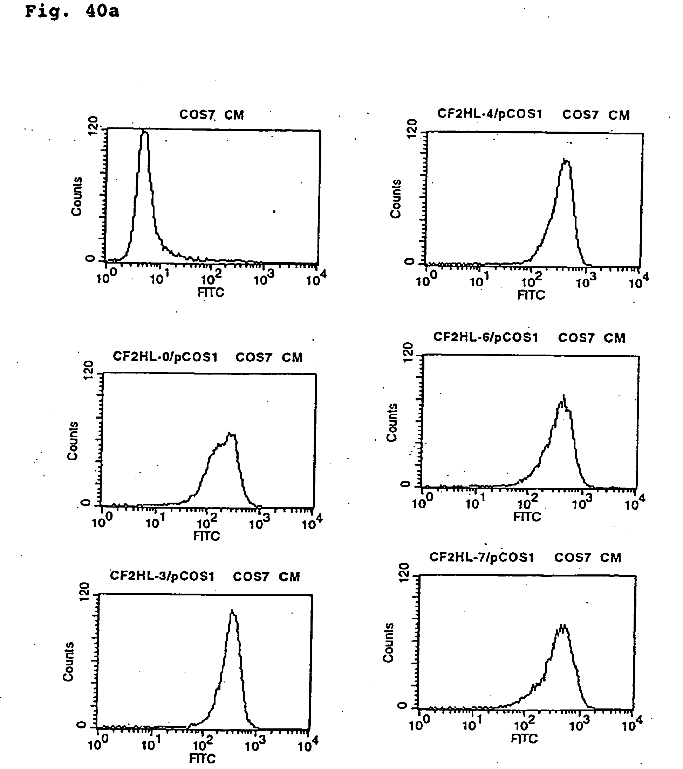

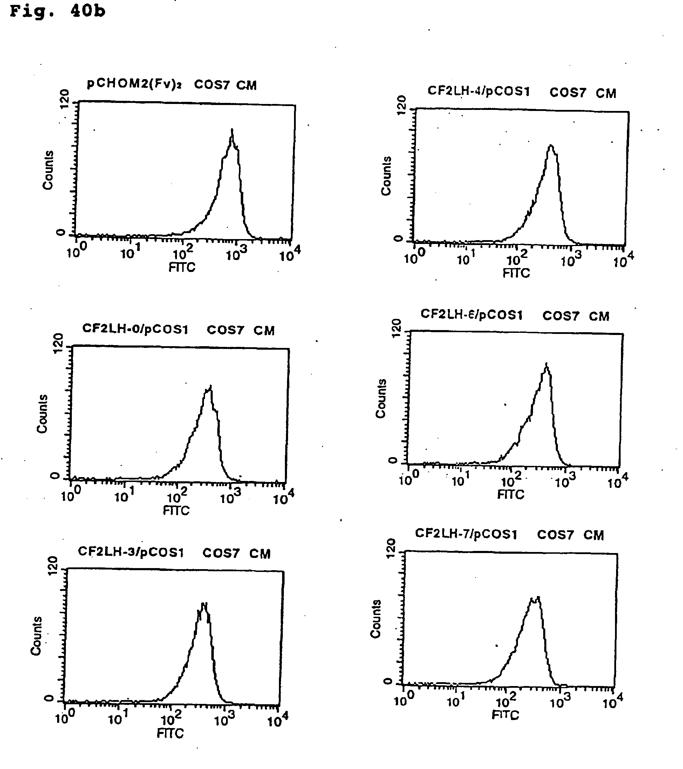

Flow cytometry was performed using the culture supernatants of COS7 cells prepared in Example 6.3 (1) to measure the binding of the MABL2-scFVs and sc(Fv)₂ to human Integrin Associated Protein (IAP) antigen. The culture supernatants to be tested or a culture supernatant of COS7 cells as a control was added to 2 × 10⁵ cells of the mouse leukemia cell line L1210 expressing human IAP. After incubating on ice and washing, 10 µg/mL of the mouse anti-FLAG antibody (SIGMA) was added and then the cells were incubated and washed. Then, the FITC labeled anti-mouse IgG antibody (BECTON DICKINSON) was added thereto and the cells were incubated and washed again. The fluorescence intensity was measured using the FACScan apparatus (BECTON DICKINSON). The results of the flow cytometry show that the MABL2-scFvs having linkers with different length and the sc(Fv)₂ in the culture supernatants of COS7 have high affinity to human IAP (see Figures 40a and 40b).

### 6.6 Apoptosis-inducing Effect in vitro

An apoptosis-inducing action of the culture supernatants of COS7 prepared in Example 6.3 (1) was examined by Annexin-V staining (Boehringer Mannheim) using the L1210 cells transfected with human IAP gene (hIAP/L1210).

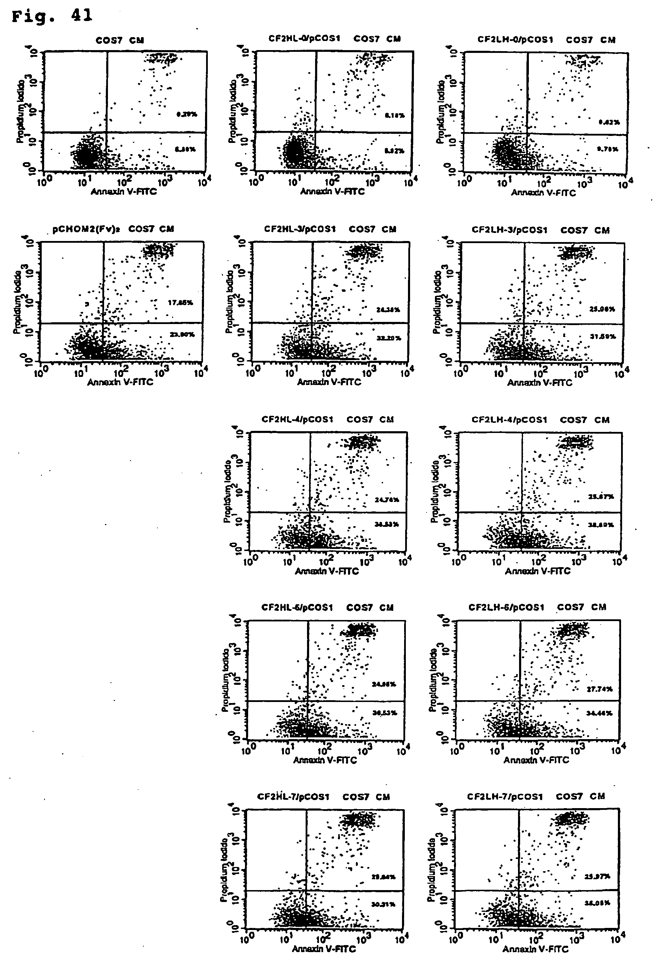

To 5 × 10⁴ cells of the hIAP/L1210 cells were added the culture supernatants of COS7 cells transfected with each vectors or a culture supernatant of COS7 cells as a control at 10% of the final concentration and the mixtures were cultured for 24 hours. Then, the Annexin-V/PI staining was performed and the fluorescence intensity was measured using the FACScan apparatus (BECTON DICKINSON). The results revealed that scFvs <HL3, 4, 6, 7, LH3, 4, 6, 7> and sc(Fv)₂ in CM of COS7 induced remarkable cell death of hIAP/L1210 cells. These results are shown in Figure 41.

### 6.7 Construction of vectors for the expression of scFvs and sc(Fv)₂ in CHO cells

To isolate and purify MABL2-scFvs and sc(Fv)₂ from culture supernatant, the expression vectors for expressing in CHO cells were constructed as below.

The EcoRI-BamHI fragments of pCF2HL-0, 3 - 7, and pCF2LH-0, 3 ∼ 7 prepared in Example 6.2 were introduced between EcoRI and BamHI sites in an expression vector pCHO1 for CHO cells using the Ligation High. Competent E. coli DH5α was transformed with them. The plasmids were isolated from the transformed E. coli using QIAGEN Plasmid Midi kit (QIAGEN) to prepare expression plasmids pCHOM2HL-0, 3 - 7, and pCHOM2LH-0, 3 - 7.

### 6.8 Production of CHO cells expressing HABL2-scFvs <HL-0, 3 ∼ 7>, MABL2-scFvs <LH-0, 3 ∼ 7> and sc(Fv)₂ and preparation of the culture supernatants thereof

CHO cells were transformed with each of the expression plasmids pCHOM2HL-0, 3 - 7, and pCHOM2LH-0, 3 ∼ 7, constructed in Example 6.7 and pCHOM2(Fv)₂ vector to prepare the CHO cells constantly expressing each reconstructed polypeptide. As a typical example thereof, the production of the CHO cells constantly expressing MABL2-scFv <HL-5> or sc(Fv)₂ is illustrated as follows.

The expression plasmids pCHOM2HL-5 and pCHOM2(Fv)₂ were linearized by digesting with a restriction enzyme PvuI and subjected to transfection to CHO cells by electroporation using Gene Pulser apparatus (BioRad). The DNA (10 µg) and 0.75 ml of PBS with 1 × 10⁷ cells/ml were added to a cuvette and treated with pulse at 1.5 kV, 25 µF of electric capacity. After the restoration for 10 minutes at room temperature, the electroporated cells were transferred into nucleic acid-containing α-MEM culture medium (GIBCO BRL) containing 10% fetal bovine serum and cultured. After culturing overnight, the supernatant was discarded. The cells were washed with PBS and added to nucleic acid-free α-MEM culture medium (GIBCO BRL) containing 10% fetal bovine serum. After culturing for two weeks, the cells were cultured in a medium containing 10 nM (final concentration) methotrexate (SIGMA), then 50 nM and 100 nM methotrexate. The resultant cells were cultured in serum-free CHO-S-SFM II medium (GIBCO BRL) in a roller bottle. The culture supernatant was collected, centrifuged to remove cell fragments and filtered using a filter with 0.22 µm of pore size to obtain CM, respectively.

According to the above, CHO cells which constantly express MABL2-scFvs <HL-0, -3, -4, -6, -7> and <LH-0, -3, - 4, -5, -6, -7> and CMs thereof were obtained.

### 6.9 Purification of dimer of MABL2-scFv <HL-5> and sc(Fv)₂

The MABL2-scFv <HL-5> and the sc(Fv)₂ were purified from CMs prepared in Example 6.8 by two types of purification method as below.

### <Purification Method 1>

HL-5 and sc(Fv)₂ were purified by the anti-FLAG antibody affinity column chromatography utilizing the FLAG sequence located at C-terminal of the polypeptides and by gel filtration. One liter of CM as obtained in 6.8 was applied onto a column (7.9ml) prepared with anti-FLAG M2 Affinity gel (SIGMA) equilibrated with 50 mM Tris-HCl buffer (TBS, pH 7.5) containing 150 mM NaCl. After washing the column with TBS, the scFv was eluted by 0.1 M glycine-HCl buffer, pH 3.5. The resultant fractions were analyzed by SDS-PAGE and the elution of the scFv was confirmed. The scFv fraction was mixed with Tween 20 up to 0.01% of the final concentration and concentrated using Centricon-10 (MILIPORE). The concentrate was applied onto TSKgel G3000SWG column (7.5 × 600 mm) equilibrated with 20 mM acetate buffer (pH 6.0) containing 150 mM NaCl and 0.01% Tween 20. At 0.4 mL/minute of the flow rate, the scFv was detected by the absorption at 280 nm. The HL-5 was eluted as the major fraction in the position of the dimer and the sc(Fv)₂ was eluted in the position of the monomer.

### <Purification Method 2>

HL-5 and sc(Fv)₂ were purified using three steps comprising ion exchange chromatography, hydroxyapatite and gel filtration. In the ion exchange chromatography, Q sepharose fast flow column (Pharmacia) was employed for HL-5 and SP-sepharose fast flow column was employed for sc(Fv)₂. In and after the second step, HL-5 and sc(Fv)₂ were processed by the same procedure.

### First step for HL-5

CM of HL-5 was diluted to two times with 20 mM Tris-HCl buffer (pH 9.0) containing 0.02% Tween 20 and then the pH was adjusted to 9.0 with 1 M Tris. The solution was applied onto Q Sepharose fast flow column equilibrated with 20 mM Tris-HCl buffer (pH 8.5) containing 0.02% Tween 20. A polypeptide adsorbed to the column was eluted by a linear gradient of NaCl in the same buffer, from 0.1 to 0.55 M. Monitoring the eluted fractions by SDS-PAGE, the fractions containing HL-5 were collected and subjected to hydroxyapatite of the second step.

### First step for sc(Fv)₂

CM of the sc(Fv)₂ was diluted to two times with 20 mM acetate buffer (pH 5.5) containing 0.02% Tween 20 and its pH was adjusted to 5.5 with 1 M acetic acid. The solution was applied onto a SP-Sepharose fast flow column equilibrated with 20 mM acetate buffer (pH 5.5) containing 0.02% Tween 20. A polypeptide adsorbed to the column was eluted by a linear gradient of NaCl in the buffer, from 0 to 0.5 M. Monitoring the eluted fractions by SDS-PAGE, the fractions containing the sc(Fv)₂ were collected and subjected to hydroxyapatite of the second step.

### Second step: Hydroxyapatite chromatography of HL-5 and sc(Fv)₂

The fractions of HL-5 and sc(Fv)₂ obtained in the first step were separately applied onto the hydroxyapatite column (Type I, BIORAD) equilibrated with 10 mM phosphate buffer containing 0.02% Tween 20, pH 7.0. After washing the column with the same buffer, polypeptides adsorbed to the column were eluted by a linear gradient of the phosphate buffer up to 0.5 M. Monitoring the eluted fractions by SDS-PAGE, the fractions containing the desired polypeptides were collected.

### Third step: Gel filtration of HL-5 and sc(Fv)₂

Each fraction obtained at the second step was separately concentrated with CentriPrep-10 (MILIPORE) and applied onto a Superdex 200 column (2.6 × 60 cm, Pharmacia) equilibrated with 20 mM acetate buffer (pH 6.0) containing 0.02% Tween 20 and 0.15 M NaCl. HL-5 was eluted in the position of the dimer, and sc(Fv)HL-5 and sc(Fv)₂ were eluted in the position of the monomer as a major peek respectively.

Since the monomer of HL-5 was hardly detected by both purification methods, it is proved that the dimers of single-chain Fvs are formed in high yields when the linker for the single-chain Fv contains around 5 amino acids. Furthermore, the dimer of HL-5 and the sc(Fv)₂ were stably preserved for a month at 4°C after the purification.

### 6.10 Evaluation of the binding activity of purified dimer of scFv <HL-5> and sc(Fv)₂ against antigen

Flow cytometry was performed using the purified dimer of MABL2-scFv <HL-5> and the purified sc(Fv)₂ in order to evaluate the binding to human Integrin Associated Protein (IAP) antigen. 10µg/ml of the purified dimer of MABL2-scFv <HL-5>, the purified sc(Fv)₂, the antibody MABL-2 as a positive control or a mouse IgG (Zymed) as a negative control was added to 2 × 10⁵ cells of the mouse leukemia cell line L1210 expressing human IAP (hIAP/L1210) or the cell line L1210 transformed with pCOS1 (pCOS1/L1210) as a control. After incubating on ice and washing, 10µg/mL of the mouse anti-FLAG antibody (SIGMA) was added and then the cells were incubated and washed. FITC labeled anti-mouse IgG antibody (BECTON DICKINSON) was added thereto and the cells were incubated and washed again. Then the fluorescence intensity was measured using the FACScan apparatus (BECTON DICKINSON).

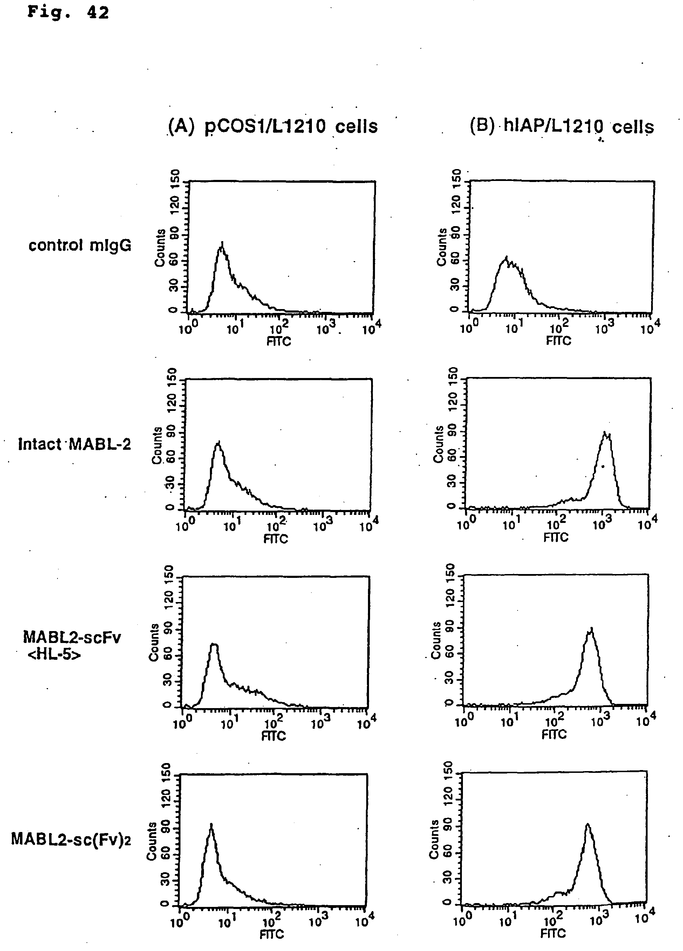

Since the purified dimer of MABL2-scFv <HL-5> and the purified sc(Fv)₂ were specifically bound to hIAP/L1210 cells, it is confirmed that the dimer of scFv <HL-5> and the sc(Fv)₂ have high affinity to human IAP (see Figure 42).

### 6.11 Apoptosis-inducing activity in vitro of purified dimer of scFv <HL-5> and sc(Fv)₂

An apoptosis-inducing action of the purified dimer of MABL2-scFv <HL-5> and the purified sc(Fv)₂ were examined by Annexin-V staining (Boehringer Mannheim) using the L1210 cells (hIAP/L1210) in which human IAP gene had been introduced and cells of human leukemic cell line CCRF-CEM.

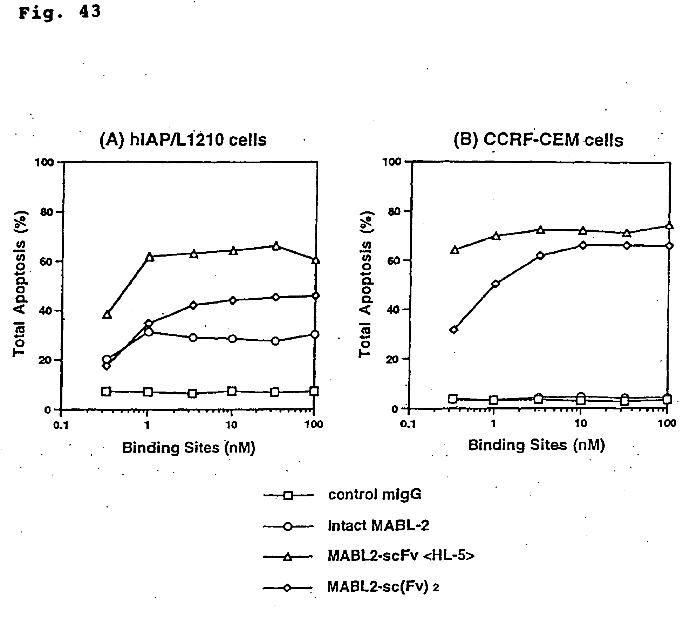

Different concentrations of the purified dimer of MABL2-scFv <HL-5>, the purified MABL2-sc(Fv)₂, the antibody MABL-2 as a positive control or a mouse IgG as a negative control were added to 5 × 10⁴ cells of hIAP/L1210 cell line or 1 × 10⁵ cells of CCRF-CEM cell line. After culturing for 24 hours, the Annexin-V staining was carried out and the fluorescence intensity thereof was measured using the FACScan apparatus (BECTON DICKINSON). As a result the dimer of MABL2-scFv <HL-5> and the MABL2-sc(Fv)₂ remarkably induced cell death of hHIAP/L1210 and CCRF-CEM in concentration-dependent manner (see Figure 43).

### 6.12 Hemagglutination Test of the purified dimer of scFv <HL-5> and the sc(Fv)₂

Hemagglutination test was carried out using different concentrations of the purified dimer of scFv <HL-5> and the purified sc(Fv)₂ in accordance with Example 5.15.

The hemagglutination was observed with the antibody MABL-2 as a positive control, whereas no hemagglutination was observed with both the single chain antibody MABL2-sc(Fv)₂ and the MABL2-scFv <HL-5>. Further, there was no substantial difference in the hemagglutination between two buffers employed with the antibody MABL-2. These results are shown in Table 3.

**TABLE 3**

| **Hemagglutination Test** | | | | | | | | | | | | | | | | |
|---|---|---|---|---|---|---|---|---|---|---|---|---|---|---|---|---|
| | **Diluent : PBS** | | | | | | | | | | | | | | (µg/ml) | |
| | cont | 28.9 | 14.45 | 7.225 | 3.6125 | 1.8063 | 0.9031 | 0.4516 | 0.2258 | 0.1129 | 0.0564 | 0.0282 | 0.0141 | 0.0071 | 0.0035 | 0.0018 |
| **MABL2-SC(Fv)₂** | - | - | - | - | - | - | - | - | - | - | - | - | - | - | - | - |
| | cont | 28.0 | 14.0 | 7.0 | 3.5 | 1.75 | 0.875 | 0.4375 | 0.2188 | 0.1094 | 0.0547 | 0.0273 | 0.0137 | 0.0068 | 0.0034 | 0.0017 |
| **MABL-2-sc(Fv) <HL5>** | - | - | - | - | - | - | - | - | - | - | - | - | - | - | - | - |
| | cont | 80 | 40 | 20 | 10 | 5 | 2.5 | 1.25 | 0.625 | 0.3125 | 0.1563 | 0.0781 | 0.0391 | 0.0195 | 0.0098 | 0.0049 |
| **MABL2 (intact)** | - | + | + | + | + | + | + | + | + | + | ± | - | - | - | - | - |
| **mlgG** | - | - | - | - | - | - | - | - | - | - | - | - | - | - | - | - |

| | **Diluent : Acetate** | | | | | | | | | | | | | | (µg/ml) | |
|---|---|---|---|---|---|---|---|---|---|---|---|---|---|---|---|---|
| | cont | 80 | 40 | 20 | 10 | 5 | 2.5 | 1.25 | 0.625 | 0.3125 | 0.1563 | 0.0781 | 0.0391 | 0.0195 | 0.0098 | 0.0049 |
| **MABL2 (intact)** | - | + | + | + | + | + | + | + | + | + | + | + | - | - | - | - |

### 6.13 Antitumor effect of the purified dimer of scFv <HL-5> and the sc(Fv)₂ for a model mouse of human myeloma

The antitumor effects were tested for the dimer of scFv <HL-5> and the sc(Fv)₂ prepared and purified in Examples 6.8 and 6.9. The test was performed by using the mouse model for human myeloma produced in Example 5.1 and determining the amount of M protein produced by human myeloma cells in the mouse serum using ELISA and examing survival time of the mice. Then, the antitumor effects of the dimer of scFv <HL-5> and the sc(Fv)₂ were evaluated in terms of the change of the amount of M protein in the mouse serum and the survival time of the mice.

In the test, the HL-5 and the sc(Fv)₂ were employed as a solution at 0.01, 0.1 or 1 mg/mL in vehicle consisting of 150 mM NaCl, 0.02% Tween and 20 mM acetate buffer, pH 6.0 and administered to the mice at 0.1, 1 or 10 mg/kg of dosage. Control group of mice were administered only with the vehicle.

The mouse serum was gathered 26 days after the transplantation of the human myeloma cells and the amount of M protein in the serum was measured using ELISA according to Example 5.14. As a result, the amount of M protein in the serum of both mice groups administered with HL-5, the dimer and the sc(Fv)₂ decreased in dose-dependent manner (see Figure 44). Furthermore, a significant elongation of the survival time was observed in both groups administered with the HL-5 (Figure 45) and with the sc(Fv)₂ (Figure 46) in comparison with the control group administered with the vehicle. These results show that the HL-5 and the sc(Fv)₂ of the invention have excellent antitumor effect in vivo.

### EXPLANATION OF DRAWINGS

Figure 1 shows the result of flow cytometry, illustrating that human IgG antibody does not bind to L1210 cells expressing human IAP (hIAP/L1210).
Figure 2 shows the result of flow cytometry, illustrating that the chimera MABL-1 antibody specifically binds to L1210 cells expressing human IAP (hIAP/L1210).

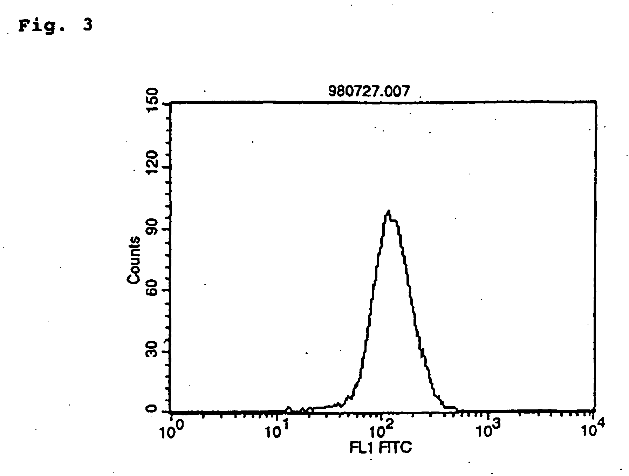

Figure 3 shows the result of flow cytometry, illustrating that the chimera MABL-2 antibody specifically binds to L1210 cells expressing human IAP (hIAP/L1210).
Figure 4 schematically illustrates the process for producing the single-chain Fv according to the present invention.
Figure 5 illustrates a structure of an expression plasmid which can be used to express a DNA encoding the single-chain Fv of the invention in E. coli.
Figure 6 illustrates a structure of an expression plasmid which is used to express a DNA encoding the single-chain Fv of the invention in mammalian cells.
Figure 7 shows a photograph showing the result of western blotting in Example 5.4. From the left, a molecular weight marker (which indicates 97.4, 66, 45, 31, 21.5 and 14.5 kDa from the top), the culture supernatant of pCHO1-introduced COS7 cells and the culture supernatant of pCHOM2-introduced COS7 cells. It illustrates that the reconstructed single-chain Fv of the antibody MABL-2 (arrow) is contained in the culture supernatant of the pCHOM2-introduced cells.
Figure 8 shows the result of flow cytometry, illustrating that an antibody in the culture supernatant of pCHO1/COS7 cell as a control does not bind to pCOS1/L1210 cell as a control.
Figure 9 shows the result of flow cytometry, illustrating that an antibody in the culture supernatant of MABL2-scFv/COS7 cells does not bind to pCOS1/L1210 cells as a control.

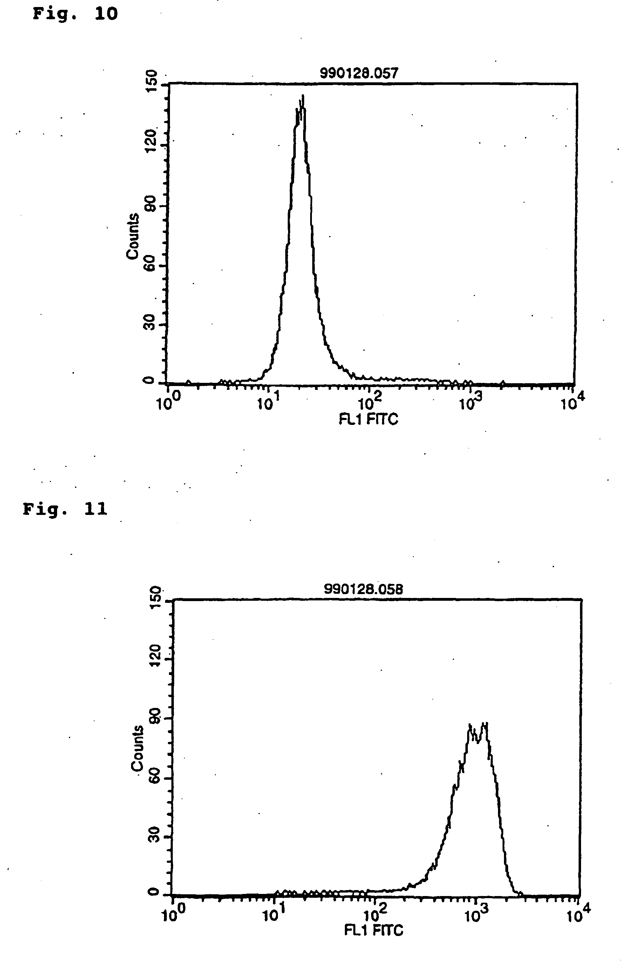

Figure 10 shows the result of flow cytometry, illustrating that an antibody in the culture supernatant of pCOS1/COS7 cells as a control does not bind to hIAP/L1210 cells.
Figure 11 shows the result of flow cytometry, illustrating that an antibody in the culture supernatant of MABL2-scFv/COS7 cells specifically binds to hIAP/L1210 cells.
Figure 12 shows the result of the competitive ELISA in Example 5.6, wherein the binding activity of the single-chain Fv of the invention (MABL2-scFv) to the antigen is demonstrated in terms of the inhibition of binding of the mouse monoclonal antibody MABL-2 to the antigen as an index, in comparison with the culture supernatant of pCHO1/COS7 cells as a control.
Figure 13 shows the results of the apoptosis-inducing effect in Example 5.7, illustrating that the antibody in the culture supernatant of pCHO1/COS7 cells as a control does not induce the apoptosis of pCOS1/L1210 cells as a control.

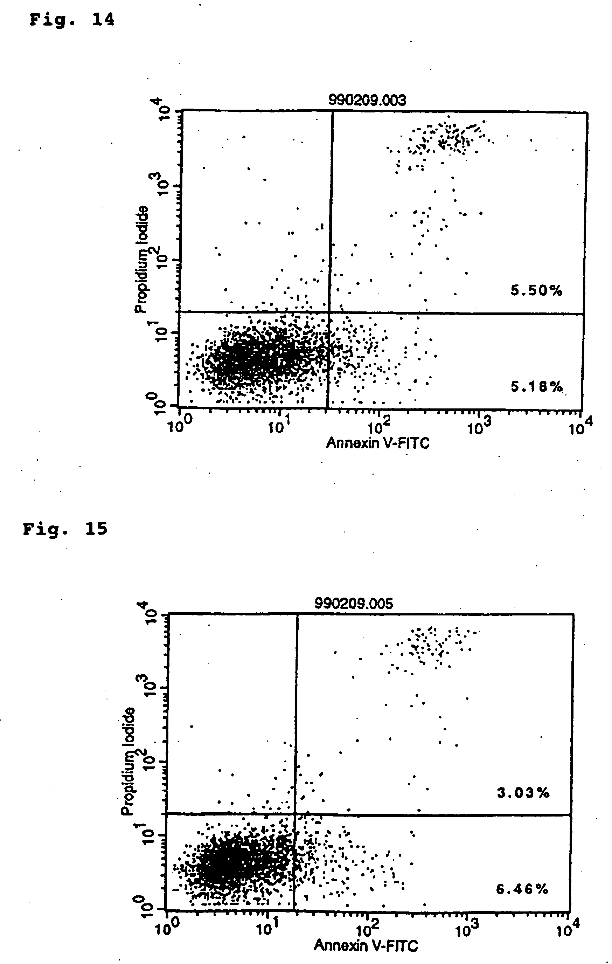

Figure 14 shows the results of the apoptosis-inducing effect in Example 5.7, illustrating that the antibody in the culture supernatant of MABL2-scFv/COS7 cells does not induce apoptosis of pCOS1/L1210 cells as a control.
Figure 15 shows the results of the apoptosis-inducing effect in Example 5.7, illustrating that the antibody in the culture supernatant of pCHO1/COS7 cells as a control does not induce apoptosis of hIAP/L1210 cells.
Figure 16 shows the results of the apoptosis-inducing effect in Example 5.7, illustrating that the antibody in the culture supernatant of MABL2-scFv/COS7 cells specifically induces apoptosis of hIAP/L1210 cells.
Figure 17 shows the results of the apoptosis-inducing effect in Example 5.7, illustrating that the antibody in the culture supernatant of pCHOl/COS7 cells as a control does not induce apoptosis of CCRF-CEM cells (at 50% of the final concentration).

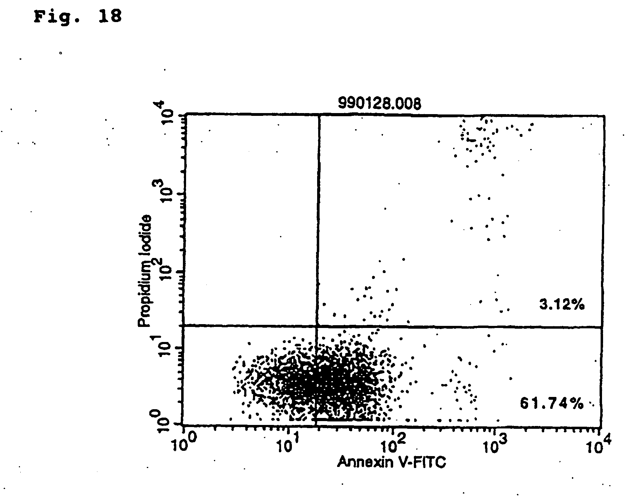

Figure 18 shows the results of the apoptosis-inducing effect in Example 5.7, illustrating that the antibody in the culture supernatant of MABL2-scFv/COS7 cells specifically induces apoptosis of CCRF-CEM cells (at 50% of the final concentration).
Figure 19 shows the chromatogram obtained in the purification of the single-chain Fv derived form the antibody MABL-2 produced by the CHO cells in Example 5.9, illustrating that fraction A and fraction B were obtained as the major peaks when the fraction from Blue-sepharose column was purified with hydroxyapatite column.
Figure 20 shows the results of purification by gel filtration of fraction A and fraction B obtained in Example 5.9-(2), illustrating that the major peaks (AI and BI, respectively) were eluted from fraction A at approximately 36 kD of the apparent molecular weight and from fraction B at approximately 76 kD.
Figure 21 is the analysis on SDS-PAGE of the fractions obtained in the purification of the single chain Fv derived from the antibody MABL-2 produced by the CHO cells in Example 5.9, illustrating that a single band of approximately 35 kD of molecular weight was observed in both fractions.
Figure 22 shows the results of analysis of fractions AI and BI obtained by gel filtration in the purification of the single-chain Fv derived from the antibody MABL-2 produced by the CHO cells, wherein fraction AI comprises monomer and fraction BI comprises dimer.
Figure 23 illustrates a structure of an expression plasmid which can be used to express a DNA encoding the single-chain Fv of the invention in E. coli.
Figure 24 shows the results of purification on the gel filtration column of crude products of the single-chain Fv polypeptide derived from the antibody MABL-2 produced by E. coli obtained in Example 5.12, wherein each peak indicates monomer or dimer, respectively, of the single-chain Fv produced by E. coli.
Figure 25 shows the results of the apoptosis-inducing effect in Example 5.13, illustrating that mouse IgG antibody as a control does not induce apoptosis of hIAP/L1210 cells (the final concentration of 3 µg/ml).
Figure 26 shows the results of the apoptosis-inducing effect in Example 5.13, illustrating that the dimer of MABL2-scFv produced by the CHO cells remarkably induces apoptosis of hIAP/L1210 cells (the final concentration of 3 µg/ml).
Figure 27 shows the results of the apoptosis-inducing effect in Example 5.13, illustrating that the dimer of MABL2-scFv produced by E. coli remarkably induces apoptosis of hIAP/L1210 cells (the final concentration of 3 µg/ml).
Figure 28 shows the results of the apoptosis-inducing effect in Example 5.13, illustrating that apoptosis induction to hIAP/L1210 cells by the MABL2-scFv monomer produced by the CHO cells is the same level as that of the control (the final concentration of 3 µg/ml).
Figure 29 shows the results of the apoptosis-inducing effect in Example 5.13, illustrating that apoptosis induction to hIAP/L1210 cells of the MABL2-scFv monomer produced by E. coli is the same level as that of control (the final concentration of 3 µg/ml).
Figure 30 shows the results of the apoptosis-inducting effect in Example 5.13, illustrating that mouse IgG antibody used as a control does not induce apoptosis of hIAP/L1210 cells even when anti-FLAG antibody is added (the final concentration of 3 µg/ml).
Figure 31 shows the results of the apoptosis-inducing effect in Example 5.13, illustrating that MABL2-scFv monomer produced by the CHO cells remarkably induces apoptosis of hIAP/L1210 cells when anti-FLAG antibody is added (the final concentration of 3 µg/ml).
Figure 32 shows the results of quantitative measurement of human IgG in the serum of a human myeloma cell line KPMM2-transplanted mouse, indicating amounts of human IgG produced by the human myeloma cells in the mouse. It illustrates that the dimer of scFv/CHO remarkably inhibited growth of the KPMM2 cells.
Figure 33 shows the survival time of the mouse after the transplantation of tumor, illustrating that the scFv/CHO dimer-administered group elongated remarkably the survival time.
Figure 34 illustrates a structure of an expression plasmid which expresses a reconstructed polypeptide [sc(Fv)₂] comprising two H chain V regions and two L chain V regions derived from the antibody MABL-2.
Figure 35 illustrates a structure of a plasmid which expresses a scFv (HL type) wherein the V regions are linked in the manner of [H chain]-[L chain] without a peptide linker.
Figure 36 illustrates a structure of the HL-type polypeptide and amino acid sequences of peptide linkers.
Figure 37 illustrates a structure of a plasmid which expresses a scFv (LH type) wherein the V regions are linked in the manner of [L chain]-[H chain] without a peptide linker.
Figure 38 illustrates a structure of the LH-type polypeptide and amino acid sequences of peptide linkers.
Figure 39 shows the results of the western blotting in Example 6.4, illustrating that the reconstructed polypeptide sc(FV)₂ comprising two H chain V regions and two L chain V regions, and the MABL2-scFv having peptide linkers with different length are expressed.
Figures 40a and 40b show the results of flow cytometry using the culture supernatant of COS7 cells prepared in Example 6.3 (1), illustrating that the MABL2-scFv and sc(Fv)₂ having peptide linkers with different length have high affinities against human IAP.
Figure 41 shows the results of the apoptosis-inducing effect in Example 6.6, illustrating that the scFv <HL3, 4, 6, 7, LH3, 4, 6 and 7> and the sc(Fv)2 remarkably induce cell death of hIAP/L1210 cells.
Figure 42 shows the results of the evaluation of antigen binding capacity in Example 6.10, illustrating that the dimer of scFv <HL5> and sc(Fv)₂ have high affinities against human IAP.
Figure 43 shows the results of the in vitro apoptosis-inducing effect in Example 6.11, illustrating that the dimer of scFv <HL5> and the sc(Fv)₂ induce apoptosis of hIAP/L1210 cells and CCRF-CEM cells in concentration-dependent manner.

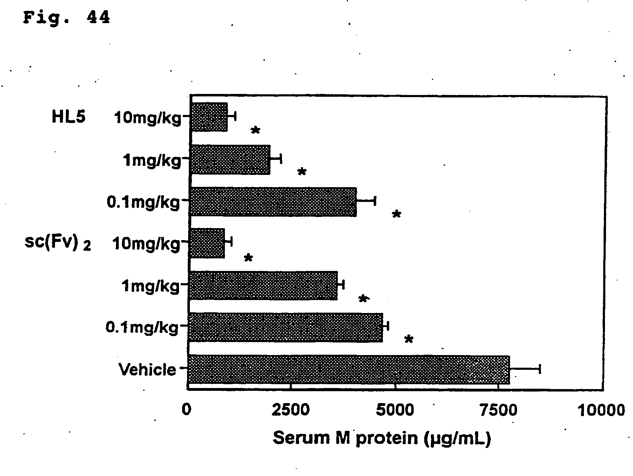

Figure 44 shows the results of the quantitative measurement of M protein produced by a human myeloma cell line KPMM2 in the serum of the human myeloma cell-transplanted mouse. It illustrates that the dimer of scFv <HL5> and the sc(Fv)₂ remarkably inhibited growth of the KPMM2 cells.

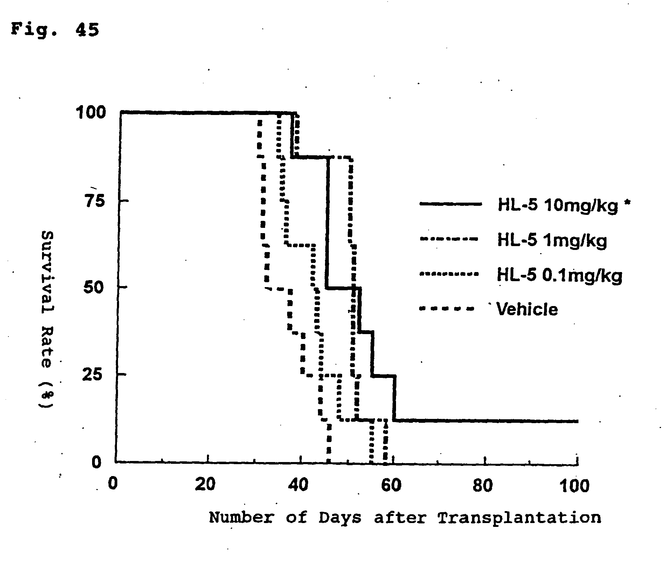

Figure 45 shows the survival time (days) of mice after the transplantation of tumor, illustrating that the survival time of the scFv <HL5> administrated-group was remarkably prolonged.

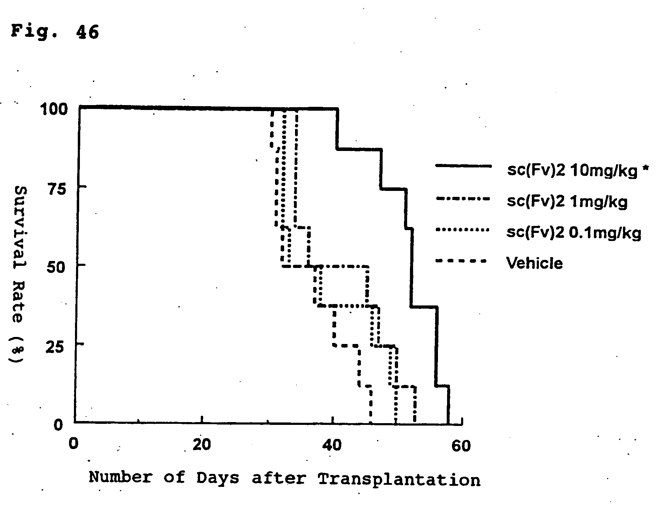

Figure 46 shows the survival time (days) of mice after the transplantation of tumor, illustrating that the survival time of the sc(Fv)₂ administrated-group was remarkably prolonged.

### INDUSTRIAL APPLICABILITY

The reconstructed polypeptides of the invention have properties of inducing apoptosis of nucleated blood cells having Integrin Associated Protein (IAP) and causing no hemagglutination, and are useful as a therapeutic agent for blood dyscrasia, for example, leukemia such as acute myeloid leukemia, chronic myelogenous leukemia, acute lymphoblastic leukemia, chronic lymphocytic leukemia, adult T-cell leukemia, multiple myeloma, mixed leukemia and hairy cell leukemia, malignant lymphoma (Hodgkin's disease, non-Hodgkin's lymphoma), aplastic anemia, myelodysplasia syndrome and polycythemia vera.

### SEQUENCE LISTING .

<110> CHUGAI SEIYAKU KABUSHIKI KAISHA
   <120> Apoptosis-inducible polypeptide
   <130> FOP-415
   <141> 2001-3-12
   <150> US 09/523,095
   <151> 2000-3-10
   <150> JP2000-115246
   <151> 2000-04-17
   <150> JP2000-321822
   <151> 2000-10-20
<160> 54
<210> 1
   <211> 27
   <212> DNA
   <213> Artificial Sequence
   <220>
   <223> PCR primer
   <400> 1
   ccatcctaat acgactcact atagggc 27
<210> 2
   <211> 27
   <212> DNA
   <213> Artificial Sequence
   <220>
   <223> PCR. primer
   <400> 2
   ggatcccggg tggatggtgg gaagata 27
<210> 3
   <211> 28
   <212> DNA
   <213> Artificial Sequence
   <220>
   <223> PCR primer
   <400> 3
   ggatcccggg ccagtggata gacagatg 28
<210> 4
   <211> 26
   <212> DNA
   <213> Artificial Sequence
   <220>
   <223> PCR primer
   <400> 4
   ggatcccggg agtggataga ccgatg 26
<210> 5
   <211> 394
   <212> DNA
   <213> Mus
   <220>
   <221> CDS
   <222> (1)...(393)
   <223> pGEM-M1L.1~57;signal peptide,58~394;mature peptide
   <400> 5
   atg aag ttg cct gtt agg ctg ttg gtg ctg atg ttc tgg att cct 45
   Met Lys Leu Pro Val Arg Leu Leu Val Leu Met Phe Trp Ile Pro
   5 10 15
   gcg tcc agc agt gat gtt gtg atg acc caa act cca ctc tcc ctg 90
   Ala Ser Ser Ser Asp Val Val Met Thr Gln Thr Pro Leu Ser Leu
   20 25 30
   cct gtc agt ctt gga gat caa gcc tcc atc tct tgc aga tct agt 135
   Pro Val Ser Leu Gly Asp Gln Ala Ser Ile Ser Cys Arg Ser Ser
   35 40 45
   cag agc ctt cta cac agt aaa gga aac acc tat tta caa tgg tac 180
   Gln Ser Leu Leu His Ser Lys Gly Asn Thr Tyr Leu Gln Trp Tyr
   50 55 60
   cta cag aag cca ggc cag tct cca aag ctc ctg atc tac aaa gtt 225
   Leu Gln Lys Pro Gly Gln Ser Pro Lys Leu Leu Ile Tyr Lys Val
   65 70 75
   tcc aac cga ttt tct ggg gtc cca gac agg ttc agt ggc agt gga 270
   Ser Asn Arg Phe Ser Gly Val Pro Asp Arg Phe Ser Gly Ser Gly
   80 85 90
   tca ggg aca gat ttc aca ctc aag atc agc aga gtg gag gct gag 315
   Ser Gly Thr Asp Phe Thr Leu Lys Ile Ser Arg Val Glu Ala Glu
   95 100 105
   gat ctg gga gtt tat ttc tgc tct caa agt aca cat gtt ccg tac 360
   Asp Leu Gly Val Tyr Phe Cys Ser Gln Ser Thr His Val Pro Tyr
   110 115 120
   acg tcc gga ggg ggg acc aag ctg gaa ata aaa c 394
   Thr Ser Gly Gly Gly Thr Lys Leu Glu Ile Lys
   125 130 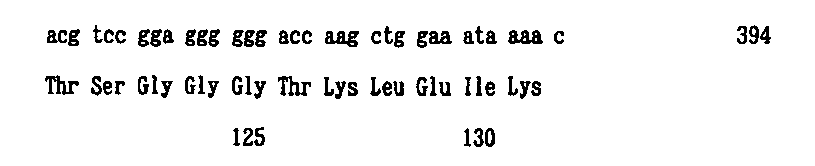
<210> 6
   <211> 409
   <212> DNA
   <213> Mus
   <220>
   <221> CDS
   <222> (1)...(408)
   <223> pGEM-M1H.1~57;signal peptide,58~409;mature peptide
   <400> 6
   atg gaa tgg agc tgg ata ttt ctc ttc ctc ctg tca gga act gca  45
   Met Glu Trp Ser Trp Ile Phe Leu Phe Leu Leu Ser Gly Thr Ala
                     5                  10                  15
   ggt gtc cac tcc cag gtc cag ctg cag cag tct gga cct gac ctg  90
   Gly Val His Ser Gln Val Gln Leu Gln Gln Ser Gly Pro Asp Leu
                    10                  25                  30
   gta aag cct ggg gct tca gtg aag atg tcc tgc aag gct tct gga 135
   Val Lys Pro Gly Ala Ser Val Lys Met Ser Cys Lys Ala Ser Gly
                    35                  40                  45
   tac acc ttc gtt aac cat gtt atg cac tgg gtg aag cag aag cca 180
   Tyr Thr Phe Val Asn His Val Met His Trp Val Lys Gln Lys Pro
                    50                  55                  60
   ggg cag ggc ctt gag tgg att gga tat att tat cct tac aat gat 225
   Gly Gln Gly Leu Glu Trp Ile Gly Tyr Ile Tyr Pro Tyr Asn Asp
                    65                  70                  75
   ggt act aag tac aat gag aag ttc aag ggc aag gcc aca ctg act 270
   Gly Thr Lys Tyr Asn Glu Lys Phe Lys Gly Lys Ala Thr Leu Thr
   80 85 90
   tca gag aaa tcc tcc agc gca gcc tac atg gag ctc agc agc ctg 315
   Ser Glu Lys Ser Ser Ser Ala Ala Tyr Met Glu Leu Ser Ser Leu
   95 100 105
   gcc tct gag gac tct gcg gtc tac tac tgt gca aga ggg ggt tac 360
   Ala Ser Glu Asp Ser Ala Val Tyr Tyr Cys Ala Arg Gly Gly Tyr
   110 115 120
   tat agt tac gac gac tgg ggc caa ggc acc act ctc aca gtc tcc 405
   Tyr Ser Tyr Asp Asp Trp Gly Gln Gly Thr Thr Leu Thr Val Ser
   125 130 135
   tca g 409
   Ser
<210> 7
   <211> 394
   <212> DNA
   <213> Mus
   <220>
   <221> CDS
   <222> (1)...(393)
   <223> pGEM-M2L. 1~57;signal peptide, 58-394;nature peptide
   <400> 7
   atg aag ttg cct gtt agg ctg ttg gtg ctg atg ttc tgg att cct 45
   Met Lys Leu Pro Val Arg Leu Leu Val Leu Met Phe Trp Ile Pro
   5 10 15
   ggt tcc agc agt gat gtt gtg atg acc caa agt cca ctc tcc ctg 90
   Gly Ser Ser Ser Asp Val Val Met Thr Gln Ser Pro Leu Ser Leu
   20 25 30
   cct gtc agt ctt gga gat caa gcc tcc atc tct tgc aga tca agt 135
   Pro Val Ser Leu Gly Asp Gln Ala Ser Ile Ser Cys Arg Ser Ser
   35 40 45
   cag agc ctt gtg cac agt aat gga aag acc tat tta cat tgg tac 180
   Gln Ser Leu Val His Ser Asn Gly Lys Thr Tyr Leu His Trp Tyr
   50 55 60
   ctg cag aag cca ggc cag tct cca aaa ctc ctg atc tac aaa gtt 225
   Leu Gln Lys Pro Gly Gln Ser Pro Lys Leu Leu Ile Tyr Lys Val
   65 70 75
   tcc aac cga ttt tct ggg gtc cca gac agg ttc agt ggc agt gga 270
   Ser Asn Arg Phe Ser Gly Val Pro Asp Arg Phe Ser Gly Ser Gly
   80 85 90
   tca gtg aca gat ttc aca ctc atg atc agc aga gtg gag gct gag 315
   Ser Val Thr Asp Phe Thr Leu Met Ile Ser Arg Val Glu Ala Glu
   95 100 105
   gat ctg gga gtt tat ttc tgc tct caa agt aca cat gtt ccg tac 360
   Asp Leu Gly Val Tyr Phe Cys Ser Gln Ser Thr His Val Pro Tyr
   110 115 120
   acg ttc gga ggg ggg acc aag ctg gaa ata aaa c 394
   Thr Phe Gly Gly Gly Thr Lys Leu Glu Ile Lys
   125 130
<210> 8
   <211> 409
   <212> DNA
   <213> Mus
   <220>
   <221> CDS
   <222> (1)...(408)
   <223> pGEM-M2H. 1~57;signal peptide, 58-409;mature peptide
   <400> 8
   atg gaa tgg agc tgg ata ttt ctc ttc ctc ctg tca gga act gca 45
   Met Glu Trp Ser Trp Ile Phe Leu Phe Leu Leu Ser Gly Thr Ala
   5 10 15
   ggt gtc cac tcc cag gtc cag ctg cag cag tct gga cct gaa ctg 90
   Gly Val His Ser Gln Val Gln Leu Gln Gln Ser Gly Pro Glu Leu
   20 25 30
   gta aag cct ggg gct tca gtg aag atg tcc tgc aag gct tct gga 135
   Val Lys Pro Gly Ala Ser Val Lys Met Ser Cys Lys Ala Ser Gly
   35 40 45
   tac acc ttc gct aac cat gtt att cac tgg gtg aag cag aag cca 180
   Tyr Thr Phe Ala Asn His Val Ile His Trp Val Lys Gln Lys Pro
   50 55 60
   ggg cag ggc ctt gag tgg att gga tat att tat cct tac aat gat 225
   Gly Gln Gly Leu Glu Trp Ile Gly Tyr Ile Tyr Pro Tyr Asn Asp
   65 70 75
   ggt act aag tat aat gag aag ttc aag gac aag gcc act ctg act 270
   Gly Thr Lys Tyr Asn Glu Lys Phe Lys Asp Lys Ala Thr Leu Thr
   80 85 90
   tca gac aaa tcc tcc acc aca gcc tac atg gac ctc agc agc ctg 315
   Ser Asp Lys Ser Ser Thr Thr Ala Tyr Met Asp Leu Ser Ser Leu
   95 100 105
   gcc tct gag gac tct gcg gtc tat tac tgt gca aga ggg ggt tac 360
   Ala Ser Glu Asp Ser Ala Val Tyr Tyr Cys Ala Arg Gly Gly Tyr
   110 115 120
   tat act tac gac gac tgg ggc caa ggc acc act ctc aca gtc tcc 405
   Tyr Thr Tyr Asp Asp Trp Gly Gln Gly Thr Thr Leu Thr Val Ser
   125 130 135
   tca g 409
   Ser
<210> 9
   <211> 32
   <212> DNA
   <213> Artificial Sequence
   <220>
   <223> PCR primer
   <400> 9
   cccaagcttc caccatgaag ttgcctgtta gg 32
<210> 10
   <211> 32
   <212> DNA
   <213> Artificial Sequence
   <220>
   <223> PCR primer
   <400> 10
   cccaagcttc caccatggaa tggagctgga ta 32
<210> 11
   <211> 34
   <212> DNA.
   <213> Artificial Sequence
   <220>
   <223> PCR primer
   <400> 11
   cgcggatcca ctcacgtttt atttccagct tggt 34
<210> 12
   <211> 34
   <212> DNA
   <213> Artificial Sequence
   <220>
   <223> PCR primer
   <400> 12
   cgcggatcca ctcacctgag gagactgtga gagt 34
<210> 13
   <211> 30
   <212> DNA
   <213> Artificial Sequence
   <220> -
   <223> PCR primer
   <400> 13
   catgccatgg cgcaggtcca gctgcagcag 30
<210> 14
   <211> 27
   <212> DNA
   <213> Artificial Sequence
   <220>
   <223> PCR primer
   <400> 14
   accaccacct gaggagactg tgagagat 27
<210> 15
   <211> 27
   <212> DNA
   <213> Artificial Sequence
   <220>
   <223> PCR primer
   <400> 15
   gtctcctcag gtggtggtgg ttccgggt 27
<210> 16
   <211> 27
   <212> DNA
   <213> Artificial Sequence
<220>
   <223> PCR primer
<400> 16
   cacaacatcc gatccgccac cacccga 27
<210> 17
   <211> 27
   <212> DNA
   <213> Artificial Sequence
<220>
   <223> PCR primer
<400> 17
   ggcggatcgg atgttgtgatgacccaa 27
<210> 18
   <211> 57
   <212> DNA
   <213> Artificial Sequence
<220>
   <223> PCR primer
<400> 18
   ccggaattct cattatttat cgtcatcgtc tttgtagtct tttatttcca gcttggt 57
<210> 19
   <211> 45
   <212> DNA
   <213> Artificial Sequence
<220>
   <223> Linker amino acid sequence and nucleotide sequence
<400> 19
   ggt ggt ggt ggt tcg ggt ggt ggt ggt tcg ggt ggt ggc gga tcg 45
   Gly Gly Gly Gly Ser Gly Gly Gly Gly Ser Gly Gly Gly Gly Ser
   5 10 15
<210> 20
   <211> 828
   <212> DNA
   <213> Mus
<220>
   <221>CDS
   <222>(1)...(826)
   <223> pscM1. MABL1-scFv
<400> 20
   atg aaa tac cta ttg cct acg gca gcc gct gga ttg tta tta ctc 45
   Met Lys Tyr Leu Leu Pro Thr Ala Ala Ala Gly Leu Leu Leu Leu
   5 10 15
   gct gcc caa cca gcc atg gcg cag gtc cag ctg cag cag tct gga 90
   Ala Ala Gln Pro Ala Met Ala Gln Val Gln Leu Gln Gln Ser Gly
   20 25 30
   cct gac ctg gta aag cct ggg gct tca gtg aag atg tcc tgc aag 135
   Pro Asp Leu Val Lys Pro Gly Ala Ser Val Lys Met Ser Cys Lys
   35 40 45
   gct tct gga tac acc ttc gtt aac cat gtt atg cac tgg gtg aag 180
   Ala Ser Gly Tyr Thr Phe Val Asn His Val Met His Trp Val Lys
   50 55 60
   cag aag cca ggg cag ggc ctt gag tgg att gga tat att tat cct 225
   Gln Lys Pro Gly Gln Gly Leu Glu Trp Ile Gly Tyr Ile Tyr Pro
   65 70 75
   tac aat gat ggt act aag tac aat gag aag ttc aag ggc aag gcc 270
   Tyr Asn Asp Gly Thr Lys Tyr Asn Glu Lys Phe Lys Gly Lys Ala
   80 85 90
   aca ctg act tca gag aaa tcc tcc agc gca gcc tac atg gag ctc 315
   Thr Leu Thr Ser Glu Lys Ser Ser Ser Ala Ala Tyr Met Glu Leu
   95 100 105
   agc agc ctg gcc tct gag gac tct gcg gtc tac tac tgt gca aga 360
   Ser Ser Leu Ala Ser Glu Asp Ser Ala Val Tyr Tyr Cys Ala Arg
   110 115 120
   ggg ggt tac tat agt tac gac gac tgg ggc caa ggc acc act ctc 405
   Gly Gly Tyr Tyr Ser Tyr Asp Asp Trp Gly Gln Gly Thr Thr Leu
   125 130 135
   aca gtc tcc tca ggt ggt ggt ggt tcg ggt ggt ggt ggt tcg ggt 450
   Thr Val Ser Ser Gly Gly Gly Gly Ser Gly Gly Gly Gly Ser Gly
   140 145 150
   ggt ggc gga tcg gat gtt gtg atg acc caa act cca ctc tcc ctg 495
   Gly Gly Gly Ser Asp Val Val Met Thr Gln Thr Pro Leu Ser Leu
   155 160 165
   cct gtc agt ctt gga gat caa gcc tcc atc tct tgc aga tct agt 540
   Pro Val Ser Leu Gly Asp Gln Ala Ser Ile Ser Cys Arg Ser Ser
   170 175 180
   cag agc ctt cta cac agt aaa gga aac acc tat tta caa tgg tac 585
   Gln Ser Leu Leu His Ser Lys Gly Asn Thr Tyr Leu Gln Trp Tyr
   185 190 195
   cta cag aag cca ggc cag tct cca aag ctc ctg atc tac aaa gtt 630
   Leu Gln Lys Pro Gly Gln Ser Pro Lys Leu Leu Ile Tyr Lys Val
   200 205 210
   tcc aac cga ttt tct ggg gtc cca gac agg ttc agt ggc agt gga 675
   Ser Asn Arg Phe Ser Gly Val Pro Asp Arg Phe Ser Gly Ser Gly
   215 220 225
   tca ggg aca gat ttc aca ctc aag atc agc aga gtg gag gct gag 720
   Ser Gly Thr Asp Phe Thr Leu Lys Ile Ser Arg Val Glu Ala Glu
   230 235 240
   gat ctg gga gtt tat ttc tgc tct caa agt aca cat gtt ccg tac 765
   Asp Leu Gly Val Tyr Phe Cys Ser Gln Ser Thr His Val Pro Tyr
   245 250 255
   acg tcc gga ggg ggg acc aag ctg gaa ata aaa gac tac aaa gac 810
   Thr Ser Gly Gly Gly Thr Lys Leu Glu Ile Lys Asp Tyr Lys Asp
   260 265 270
   gat gac gat aaa taa tga 828
   Asp Asp Asp Lys
<210> 21
   <211> 31 .
   <212> DNA
   <213> Artificial Sequence
<220>
   <223> PCR primer
<400> 21
   acgcgtcgac tcccaggtcc agctgcagca g 31
<210> 22
   <211> 18
   <212> DNA
   <213> Artificial Sequence
<220>
   <223> PCR primer
<400> 22
   gaaggtgtat ccagaagc 18
<210> 23
   <211> 819
   <212> DNA
   <213> Mus
<220>
   <221> CDS
   <222>(1)...(813)
   <223> pCHOM1. MABL1-scFv
<400> 23
   atg gga tgg agc tgt atc atc ctc ttc ttg gta gca aca gct aca 45
   Met Gly Trp Ser Cys Ile Ile Leu Phe Leu Val Ala Thr Ala Thr
   5 10 15
   ggt gtc gac tcc cag gtc cag ctg cag cag tct gga cct gac ctg 90
   Gly Val Asp Ser Gln Val Gln Leu Gln Gln Ser Gly Pro Asp Leu
   20 25 30
   gta aag cct ggg gct tca gtg aag atg tcc tgc aag gct tct gga 135
   Val Lys Pro Gly Ala Ser Val Lys Met Ser Cys Lys Ala Ser Gly
   35 40 45
   tac acc ttc gtt aac cat gtt atg cac tgg gtg aag cag aag cca 180
   Tyr Thr Phe Val Asn His Val Met His Trp Val Lys Gln Lys Pro
   50 55 60
   ggg cag ggc ctt gag tgg att gga tat att tat cct tac aat gat 225
   Gly Gln Gly Leu Glu Trp Ile Gly Tyr Ile Tyr Pro Tyr Asn Asp
   65 70 75
   ggt act aag tac aat gag aag ttc aag ggc aag gcc aca ctg act 270
   Gly Thr Lys Tyr Asn Glu Lys Phe Lys Gly Lys Ala Thr Leu Thr
   80 85 90
   tca gag aaa tcc tcc agc gca gcc tac atg gag ctc agc agc ctg 315
   Ser Glu Lys Ser Ser Ser Ala Ala Tyr Met Glu Leu Ser Ser Leu
   95 100 105
   gcc tct gag gac tct gcg gtc tac tac tgt gca aga ggg ggt tac 360
   Ala Ser Glu Asp Ser Ala Val Tyr Tyr Cys Ala Arg Gly Gly Tyr
   110 115 120
   tat agt tac gac gac tgg ggc caa ggc acc act ctc aca gtc tcc 405
   Tyr Ser Tyr Asp Asp Trp Gly Gln Gly Thr Thr Leu Thr Val Ser
   125 130 135
   tca ggt ggt ggt ggt tcg ggt ggt ggt ggt tcg ggt ggt ggc gga 450
   Ser Gly Gly Gly Gly Ser Gly Gly Gly Gly Ser Gly Gly Gly Gly
   140 145 150
   tcg gat gtt gtg atg acc caa act cca ctc tcc ctg cct gtc agt 495
   Ser Asp Val Val Met Thr Gln Thr Pro Leu Ser Leu Pro Val Ser
   155 160 165
   ctt gga gat caa gcc tcc atc tct tgc aga tct agt cag agc ctt 540
   Leu Gly Asp Gln Ala Ser Ile Ser Cys Arg Ser Ser Gln Ser Leu
   170 175 180
   cta cac agt aaa gga aac acc tat tta caa tgg tac cta cag aag 585
   Leu His Ser Lys Gly Asn Thr Tyr Leu Gln Trp Tyr Leu Gln Lys
   185 190 195
   cca ggc cag tct cca aag ctc ctg atc tac aaa gtt tcc aac cga 630
   Pro Gly Gln Ser Pro Lys Leu Leu Ile Tyr Lys Val Ser Asn Arg
   200 205 210
   TTT TCT GGG GTC CCA GAC AGG TTC AGT GGC AGT GGA TCA GGG ACA 675
   Phe Ser Gly Val Pro Asp Arg Phe Ser Gly Ser Gly Ser Gly Thr
   215 220 225
   gat ttc aca ctc aag atc agc aga gtg gag gct gag gat ctg gga 720
   Asp Phe Thr Leu Lys Ile Ser Arg Val Glu Ala Glu Asp Leu Gly
   230 235 240
   gtt tat ttc tgc tct caa agt aca cat gtt ccg tac acg tcc gga 765
   Val Tyr Phe Cys Ser Gln Ser Thr His Val Pro Tyr Thr Ser Gly
   245 250 255
   ggg ggg acc aag ctg gaa ata aaa gac tac aaa gac gat gac gat 810
   Gly Gly Thr Lys Leu Glu Ile Lys Asp Tyr Lys Asp Asp Asp Asp
   260 265 270
   aaa taa tga 819
   Lys
<210> 24
   <211> 828
   <212> DNA
   <213> Mus
<220>
   <221> CDS
   <222> (1)...(822)
   <223> pscM2. MABL2-scFv
<400> 24
   atg aaa tac cta ttg cct acg gca gcc gct gga ttg tta tta ctc 45
   Met Lys Tyr Leu Leu Pro Thr Ala Ala Ala Gly Leu Leu Leu Leu
   5 10 15
   gct gcc caa cca gcc atg gcg cag gtc cag ctg cag cag tct gga 90
   Ala Ala Gln Pro Ala Met Ala Gln Val Gln Leu Gln Gln Ser Gly
   20 25 30
   cct gaa ctg gta aag cct ggg gct tca gtg aag atg tcc tgc aag 135
   Pro Glu Leu Val Lys Pro Gly Ala Ser Val Lys Met Ser Cys Lys
   35 40 45
   gct tct gga tac acc ttc gct aac cat gtt att cac tgg gtg aag 180
   Ala Ser Gly Tyr Thr Phe Ala Asn His Val Ile His Trp Val Lys
   50 55 60
   cag aag cca ggg cag ggc ctt gag tgg att gga tat att tat cct 225
   Gln Lys Pro Gly Gln Gly Leu Glu Trp Ile Gly Tyr Ile Tyr Pro
   65 70 75
   tac aat gat ggt act aag tat aat gag aag ttc aag gac aag gcc 270
   Tyr Asn Asp Gly Thr Lys Tyr Asn Glu Lys Phe Lys Asp Lys Ala
   80 85 90
   act ctg act tca gac aaa tcc tcc acc aca gcc tac atg gac ctc 315
   Thr Leu Thr Ser Asp Lys Ser Ser Thr Thr Ala Tyr Met Asp Leu
   95 100 105
   agc agc ctg gcc tct gag gac tct gcg gtc tat tac tgt gca aga 360
   Ser Ser Leu Ala Ser Glu Asp Ser Ala Val Tyr Tyr Cys Ala Arg
   110 115 120
   ggg ggt tac tat act tac gac gac tgg ggc caa ggc acc act ctc 405
   Gly Gly Tyr Tyr Thr Tyr Asp Asp Trp Gly Gln Gly Thr Thr Leu
   125 130 135
   aca gtc tcc tca ggt ggt ggt ggt tcg ggt ggt ggt ggt tcg ggt 450
   Thr Val Ser Ser Gly Gly Gly Gly Ser Gly Gly Gly Gly Ser Gly
   140 145 150
   ggt ggc gga tcg gat gtt gtg atg acc caa agt cca ctc tcc ctg 495
   Gly Gly Gly Ser Asp Val Val Met Thr Gln Ser Pro Leu Ser Leu
   155 160 165
   cct gtc agt ctt gga gat caa gcc tcc atc tct tgc aga tca agt 540
   Pro Val Ser Leu Gly Asp Gln Ala Ser Ile Ser Cys Arg Ser Ser
   170 175 180
   cag agc ctt gtg cac agt aat gga aag acc tat tta cat tgg tac 585
   Gln Ser Leu Val His Ser Asn Gly Lys Thr Tyr Leu His Trp Tyr
   185 190 195
   ctg cag aag cca ggc cag tct cca aaa ctc ctg atc tac aaa gtt 630
   Leu Gln Lys Pro Gly Gln Ser Pro Lys Leu Leu Ile Tyr Lys Val
   200 205 210
   tcc aac cga ttt tct ggg gtc cca gac agg ttc agt ggc agt gga 675
   Ser Asn Arg Phe Ser Gly Val Pro Asp Arg Phe Ser Gly Ser Gly
   215 220 225
   tca gtg aca gat ttc aca ctc atg atc agc aga gtg gag gct gag 720
   Ser Val Thr Asp Phe Thr Leu Met Ile Ser Arg Val Glu Ala Glu
   230 235 240
   gat ctg gga gtt tat ttc tgc tct caa agt aca cat gtt ccg tac 765
   Asp Leu Gly Val Tyr Phe Cys Ser Gln Ser Thr His Val Pro Tyr
   245 250 255
   acg ttc gga ggg ggg acc aag ctg gaa ata aaa gac tac aaa gac 810
   Thr Phe Gly Gly Gly Thr Lys Leu Glu Ile Lys Asp Tyr Lys Asp
   260 265 270
   gat gac gat aaa taa tga 828
   Asp Asp Asp Lys
<210> 25
   <211> 819
   <212> DNA
   <213> Mus
<220>
   <221> CDS
   <222> (1)...(813)
   <223> pCHOM2. MABL2-scFv
<400> 25
   atg gga tgg agc tgt atc atc ctc ttc ttg gta gca aca gct aca 45
   Met Gly Trp Ser Cys Ile Ile Leu Phe Leu Val Ala Thr Ala Thr
   5 10 15
   ggt gtc gac tcc cag gtc cag ctg cag cag tct gga cct gaa ctg 90
   Gly Val Asp Ser Gln Val Gln Leu Gln Gln Ser Gly Pro Glu Leu
   20 25 30
   gta aag cct ggg gct tca gtg aag atg tcc tgc aag gct tct gga 135
   Val Lys Pro Gly Ala Ser Val Lys Met Ser Cys Lys Ala Ser Gly
   35 40 45
   tac acc ttc gct aac cat gtt att cac tgg gtg aag cag aag cca 180
   Tyr Thr Phe Ala Asn His Val Ile His Trp Val Lys Gln Lys Pro
   50 55 60
   ggg cag ggc ctt gag tgg att gga tat att tat cct tac aat gat 225
   Gly Gln Gly Leu Glu Trp Ile Gly Tyr Ile Tyr Pro Tyr Asn Asp
   65 70 75
   ggt act aag tat aat gag aag ttc aag gac aag gcc act ctg act 270
   Gly Thr Lys Tyr Asn Glu Lys Phe Lys Asp Lys Ala Thr Leu Thr
   80 85 90
   tca gac aaa tcc tcc acc aca gcc tac atg gac ctc agc agc ctg 315
   Ser Asp Lys Ser Ser Thr Thr Ala Tyr Met Asp Leu Ser Ser Leu
   95 100 105
   gcc tct gag gac tct gcg gtc tat tac tgt gca aga ggg ggt tac 360
   Ala Ser Glu Asp Ser Ala Val Tyr Tyr Cys Ala Arg Gly Gly Tyr
   110 115 120
   tat act tac gac gac tgg ggc caa ggc acc act ctc aca gtc tcc 405
   Tyr Thr Tyr Asp Asp Trp Gly Gln Gly Thr Thr Leu Thr Val Ser
   125 130 135
   tca ggt ggt ggt ggt tcg ggt ggt ggt ggt tcg ggt ggt ggc gga 450
   Ser Gly Gly Gly Gly Ser Gly Gly Gly Gly Ser Gly Gly Gly Gly
   140 145 150
   tcg gat gtt gtg atg acc caa agt cca ctc tcc ctg cct gtc agt 495
   Ser Asp Val Val Met Thr Gln Ser Pro Leu Ser Leu Pro Val Ser
   155 160 165
   ctt gga gat caa gcc tcc atc tct tgc aga tca agt cag agc ctt 540
   Leu Gly Asp Gln Ala Ser Ile Ser Cys Arg Ser Ser Gln Ser Leu
   170 175 180
   gtg cac agt aat gga aag acc tat tta cat tgg tac ctg cag aag 585
   Val His Ser Asn Gly Lys Thr Tyr Leu His Trp Tyr Leu Gln Lys
   185 190 195
   cca ggc cag tct cca aaa ctc ctg atc tac aaa gtt tcc aac cga 630
   Pro Gly Gln Ser Pro Lys Leu Leu Ile Tyr Lys Val Ser Asn Arg
   200 205 210
   ttt tct ggg gtc cca gac agg ttc agt ggc agt gga tca gtg aca 675
   Phe Ser Gly Val Pro Asp Arg Phe Ser Gly Ser Gly Ser Val Thr
   215 220 225
   gat ttc aca ctc atg atc agc aga gtg gag gct gag gat ctg gga 720
   Asp Phe Thr Leu Met Ile Ser Arg Val Glu Ala Glu Asp Leu Gly
   230 235 240
   gtt tat ttc tgc tct caa agt aca cat gtt ccg tac acg ttc gga 765
   Val Tyr Phe Cys Ser Gln Ser Thr His Val Pro Tyr Thr Phe Gly
   245 250 255
   ggg ggg acc aag ctg gaa ata aaa gac tac aaa gac gat gac gat 810
   Gly Gly Thr Lys Leu Glu Ile Lys Asp Tyr Lys Asp Asp Asp Asp
   260 265 270
   aaa taa tga 819
   Lys
<210> 26
   <211> 456
   <212> DNA
   <213> Mus
<220>
   <221> CDS
   <222> (1)...(450)
   <223> pCHO-shIAP. Soluble human IAP
<400> 26
   atg tgg ccc ctg gta gcg gcg ctg ttg ctg ggc tcg gcg tgc tgc 45
   Met Trp Pro Leu Val Ala Ala Leu Leu Leu Gly Ser Ala Cys Cys
   5 10 15
   gga tca gct cag cta cta ttt aat aaa aca aaa tct gta gaa ttc 90
   Gly Ser Ala Gln Leu Leu Phe Asn Lys Thr Lys Ser Val Glu Phe
   20 25 30
   acg ttt tgt aat gac act gtc gtc att cca tgc ttt gtt act aat 135
   Thr Phe Cys Asn Asp Thr Val Val Ile Pro Cys Phe Val Thr Asn
   35 40 45
   atg gag gca caa aac act act gaa gta tac gta aag tgg aaa ttt 180
   Met Glu Ala Gln Asn Thr Thr Glu Val Tyr Val Lys Trp Lys Phe
   50 55 60
   aaa gga aga gat att tac acc ttt gat gga gct cta aac aag tcc 225
   Lys Gly Arg Asp Ile Tyr Thr Phe Asp Gly Ala Leu Asn Lys Ser
   65 70 75
   act gtc ccc act gac ttt agt agt gca aaa att gaa gtc tca caa 270
   Thr Val Pro Thr Asp Phe Ser Ser Ala Lys Ile Glu Val Ser Gln
   80 85 90
   tta cta aaa gga gat gcc tct ttg aag atg gat aag agt gat gct 315
   Leu Leu Lys Gly Asp Ala Ser Leu Lys Met Asp Lys Ser Asp Ala
   95 100 105
   gtc tca cac aca gga aac tac act tgt gaa gta aca gaa tta acc 360
   Val Ser His Thr Gly Asn Tyr Thr Cys Glu Val Thr Glu Leu Thr
   110 115 120
   aga gaa ggt gaa acg atc atc gag cta aaa tat cgt gtt gtt tca 405
   Arg Glu Gly Glu Thr Ile Ile Glu Leu Lys Tyr Arg Val Val Ser
   125 130 135
   tgg ttt tct cca aat gaa aat gac tac aag gac gac gat gac aag 450
   Trp Phe Ser Pro Asn Glu Asn Asp Tyr Lys Asp Asp Asp Asp Lys
   140 145 150
   tga tag 456
<210> 27
   <211> 46
   <212> DNA
   <213> Artificial Sequence
<220>
   <223> PCR primer
<400> 27
   ggaattccat atgcaagtgc aacttcaaca gtctggacct gaactg 46
<210> 28.
   <211> 31
   <212> DNA
   <213> Artificial Sequence
<220>
   <223> PCR primer
<400> 28
   ggaattctca ttattttatt tccagcttgg t 31
<210> 29
   <211> 741
   <212> DNA
   <213> Mus
<220>
   <221> CDS
   <222> (1)...(735)
   <223> pscM2DEm02. MABL2-scFv
<400> 29
   atg caa gtg caa ctt caa cag tct gga cct gaa ctg gta aag cct 45
   Met Gln Val Gln Leu Gln Gln Ser Gly Pro Glu Leu Val Lys Pro
   5 10 15
   ggg gct tca gtg aag atg tcc tgc aag gct tct gga tac acc ttc 90
   Gly Ala Ser Val Lys Met Ser Cys Lys Ala Ser Gly Tyr Thr Phe
   20 25 30
   gct aac cat gtt att cac tgg gtg aag cag aag cca ggg cag ggc 135
   Ala Asn His Val Ile His Trp Val Lys Gln Lys Pro Gly Gln Gly
   35 40 45
   ctt gag tgg att gga tat att tat cct tac aat gat ggt act aag 180
   Leu Glu Trp Ile Gly Tyr Ile Tyr Pro Tyr Asn Asp Gly Thr Lys
   50 55 60
   tat aat gag aag ttc aag gac aag gcc act ctg act tca gac aaa 225
   Tyr Asn Glu Lys Phe Lys Asp Lys Ala Thr Leu Thr Ser Asp Lys
   65 70 75
   tcc tcc acc aca gcc tac atg gac ctc agc agc ctg gcc tct gag 270
   Ser Ser Thr Thr Ala Tyr Met Asp Leu Ser Ser Leu Ala Ser Glu
   80 85 90
   gac tct gcg gtc tat tac tgt gca aga ggg ggt tac tat act tac 315
   Asp Ser Ala Val Tyr Tyr Cys Ala Arg Gly Gly Tyr Tyr Thr Tyr
   95 100 105
   gac gac tgg ggc caa ggc acc act ctc aca gtc tcc tca ggt ggt 360
   Asp Asp Trp Gly Gln Gly Thr Thr Leu Thr Val Ser Ser Gly Gly
   110 115 120
   ggt ggt tcg ggt ggt ggt ggt tcg ggt ggt ggc gga tcg gat gtt 405
   Gly Gly Ser Gly Gly Gly Gly Ser Gly Gly Gly Gly Ser Asp Val
   125 130 135
   gtg atg acc caa agt cca ctc tcc ctg cct gtc agt ctt gga gat 450
   Val Met Thr Gln Ser Pro Leu Ser Leu Pro Val Ser Leu Gly Asp
   140 145 150
   caa gcc tcc atc tct tgc aga tca agt cag agc ctt gtg cac agt 495
   Gln Ala Ser Ile Ser Cys Arg Ser Ser Gln Ser Leu Val His Ser
   155 160 165
   aat gga aag acc tat tta cat tgg tac ctg cag aag cca ggc cag 540
   Asn Gly Lys Thr Tyr Leu His Trp Tyr Leu Gln Lys Pro Gly Gln
   170 175 180
   tct cca aaa ctc ctg atc tac aaa gtt tcc aac cga ttt tct ggg 585
   Ser Pro Lys Leu Leu Ile Tyr Lys Val Ser Asn Arg Phe Ser Gly
   185 190 195
   gtc cca gac agg ttc agt ggc agt gga tca gtg aca gat ttc aca 630
   Val Pro Asp Arg Phe Ser Gly Ser Gly Ser Val Thr Asp Phe Thr
   200 205 210
   ctc atg atc agc aga gtg gag gct gag gat ctg gga gtt tat ttc 675
   Leu Met Ile Ser Arg Val Glu Ala Glu Asp Leu Gly Val Tyr Phe
   215 220 225
   tgc tct caa agt aca cat gtt ccg tac acg ttc gga ggg ggg acc 720
   Cys Ser Gln Ser Thr His Val Pro Tyr Thr Phe Gly Gly Gly Thr
   230 235 240
   aag ctg gaa ata aaa taa tga 741
   Lys Leu Glu Ile Lys
<210> 30
   <211> 18
   <212> DNA
   <213> Artificial Sequence
<220>
   <223> PCR primer
<400> 30
   cagacagtgg ttcaaagt 18
<210> 31
   <211> 72
   <212> DNA
   <213> Artificial Sequence
<220>
   <223> PCR primer
<400> 31
   cgcgtcgacc gatccgccac cacccgaacc accaccaccc gaaccaccac caccttttat 60
   ttccagcttg gt 72
<210> 32
   <211> 1605
   <212> DNA
   <213> Mus
<220>
   <221> CDS
   <222> (1)...(1599)
   <223> pCHOM2(Fv)2. MABL2-sc(Fv)2
<400> 32
   atg gga tgg agc tgt atc atc ctc ttc ttg gta gca aca gct aca 45
   Met Gly Trp Ser Cys Ile Ile Leu Phe Leu Val Ala Thr Ala Thr
   5 10 15
   ggt gtc gac tcc cag gtc cag ctg cag cag tct gga cct gaa ctg 90
   Gly Val Asp Ser Gln Val Gln Leu Gln Gln Ser Gly Pro Glu Leu
   20 25 30
   gta aag cct ggg gct tca gtg aag atg tcc tgc aag gct tct gga 135
   Val Lys Pro Gly Ala Ser Val Lys Met Ser Cys Lys Ala Ser Gly
   35 40 45
   tac acc ttc gct aac cat gtt att cac tgg gtg aag cag aag cca 180
   Tyr Thr Phe Ala Asn His Val Ile His Trp Val Lys Gln Lys Pro
   50 55 60
   ggg cag ggc ctt gag tgg att gga tat att tat cct tac aat gat 225
   Gly Gln Gly Leu Glu Trp Ile Gly Tyr Ile Tyr Pro Tyr Asn Asp
   65 70 75
   ggt act aag tat aat gag aag ttc aag gac aag gcc act ctg act 270
   Gly Thr Lys Tyr Asn Glu Lys Phe Lys Asp Lys Ala Thr Leu Thr
   80 85 90
   tca gac aaa tcc tcc acc aca gcc tac atg gac ctc agc agc ctg 315
   Ser Asp Lys Ser Ser Thr Thr Ala Tyr Met Asp Leu Ser Ser Leu
   95 100 105
   gcc tct gag gac tct gcg gtc tat tac tgt gca aga ggg ggt tac 360
   Ala Ser Glu Asp Ser Ala Val Tyr Tyr Cys Ala Arg Gly Gly Tyr
   110 115 120
   tat act tac gac gac tgg ggc caa ggc acc act ctc aca gtc tcc 405
   Tyr Thr Tyr Asp Asp Trp Gly Gln Gly Thr Thr Leu Thr Val Ser
   125 130 135
   tca ggt ggt ggt ggt tcg ggt ggt ggt ggt tcg ggt ggt ggc gga 450
   Ser Gly Gly Gly Gly Ser Gly Gly Gly Gly Ser Gly Gly Gly Gly
   140 145 150
   tcg gat gtt gtg atg acc caa agt cca ctc tcc ctg cct gtc agt 495
   Ser Asp Val Val Met Thr Gln Ser Pro Leu Ser Leu Pro Val Ser
   155 160 165
   ctt gga gat caa gcc tcc atc tct tgc aga tca agt cag agc ctt 540
   Leu Gly Asp Gln Ala Ser Ile Ser Cys Arg Ser Ser Gln Ser Leu
   170 175 180
   gtg cac agt aat gga aag acc tat tta cat tgg tac ctg cag aag 585
   Val His Ser Asn Gly Lys Thr Tyr Leu His Trp Tyr Leu Gln Lys
   185 190 195
   cca ggc cag tct cca aaa ctc ctg atc tac aaa gtt tcc aac cga 630
   Pro Gly Gln Ser Pro Lys Leu Leu Ile Tyr Lys Val Ser Asn Arg
   200 205 210
   ttt tct ggg gtc cca gac agg ttc agt ggc agt gga tca gtg aca 675
   Phe Ser Gly Val Pro Asp Arg Phe Ser Gly Ser Gly Ser Val Thr
   215 220 225
   gat ttc aca ctc atg atc agc aga gtg gag gct gag gat ctg gga 720
   Asp Phe Thr Leu Met Ile Ser Arg Val Glu Ala Glu Asp Leu Gly
   230 235 240
   gtt tat ttc tgc tct caa agt aca cat gtt ccg tac acg ttc gga 765
   Val Tyr Phe Cys Ser Gln Ser Thr His Val Pro Tyr Thr Phe Gly
   245 250 255
   ggg ggg acc aag ctg gaa ata aaa ggt ggt ggt ggt tcg ggt ggt 810
   Gly Gly Thr Lys Leu Glu Ile Lys Gly Gly Gly Gly Ser Gly Gly
   260 265 270
   ggt ggt tcg ggt ggt ggc gga tcg gtc gac tcc cag gtc cag ctg 855
   Gly Gly Ser Gly Gly Gly Gly Ser Val Asp Ser Gln Val Gln Leu
   275 280 285
   cag cag tct gga cct gaa ctg gta aag cct ggg gct tca gtg aag 900
   Gln Gln Ser Gly Pro Glu Leu Val Lys Pro Gly Ala Ser Val Lys
   290 295 300
   atg tcc tgc aag gct tct gga tac acc ttc gct aac cat gtt att 945
   Met Ser Cys Lys Ala Ser Gly Tyr Thr Phe Ala Asn His Val Ile
   305 310 315
   cac tgg gtg aag cag aag cca ggg cag ggc ctt gag tgg att gga 990
   His Trp Val Lys Gln Lys Pro Gly Gln Gly Leu Glu Trp Ile Gly
   320 325 330
   tat att tat cct tac aat gat ggt act aag tat aat gag aag ttc 1035
   Tyr Ile Tyr Pro Tyr Asn Asp Gly Thr Lys Tyr Asn Glu Lys Phe
   335 340 345
   aag gac aag gcc act ctg act tca gac aaa tcc tcc acc aca gcc 1080
   Lys Asp Lys Ala Thr Leu Thr Ser Asp Lys Ser Ser Thr Thr Ala
   350 355 360
   tac atg gac ctc agc agc ctg gcc tct gag gac tct gcg gtc tat 1125
   Tyr Met Asp Leu Ser Ser Leu Ala Ser Glu Asp Ser Ala Val Tyr
   365 370 375
   tac tgt gca aga ggg ggt tac tat act tac gac gac tgg ggc caa 1170
   Tyr Cys Ala Arg Gly Gly Tyr Tyr Thr Tyr Asp Asp Trp Gly Gln
   380 385 390
   ggc acc act ctc aca gtc tcc tca ggt ggt ggt ggt tcg ggt ggt 1215
   Gly Thr Thr Leu Thr Val Ser Ser Gly Gly Gly Gly Ser Gly Gly
   395 400 405
   ggt ggt tcg ggt ggt ggc gga tcg gat gtt gtg atg acc caa agt 1260
   Gly Gly Ser Gly Gly Gly Gly Ser Asp Val Val Met Thr Gln Ser
   410 415 420
   cca ctc tcc ctg cct gtc agt ctt gga gat caa gcc tcc atc tct 1305
   Pro Leu Ser Leu Pro Val Ser Leu Gly Asp Gln Ala Ser Ile Ser
   425 430 435
   tgc aga tca agt cag agc ctt gtg cac agt aat gga aag acc tat 1350
   Cys Arg Ser Ser Gln Ser Leu Val His Ser Asn Gly Lys Thr Tyr
   440 445 450
   tta cat tgg tac ctg cag aag cca ggc cag tct cca aaa ctc ctg 1395
   Leu His Trp Tyr Leu Gln Lys Pro Gly Gln Ser Pro Lys Leu Leu
   455 460 465
   atc tac aaa gtt tcc aac cga ttt tct ggg gtc cca gac agg ttc 1440
   Ile Tyr Lys Val Ser Asn Arg Phe Ser Gly Val Pro Asp Arg Phe
   470 475 480
   agt ggc agt gga tca gtg aca gat ttc aca ctc atg atc agc aga 1485
   Ser Gly Ser Gly Ser Val Thr Asp Phe Thr Leu Met Ile Ser Arg
   485 490 495
   gtg gag gct gag gat ctg gga gtt tat ttc tgc tct caa agt aca 1530
   Val Glu Ala Glu Asp Leu Gly Val Tyr Phe Cys Ser Gln Ser Thr
   500 505 510
   cat gtt ccg tac acg ttc gga ggg ggg acc aag ctg gaa ata aaa 1575
   His Val Pro Tyr Thr Phe Gly Gly Gly Thr Lys Leu Glu Ile Lys
   515 520 525
   gac tac aaa gac gat gac gat aaa taa tga 1605
   Asp Tyr Lys Asp Asp Asp Asp Lys
   530
<210> 33
   <211> 23
   <212> DNA
   <213> Artificial Sequence
<220>
   <223> PCR primer
<400> 33
   tgaggaattc ccaccatggg atg 33
<210> 34
   <211> 40
   <212> DNA
   <213> Artificial Sequence
<220.>
   <223> PCR primer
<400> 34
   cacgacgtca ctcgagactg tgagagtggt gccttggccc 40
<210> 35
   <211> 40
   <212> DNA
   <213> Artificial Sequence
<220>
   <223> PCR primer
<400> 35
   agtctcgagt gacgtcgtga tgacccaaag tccactctcc 40
<210> 36
   <211> 31
   <212> DNA
   <213> Artificial Sequence
<220>
   <223> PCR primer
<400> 36
   gactggatcc tcattattta tcgtcatcgt c 31
<210> 37
   <211> 22
   <212> DNA
   <213> Artificial Sequence
<220>
   <223> PCR primer
<400> 37
   cgcgtaatac gactcactat ag 22
<210> 38
   <211> 46
   <212> DNA
   <213> Artificial Sequence
<220>
   <223> PCR primer
<400> 38
   gcaattggac ctgttttatc tcgagcttgg tcccccctcc gaacgt 46
<210> 39
   <211> 45
   <212> DNA
   <213> Artificial Sequence
<220>
   <223> PCR primer
<400> 39
   gctcgagata aaacaggtcc aattgcagca gtctggacct gaact 45
<210> 40
   <211> 60
   <212> DNA
   <213> Artificial Sequence
<220>
   <223> PCR primer .
<400> 40
   gactggatcc tcattattta tcgtcatcgt ctttgtagtc tgaggagact gtgagagtgg 60
<210> 41
   <211> 32
   <212> DNA
   <213> Artificial Sequence
<220>
   <223> PCR primer
<400> 41
   gactgaattc ccaccatgaa gttgcctgtt ag 32
<210> 42
   <211> 40
   <212> DNA
   <213> Artificial Sequence
<220>
   <223> PCR primer
<400> 42
   cagtctcgag tggtggttcc gacgtcgtga tgacccaaag 40
<210> 43
   <211> 43
   <212> DNA
   <213> Artificial Sequence
<220>
   <223> PCR primer
<400> 43
   cagtctcgag tggtggtggt tccgacgtcg tgatgaccca aag 43
<210> 44
   <211> 46
   <212> DNA
   <213> Artificial Sequence
<220>
   <223> PCR. primer
<400> 44
   cagtctcgag tggtggtggt ggttccgacg tcgtgatgac ccaaaag 46
<210> 45
   <211> 49
   <212> DNA
   <213> Artificial Sequence
<220>
   <223> PCR primer
<400> 45
   cagtctcgag tggtggtggt ggtggttccg acgtcgtgat gacccaaag 49
<210> 46
   <211> 52
   <212> DNA
   <213> Artificial Sequence
<220>
   <223> PCR primer
<400> 46
   cagtctcgag tggtggtggt ggtggtggyy ccgacgtcgt gatgacccaa ag 52
<210> 47
   <211> 20
   <212> DNA
   <213> Artificial Sequence
<220>
   <223> PCR primer
<400> 47
   ggccgcatgt tgtcacgaat 20
<210> 48
   <211> 780
   <212> DNA
   <213> Mus
<220>
   <221> CDS
   <222> (1)...(768)
   <223> CF2HL-0/pC0S1. MABL2-scFv<HL-0>
<400> 48
   atg gga tgg agc tgt atc atc ctc ttc ttg gta gca aca gct aca ggt gtc 51
   MET Gly Trp Ser Cys Ile Ile Leu Phe Leu Val Ala Thr Ala Thr Gly Val
   5 10 15
   gac tcc cag gtc cag ctg cag cag tct gga cct gaa ctg gta aag cct ggg 102
   Asp Ser Gln Val Gln Leu Gln Gln Ser Gly Pro Glu Leu Val Lys Pro Gly
   20 25 30
   gct tca gtg aag atg tcc tgc aag gct tct gga tac acc ttc gct aac cat 153
   Ala Ser Val Lys MET Ser Cys Lys Ala Ser Gly Tyr Thr Phe Ala Asn His
   35 40 45 50
   gtt att cac tgg gtg aag cag aag cca ggg cag ggc ctt gag tgg att gga 204
   Val Ile His Trp Val Lys Gln Lys Pro Gly Gln Gly Leu Glu Trp Ile Gly
   55 60 65
   tat att tat cct tac aat gat ggt act aag tat aat gag aag ttc aag gac 255
   Tyr Ile Tyr Pro Tyr Asn Asp Gly Thr Lys Tyr Asn Glu Lys Phe Lys Asp
   70 75 80 85
   aag gcc act ctg act tca gac aaa tcc tcc acc aca gcc tac atg gac ctc 306
   Lys Ala Thr Leu Thr Ser Asp Lys Ser Ser Thr Thr Ala Tyr MET Asp Leu
   90 95 100
   agc agc ctg gcc tct gag gac tct gcg gtc tat tac tgt gca aga ggg ggt 357
   Ser Ser Leu Ala Ser Glu Asp Ser Ala Val Tyr Tyr Cys Ala Arg Gly Gly
   105 110 115
   tac tat act tac gac gac tgg ggc caa ggc acc act ctc aca gtc tcg agt 408
   Tyr Tyr Thr Tyr Asp Asp Trp Gly Gln Gly Thr Thr Leu Thr Val Ser Ser
   120 125 130 135
   gac gtc gtg atg acc caa agt cca ctc tcc ctg cct gtc agt ctt gga gat 459
   Asp Val Val MET Thr Gln Ser Pro Leu Ser Leu Pro Val Ser Leu Gly Asp
   140 145 150
   caa gcc tcc atc tct tgc aga tca agt cag agc ctt gtg cac agt aat gga 510
   Gln Ala Ser Ile Ser Cys Arg Ser Ser Gln Ser Leu Val His Ser Asn Gly
   155 160 165 170
   aag acc tat tta cat tgg tac ctg cag aag cca ggc cag tct cca aaa ctc 561
   Lys Thr Tyr Leu His Trp Tyr Leu Gln Lys Pro Gly Gln Ser Pro Lys Leu
   175 180 185
   ctg atc tac aaa gtt tcc aac cga ttt tct ggg gtc cca gac agg ttc agt 612
   Leu Ile Tyr Lys Val Ser Asn Arg Phe Ser Gly Val Pro Asp Arg Phe Ser
   190 195 200
   ggc agt gga tca gtg aca gat ttc aca ctc atg atc agc aga gtg gag gct 663
   Gly Ser Gly Ser Val Thr Asp Phe Thr Leu MET Ile Ser Arg Val Glu Ala
   205 210 215 220
   gag gat ctg gga gtt tat ttc tgc tct caa agt aca cat gtt ccg tac acg 714
   Glu Asp Leu Gly Val Tyr Phe Cys Ser Gln Ser Thr His Val Pro Tyr Thr
   225 230 235
   ttc gga ggg ggg acc aag ctg gaa ata aaa gac tac aaa gac gat gac gat 765
   Phe Gly Gly Gly Thr Lys Leu Glu Ile Lys Asp Tyr Lys Asp Asp Asp Asp
   240 245 250 255
   aaa taa tga gga tcc 780
   Lys
<210> 49
   <211> 45
   <212> DNA
   <213> Artificial Sequence
<220>
   <223> PCR primer
<400> 49
   caagctcgag ataaaatccg gaggccaggt ccaattgcag cagtc 45
<210> 50
   <211> 48
   <212> DNA
   <213> Artificial Sequence
<220>
   <223> PCR primer
<400> 50
   caagctcgag ataaaatccg gaggtggcca ggtccaattg cagcagtc 48
<210> 51
   <211> 51
   <212> DNA
   <213> Artificial Sequence
<220>
   <223> PCR primer
<400> 51
   caagctcgag ataaaatccg gaggtggtgg ccaggtccaa ttgcagcagt c 51
<210> 52
   <211> 54
   <212> DNA
   <213> Artificial Sequence
<220>
   <223> PCR primer
<400> 52
   caagctcgag ataaaatccg gaggtggtgg tggccaggtc caattgcagc agtc 54
<210> 53
   <211> 57
   <212> DNA
   <213> Artificial Sequence
<220>
   <223> PCR primer
<400> 53
   caagctcgag ataaaatccg gaggtggtgg tggtggccag gtccaattgc agcagtc 57
<210> 54
   <211> 780
   <212> DNA
   <213> Mus
<220>
   <221> CDS
   <222> (1)...(768)
   <223> CF2LH-0/pC0S1. MABL2-scFv<LH-0>
<400> 54
   atg aag ttg cct gtt agg ctg ttg gtg ctg atg ttc tgg att cct ggt tcc 51
   MET Lys Leu Pro Val Arg Leu Leu Val Leu MET Phe Trp Ile Pro Gly Ser
   5 10 15
   agc agt gat gtt gtg atg acc caa agt cca ctc tcc ctg cct gtc agt ctt 102
   Ser Ser Asp Val Val MET Thr Gln Ser Pro Leu Ser Leu Pro Val Ser Leu
   20 25 30
   gga gat caa gcc tcc atc tct tgc aga tca agt cag agc ctt gtg cac agt 153
   Gly Asp Gln Ala Ser Ile Ser Cys Arg Ser Ser Gln Ser Leu Val His Ser
   35 40 45 50
   aat gga aag acc tat tta cat tgg tac ctg cag aag cca ggc cag tct cca 204
   Asn Gly Lys Thr Tyr Leu His Trp Tyr Leu Gln Lys Pro Gly Gln Ser Pro
   55 60 65
   aaa ctc ctg atc tac aaa gtt tcc aac cga ttt tct ggg gtc cca gac agg 255
   Lys Leu Leu Ile Tyr Lys Val Ser Asn Arg Phe Ser Gly Val Pro Asp Arg
   70 75 80 85
   ttc agt ggc agt gga tca gtg aca gat ttc aca ctc atg atc agc aga gtg 306
   Phe Ser Gly Ser Gly Ser Val Thr Asp Phe Thr Leu MET Ile Ser Arg Val
   90 95 100
   gag gct gag gat ctg gga gtt tat ttc tgc tct caa agt aca cat gtt ccg 357
   Glu Ala Glu Asp Leu Gly Val Tyr Phe Cys Ser Gln Ser Thr His Val Pro
   105 110 115
   tac acg ttc gga ggg ggg acc aag ctc gag ata aaa cag gtc caa ttg cag 408
   Tyr Thr Phe Gly Gly Gly Thr Lys Leu Glu Ile Lys Gln Val Gln Leu Gln
   120 125 130 135
   cag tct gga cct gaa ctg gta aag cct ggg gct tca gtg aag atg tcc tgc 459
   Gln Ser Gly Pro Glu Leu Val Lys Pro Gly Ala Ser Val Lys MET Ser Cys
   140 145 150
   aag gct tct gga tac acc ttc gct aac cat gtt att cac tgg gtg aag cag 510
   Lys Ala Ser Gly Tyr Thr Phe Ala Asn His Val Ile His Trp Val Lys Gln
   155 160 165 170
   aag cca ggg cag ggc ctt gag tgg att gga tat att tat cct tac aat gat 561
   Lys Pro Gly Gln Gly Leu Glu Trp Ile Gly Tyr Ile Tyr Pro Tyr Asn Asp
   175 180 185
   ggt act aag tat aat gag aag ttc aag gac aag gcc act ctg act tca gac 612
   Gly Thr Lys Tyr Asn Glu Lys Phe Lys Asp Lys Ala Thr Leu Thr Ser Asp
   190 195 200
   aaa tcc tcc acc aca gcc tac atg gac ctc agc agc ctg gcc tct gag gac 663
   Lys Ser Ser Thr Thr Ala Tyr MET Asp Leu Ser Ser Leu Ala Ser Glu Asp
   205 210 215 220
   tct gcg gtc tat tac tgt gca aga ggg ggt tac tat act tac gac gac tgg 714
   Ser Ala Val Tyr Tyr Cys Ala Arg Gly Gly Tyr Tyr Thr Tyr Asp Asp Trp
   225 230 235
   ggc caa ggc acc act ctc aca gtc tcc tca gac tac aaa gac gat gac gat 765
   Gly Gln Gly Thr Thr Leu Thr Val Ser Ser Asp Tyr Lys Asp Asp Asp Asp
   240 245 250 255
   aaa taa tga gga tcc 780
   Lys

## Claims

1. A reconstructed polypeptide which binds to Integrin Associated Protein (IAP), induces apoptosis of nucleated blood cells and causes no hemagglutination,
wherein the reconstructed polypeptide comprises two H chain V regions and two L chain V regions of an antibody which binds to IAP and induces apoptosis of nucleated blood cells, and
wherein the reconstructed polypeptide is a dimer of a single-chain Fv comprising an H chain V region and an L chain V region or a single chain polypeptide comprising two H chain V regions and two L chain V regions.

2. The reconstructed polypeptide of claim 1, wherein the reconstructed polypeptide is a purified dimer of a single-chain Fv.

3. The reconstructed polypeptide of claim 1 or 2, wherein the H chain V region and the L chain V region are linked with a linker comprising at least one amino acid.

4. A DNA encoding the single-chain Fv of claims 1 or 3.

5. A DNA encoding the polypeptide of claim 1 or 3.

6. The reconstructed polypeptide of claim 1, wherein the H chain V region and/or the L chain V region are humanised.

7. A DNA encoding the polypeptide of claim 6.

8. An animal cell which produces the reconstructed polypeptide of any one of claims 1, 3 and 6.

9. A microorganism which produces the reconstructed polypeptide of any one of claims 1, 3 and 6.

10. A therapeutic agent for blood dyscrasia which comprises the 2, 3, and 6 as an active ingredient.

11. The therapeutic agent of claim 10 **characterised in that** the blood dyscrasia is leukaemia.

12. The therapeutic agent of claim 10 **characterised in that** the active ingredient is the dimer of the single-chain Fv of any one of claims 1 to 3.

## Patentansprüche

1. Rekonstruiertes Polypeptid, das an Integrin assoziiertes Protein (IAP) bindet, Apoptose von kernhaltigen Blutzellen induziert und keine Hämagglutination verursacht,
worin das rekonstruierte Polypeptid zwei H-Ketten V-Regionen und zwei L-Ketten V-Regionen eines Antikörpers, der an IAP bindet und Apoptose von kernhaltigen Blutzellen induziert, umfaßt und
worin das rekonstruierte Polypeptid ein Dimer eines einzelkettigen Fv, umfassend eine H-Ketten V-Region und eine L-Ketten V-Region, oder ein einzelkettiges Polypeptid, umfassend zwei H-Ketten V-Regionen und zwei L-Ketten V-Regionen, ist.

2. Rekonstruiertes Polypeptid gemäß Anspruch 1, worin das rekonstruierte Polypeptid ein gereinigtes Dimer eines einzelkettigen Fv ist.

3. Rekonstruiertes Polypeptid gemäß Anspruch 1 oder 2, worin die H-Ketten V-Region und die L-Ketten V-Region mit einem Linker, umfassend zumindest eine Aminosäure, verbunden sind.

4. DNA codierend für das einzelkettige Fv gemäß Anspruch 1 oder 3.

5. DNA codierend für das Polypeptid gemäß Anspruch 1 oder 3.

6. Rekonstruiertes Polypeptid gemäß Anspruch 1, worin die H-Ketten V-Region und/oder die L-Ketten V-Region humanisiert sind.

7. DNA codierend für das Polypeptid gemäß Anspruch 6.

8. Tierische Zelle, die das rekonstruierte Polypeptid gemäß einem der Ansprüche 1, 3 und 6 produziert.

9. Mikroorganismus, der das rekonstruierte Polypeptid gemäß einem der Ansprüche 1, 3 und 6 produziert.

10. Therapeutisches Agens für Blutdyskrasie, umfassend das rekonstruierte Polypeptid gemäß einem der Ansprüche 1, 2, 3 und 6 als aktiven Inhaltsstoff.

11. Therapeutisches Agens gemäß Anspruch 10 **gekennzeichnet dadurch, daß** die Blutdyskrasie Leukämie ist.

12. Therapeutisches Agens gemäß Anspruch 10 **gekennzeichnet dadurch, daß** der aktive Inhaltsstoff das Dimer des einzelkettigen Fv gemäß einem der Ansprüche 1 bis 3 ist.

## Revendications

1. Polypeptide reconstitué qui se lie à une protéine associée une intégrine (en Anglais, « Integrin Associated Protein », IAP), induit l'apoptose de cellules sanguines nucléées et ne provoque aucune hémoagglutination,
dans lequel le polypeptide reconstitué comprend deux régions V de chaîne H et deux régions V de chaîne L d'un anticorps qui se lie à l'IAP et induit l'apoptose de cellules sanguines nucléées, et
dans lequel le polypeptide reconstitué est un dimère de Fv monochaîne comprenant une région V chaîne H et une région V de chaîne L ou un polypeptide monochaîne comprenant deux régions V de chaîne H et deux régions V de chaîne L.

2. Polypeptide reconstitué de la revendication 1, dans lequel le polypeptide reconstitué est un dimère purifié d'une Fv monochaîne.

3. Polypeptide reconstitué selon la revendication 1 ou 2, dans lequel la région V de chaîne H et la région V de chaîne L sont liées avec un segment de liaison comprenant au moins un acide aminé.

4. ADN codant le Fv monochaîne selon les revendications 1 ou 3.

5. ADN codant le polypeptide de la revendication 1 ou 3.

6. Polypeptide reconstitué selon la revendication 1, dans lequel la région V de chaîne H et/ou la région V de chaîne L sont humanisées.

7. ADN codant le polypeptide de la revendication 6.

8. Cellule animale qui produit le polypeptide reconstitué selon l'une quelconque des revendications 1, 3 et 6.

9. Microorganisme qui produit le polypeptide reconstitué selon l'une quelconque des revendications 1, 3 et 6.

10. Agent thérapeutique destiné à une dyscrasie sanguine qui comprend le polypeptide reconstitué selon l'une quelconque des revendications 1, 2, 3 et 6 en tant qu'ingrédient actif.

11. Agent thérapeutique selon la revendication 10, **caractérisé en ce que** la dyscrasie sanguine est une leucémie.

12. Agent thérapeutique selon la revendication 10, **caractérisé en ce que** l'ingrédient actif est le dimère du Fv monochaîne selon l'une quelconque des revendications 1 à 3.
